(19) Europäisches Patentamt / European Patent Office / Office européen des brevets

(11) EP 2 721 036 B1

(12) **EUROPEAN PATENT SPECIFICATION**

(45) Date of publication and mention of the grant of the patent:
**22.07.2015 Bulletin 2015/30**

(21) Application number: **12729458.5**

(22) Date of filing: **12.06.2012**

(51) Int Cl.:
***C07D 495/04*** *(2006.01)* ***C07D 519/00*** *(2006.01)*
***A61K 31/519*** *(2006.01)* ***A61P 11/00*** *(2006.01)*

(86) International application number:
**PCT/EP2012/061091**

(87) International publication number:
**WO 2012/171903 (20.12.2012 Gazette 2012/51)**

(54) **NOVEL 3,4,4A,10B-TETRAHYDRO-1H-THIOPYRANO[4,3-C]ISOQUINOLINE COMPOUNDS**

NEUARTIGE 3,4,4A,10B-TETRAHYDRO-1H-THIOPYRANO[4,3-C]ISOCHINOLIN-VERBINDUNGEN

NOUVEAUX COMPOSÉS DE 3,4,4A,10B-TÉTRAHYDRO-1H-THIOPYRANO[4,3-C]ISOQUINOLINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.06.2011 EP 11169908**
**20.09.2011 US 201161536810 P**

(43) Date of publication of application:
**23.04.2014 Bulletin 2014/17**

(73) Proprietor: **Takeda GmbH**
**78467 Konstanz (DE)**

(72) Inventors:
- **FLOCKERZI, Dieter**
  **78476 Allensbach (DE)**
- **MANN, Alexander**
  **78315 Radolfzell (DE)**
- **STENGEL, Thomas**
  **76344 Eggenstein-Leopoldshafen (DE)**
- **OHMER, Harald**
  **78224 Singen (DE)**
- **KAUTZ, Ulrich**
  **78476 Allensbach (DE)**
- **WEINBRENNER, Steffen**
  **78465 Konstanz (DE)**
- **FISCHER, Stefan**
  **67251 Freinsheim (DE)**
- **TENOR, Hermann**
  **78467 Konstanz (DE)**
- **ZITT, Christof**
  **78464 Konstanz (DE)**
- **HATZELMANN, Armin**
  **78467 Konstanz (DE)**
- **DUNKERN, Torsten**
  **41363 Jüchen (DE)**
- **HESSLINGER, Christian**
  **78357 Zoznegg (DE)**
- **BRAUN, Clemens**
  **88400 Biberbach (DE)**
- **BENEDIKTUS, Ewald**
  **88400 Biberach (DE)**
- **PAHL, Andreas**
  **69115 Heidelberg (DE)**
- **KÜLZER, Raimund**
  **78467 Konstanz (DE)**
- **MARX, Degenhard**
  **78345 Moos (DE)**

(74) Representative: **Wild, Robert et al**
**Takeda GmbH**
**Postfach 10 03 10**
**78403 Konstanz (DE)**

(56) References cited:
**WO-A2-2006/027345**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 721 036 B1

## Description

### Field of application of the invention

The invention relates to novel 3,4,4a,10b-tetrahydro-1H-thiopyrano[4,3-c]isoquinoline compounds, which are used in the pharmaceutical industry for the manufacture of pharmaceutical compositions.

### Known technical background

In the international patent application WO2006027345 3,4,4a,10b-tetrahydro-1H-thiopyrano[4,3-c]isoquinoline compounds are described as type 4 phosphodiesterase inhibitors. The international patent application WO2011073231 also describes 3,4,4a,10b-tetrahydro-1H-thiopyrano[4,3-c]isoquinoline compounds representing type 4 and 5 phosphodiesterase inhibitors.

### Description of the invention

It has now been found that the 3,4,4a,10b-tetrahydro-1H-thiopyrano[4,3-c]isoquinoline compounds, which are described in greater details below, have surprising and particularly advantageous properties.

The invention relates to a compound of formula (1)

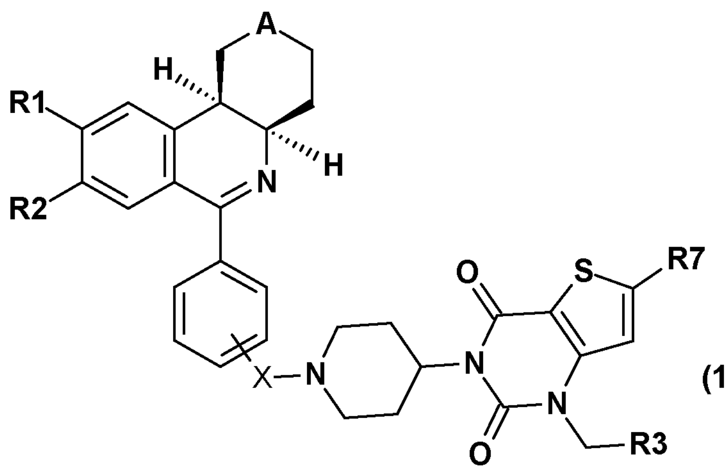

(1)

,

wherein

| | |
|---|---|
| A | is S, S(O) or $S(O)_2$, |
| X | is C(O) and is attached either in ortho, meta or para position to the phenyl, |
| R1 | is 1-4C-alkoxy, 3-7C-cycloalkoxy, 3-7C-cycloalkylmethoxy or 1-4C-alkoxy predominantly or completely substituted by fluorine, |
| R2 | is 1-4C-alkoxy, 3-7C-cycloalkoxy, 3-7C-cycloalkylmethoxy, or 1-4C-alkoxy predominantly or completely substituted by fluorine, or |
| R1 and R2 | together form a 1-2C-alkylenedioxy group, |
| R3 | is unsubstituted phenyl, phenyl substituted by R4, phenyl substituted by R4 and R5 or phenyl substituted by R4, R5 and R6, wherein R4 is 1-4C-alkyl, 1-4C-alkoxy or halogen, R5 is 1-4C-alkyl, 1-4C-alkoxy or halogen and R6 is 1-4C-alkyl, 1-4C-alkoxy or halogen, |
| R7 | is unsubstituted phenyl, phenyl substituted by R8 or phenyl substituted by R8 and R9, wherein R8 is 1-4 C-alkyl, 1-4C-alkoxy or halogen and R9 is 1-4C-alkyl, 1-4C-alkoxy or halogen, or R8 and R9 together form a 1-2C-alkylenedioxy group, |

or a stereoisomer of the compound.

1-4C-Alkyl is a straight-chain or branched alkyl group having 1 to 4 carbon atoms. Examples are butyl, isobutyl, sec-butyl, tert-butyl, propyl, isopropyl, ethyl and methyl.

1-2C-Alkyl is a straight-chain alkyl group having 1 to 2 carbon atoms. Examples are ethyl and methyl.

1-4C-Alkoxy is a group which, in addition to the oxygen atom, contains a straight-chain or branched alkyl group

having 1 to 4 carbon atoms. Alkoxy groups having 1 to 4 carbon atoms, which may be mentioned in this context are, for example, butoxy, isobutoxy, sec-butoxy, tert-butoxy, propoxy, isopropoxy, ethoxy and methoxy.

1-2C-Alkoxy is a group, which in addition to the oxygen atom, contains a straight-chain alkyl group having 1 to 2 carbon atoms. Examples are ethoxy and methoxy.

1-2C-Alkylenedioxy represents, for example, the methylenedioxy [-O-$CH_2$-O-] and the ethylenedioxy [-O-$CH_2$-$CH_2$-O-] group.

1-4C-Alkoxy, which is completely or predominantly substituted by fluorine is, for example, the 2,2,3,3,3-pentafluoropropoxy, the perfluoroethoxy, the 1,2,2-trifluoroethoxy and, in particular the 1,1,2,2-tetrafluoroethoxy, the 2,2,2-trifluoroethoxy, the trifluoromethoxy and the difluoromethoxy group, of which the difluoromethoxy group is preferred. "Predominantly" in this connection means that more than half of the hydrogen atoms of the 1-4C-alkoxy group are replaced by fluorine atoms. 1-2C-Alkoxy which is completely or predominantly substituted by fluorine is, for example, the perfluoroethoxy, the 1,2,2-trifluoroethoxy, the 1,1,2,2-tetrafluoroethoxy, the 2,2,2-trifluoroethoxy, the trifluoromethoxy and the difluoromethoxy group, of which the difluoromethoxy group is preferred. "Predominantly" in this connection means that more than half of the hydrogen atoms of the 1-2C-alkoxy group are replaced by fluorine atoms.

3-7C-Cycloalkoxy stands for cyclopropyloxy, cyclobutyloxy, cyclopentyloxy, cyclohexyloxy or cycloheptyloxy with 3-5C-Cycloalkoxy being preferred, which stands for cyclopropyloxy, cyclobutyloxy or cyclopentyloxy.

3-7C-Cycloalkylmethoxy stands for cyclopropylmethoxy, cyclobutylmethoxy, cyclopentylmethoxy, cyclohexylmethoxy or cycloheptylmethoxy with 3-5C-Cycloalkylmethoxy being preferred, which stands for cyclopropylmethoxy, cyclobutylmethoxy or cyclopentylmethoxy.

Halogen stands for fluorine, chlorine, bromine or iodine, with fluorine, chlorine or bromine being preferred and with fluorine and chlorine being more preferred.

It is to be understood that the substituent R4 of the phenyl ring R3 can be attached in 2-position, 3-position or 4-position to the phenyl ring, preferably in 3- or 4-position to the phenyl ring.

It is further to be understood that the substituents R4 and R5 of the phenyl ring R3 can be attached in 2- and 3-position, in 2- and 4-position, in 2- and 5-position, in 2- and 6-position, 3- and 4- position, in 3-and 5-position and in 3- and 6-position to the phenyl ring. Preferably, the substituents R4 and R5 can be attached in 3- and 4-position to the phenyl ring.

If the phenyl ring R3 is substituted by R4, R5 and R6 these substituents can be attached in 2-, 3- and 4-positon, in 2-, 3- and 5-position, in 2-, 3- and 6-position, in 2- , 4- and 5-position, in 2-, 4- and 6-position, in 2-, 5- and 6-position, in 3-, 4- and 5-position, in 3-, 4- and 6-position, in 3- , 5- and 6-position and in 4-, 5- and 6-position to the phenyl ring. Preferably, the substituents R4, R5 and R6 can be attached in 3-, 4- and 5-position, in 2-, 3- and 4-position or in 2-, 4- and 5-position to the phenyl ring.

Exemplary phenyl rings R3 substituted by R4 and R5 or substituted by R4, R5 and R6, which may be mentioned, are 3-fluoro-4-methoxy-phenyl, 3-fluoro-4-methyl-phenyl, 3,5-difluoro-4-methoxy-phenyl, 2,3-difluoro-4-methoxy-phenyl or 2-fluoro-4,5-dimethoxy-phenyl.

It is further to be understood that the substituent R8 of the phenyl ring R7 can be attached in 2-, 3- or 4- position to the phenyl ring, preferably, R8 can be attached in 2- or in 4-position to the phenyl ring. Furthermore, if the phenyl ring R7 is substituted by R8 and R9, these substitutents can be attached in 2- and 3-position, in 2- and 4-position, in 2- and 5-position, in 2- and 6-position, 3- and 4- position, in 3-and 5-position and in 3- and 6-position to the phenyl ring. Preferably, R8 and R9 can be attached in 2-and 4- position or in 2- and 5-position to the phenyl ring, more preferably R8 and R9 can be attached in 2- and 4-position to the phenyl ring. In case, R8 and R9 form a 1-2C-alkylenedioxygroup, this group can be attached in 2,3-position or in 3,4-position, preferably in 3,4-position, to the phenyl ring. Exemplary phenyl rings R7 substituted by R8 or substituted by R8 and R9, which may be listed are 2-fluoro-phenyl, 2-methoxy-phenyl, 4-fluoro-phenyl, 4-fluoro-2-methyl-phenyl, 4-fluoro-2-methoxy-phenyl, 5-fluoro-2-methoxy-phenyl, 2,5-dimethoxy-phenyl or 3,4-methylenedioxy-phenyl.

"Stereoisomer" as part of the phrase "or a stereoisomer of the compound" is meant to mean that in the compounds of formula (1), in case A represents S(O), two stereoisomers exist and both of these two stereoisomers are included within the scope of the invention (respectively within the scope of the particular claim). The absolute configuration of the compounds of formula (1) at the stereogenic centers in positions 4a and 10b is fixed and is R in position 4a and R in position 10b.

In a preferred embodiment, the invention relates to a compound of formula (1), wherein

| | |
|---|---|
| A | is S, S(O) or $S(O)_2$, |
| X | is C(O) and is attached either in meta or para position to the phenyl, |
| R1 | is 1-4C-alkoxy or 1-4C-alkoxy predominantly or completely substituted by fluorine, |
| R2 | is 1-4C-alkoxy or 1-4C-alkoxy predominantly or completely substituted by fluorine, or |
| R1 and R2 | together form a 1-2C-alkylenedioxy group, |
| R3 | is unsubstituted phenyl, phenyl substituted by R4 and R5 or phenyl substituted by R4, R5 and R6, wherein |

R4 is 1-4C-alkyl, 1-4C-alkoxy or halogen,
R5 is 1-4C-alkoxy or halogen and
R6 is 1-4C-alkoxy or halogen,

R7 is unsubstituted phenyl, phenyl substituted by R8 or phenyl substituted by R8 and R9, wherein R8 is 1-4C-alkoxy or halogen and R9 is 1-4C-alkyl, 1-4C-alkoxy or halogen, or R8 and R9 together form a 1-2C-alkylenedioxy group,

or a stereoisomer of the compound.

In another preferred embodiment, the invention relates to a compound of formula (1), wherein

A is S, S(O) or $S(O)_2$,
X is C(O) and is attached either in meta or para position to the phenyl, preferably in para position to the phenyl,
R1 is 1-2C-alkoxy or 1-2C-alkoxy predominantly or completely substituted by fluorine,
R2 is 1-2C-alkoxy or 1-2C-alkoxy predominantly or completely substituted by fluorine, or
R1 and R2 together form a 1-2C-alkylenedioxy group,
R3 is unsubstituted phenyl, phenyl substituted by R4 and R5 or phenyl substituted by R4, R5 and R6, wherein R4 is 1-4C-alkyl, 1-4C-alkoxy or halogen, R5 is 1-4C-alkoxy or halogen and R6 is 1-4C-alkoxy or halogen,
R7 is unsubstituted phenyl, phenyl substituted by R8 or phenyl substituted by R8 and R9, wherein R8 is 1-2C-alkoxy, fluorine, chlorine or bromine, preferably fluorine or chlorine, and R9 is 1-2C-alkyl, 1-2C-alkoxy, fluorine, chlorine or bromine, preferably fluorine or chlorine, or R8 and R9 together form a 1-2C-alkylenedioxy group,

or a stereoisomer of the compound.
In yet another preferred embodiment, the invention relates to a compound of formula (1), wherein

A is S, S(O) or $S(O)_2$,
X is C(O) and is attached either in meta or para position to the phenyl, preferably in para position to the phenyl,
R1 is ethoxy,
R2 is methoxy,
R3 is unsubstituted phenyl, phenyl substituted by R4 and R5 or phenyl substituted by R4, R5 and R6, wherein R4 is 1-2C-alkyl, 1-2C-alkoxy, fluorine, chlorine or bromine, preferably fluorine or chlorine, R5 is 1-2C-alkoxy, fluorine, chlorine or bromine, preferably fluorine or chlorine and R6 is 1-2C-alkoxy, fluorine, chlorine or bromine, preferably fluorine or chlorine,
R7 is unsubstituted phenyl, phenyl substituted by R8 or phenyl substituted by R8 and R9, wherein R8 is methoxy or fluorine and R9 is methyl, methoxy or fluorine or R8 and R9 together form a methylenedioxy group, or a stereoisomer of the compound.

In yet another preferred embodiment, the invention relates to a compound of formula (1), wherein

A is S, S(O) or $S(O)_2$,
X is C(O) and is attached either in meta or para position to the phenyl, preferably in para position to the phenyl,
R1 is ethoxy,
R2 is methoxy,
R3 is phenyl substituted by R4 and R5 or phenyl substituted by R4, R5 and R6, wherein R4 is methyl, methoxy or fluorine, R5 is methoxy or fluorine and R6 is methoxy or fluorine,
R7 is unsubstituted phenyl, phenyl substituted by R8 or phenyl substituted by R8 and R9, wherein R8 is methoxy or fluorine, R9 is methyl, methoxy or fluorine, or R8 and R9 together form a methylenedioxy group,

or a stereoisomer of the compound.

In a further preferred embodiment, the invention relates to a compound of formula (1), wherein A is S, and X, R1, R2, R3 and R7 are as defined above.

In a further preferred embodiment, the invention relates to a compound of formula (1), wherein A is S, X, R1, R2 and R7 are as defined above and R3 is unsubstituted phenyl.

In a further preferred embodiment, the invention relates to a compound of formula (1), wherein A is S, X is C(O) and is attached either in meta or para position to the phenyl, preferably in para position to the phenyl, R1 is ethoxy, R2 is methoxy and R3 as well as R7 represent an unsubstituted phenyl.

In a further preferred embodiment, the invention relates to a compound of formula (1), wherein A is S, X is C(O) and is attached either in meta or para position to the phenyl, preferably in para position to the phenyl, R1 is ethoxy, R2

is methoxy, R3 is unsubstituted phenyl and R7 is phenyl substituted either by R8 or substituted by R8 and R9, wherein R8 and R9 are defined as above. If, in this case R7 is phenyl substituted by R8 and R9, R8 preferably represents 1-2C-alkoxy, more preferably methoxy, and R9 preferably represents fluorine, chlorine or bromine, more preferably fluorine. R8 and R9 are preferably attached in 2- and 4-position to the phenyl ring.

In a further preferred embodiment, the invention relates to a compound of formula (1), wherein A is S, X, R1, R2 and R7 are as defined above and R3 is phenyl substituted either by R4 and R5 or by R4, R5 and R6, wherein R4, R5 and R6 are as defined above.

In a further preferred embodiment, the invention relates to a compound of formula (1), wherein A is S, X is C(O) and is attached either in meta or para position to the phenyl, preferably in para position to the phenyl, R1 is ethoxy, R2 is methoxy, R3 is phenyl substituted either by R4 and R5 or by R4, R5 and R6, wherein R4, R5 and R6 are as defined above and R7 represents unsubstituted phenyl. If, R3 is substituted by R4 and R5, R4 and R5 independently of each other represent 1-2C-alkoxy, 1-2C-alkyl, fluorine, chlorine or bromine, preferably methoxy, methyl or fluorine. More preferably, R4 represents fluorine and R5 either represents methoxy or methyl. Furthermore, R4 and R5 are preferably attached in 3- and 4-position to the phenyl ring. If, R3 is substituted by R4, R5 and R6, R4, R5 and R6 inde-pendently of each other represent 1-2C-alkoxy, fluorine, chlorine or bromine, preferably methoxy or fluorine. More preferably, either two out of the substituents R4, R5 and R6 represent fluorine and the third substituent is methoxy or two out of the substituents R4, R5 and R6 represent methoxy and the third substituent is fluorine. R4, R5 and R6 are preferably attached in 3-, 4- and 5-position, in 2-, 3- and 4-position or in 2-, 4- and 5-position to the phenyl ring.

In a further preferred embodiment, the invention relates to a compound of formula (1), wherein A is S, X is C(O) and is attached either in meta or para position to the phenyl, preferably in para position to the phenyl, R1 is ethoxy, R2 is methoxy, R3 is phenyl substituted either by R4 and R5 or by R4, R5 and R6 and R7 represents phenyl either substituted by R8 or by R8 and R9, wherein R4, R5, R6 , R8 and R9 are as defined above.

In a further preferred embodiment, the invention relates to a compound of formula (1), wherein A is S, X is C(O) and is attached either in meta or para position to the phenyl, preferably in para position to the phenyl, R1 is ethoxy, R2 is methoxy, R3 is preferably substituted by R4 and R5 and R7 is substituted by R8. R4 and R5 independently of each other represent 1-2C-alkoxy, fluorine, chlorine or bromine, preferably methoxy or fluorine, and R8 represents fluorine, chlorine, bromine or 1-2C-alkoxy, preferably fluorine or methoxy. More preferably, R4 is fluorine and R5 is methoxy. The substituents R4 and R5 are preferably attached in 3- and 4-position to the phenyl ring and the substituent R8 is preferably attached in 2- or 4-position to the phenyl ring.

In a further preferred embodiment, the invention relates to a compound of formula (1), wherein A is S, X is C(O) and is attached either in meta or para position to the phenyl, preferably in para position to the phenyl, R1 is ethoxy, R2 is methoxy, R3 is either substituted by R4 and R5 or by R4, R5 and R6 and R7 is substituted by R8 and R9. If, R3 is substituted by R4 and R5, R4 and R5 independently of each other represent 1-2C-alkoxy, 1,2-C-alkyl, fluorine, chlorine or bromine, preferably methoxy, methyl or fluorine. More preferably, one of the substituents R4 or R5 is fluorine and the other substituent represents methoxy or methyl, preferably methoxy. R4 and R5 are preferably attached in 3- and 4-position to the phenyl ring. If, R3 is substituted by R4, R5 and R6, R4, R5 and R6 independently of each other represent 1-2C-alkoxy, fluorine, chlorine or bromine, preferably methoxy or fluorine. More preferably, two out of the substituents R4, R5 and R6 represent fluorine and the third substituent is methoxy. R4, R5 and R6 are preferably attached in 2-, 3- and 4-position or in 3-, 4- and 5-position to the phenyl ring. The substituents R8 and R9 independently of each other represent 1-2C-alkoxy, 1-2C-alkyl, fluorine, chlorine or bromine, or R8 and R9 together form a 1-2C-alkylenedioxy group. Preferably, R8 and R9 independently of each other represent methoxy, methyl or fluorine, or R8 and R9 preferably together form a methylenedioxy group. R8 and R9 are preferably attached in 2- and 4-position or in 2- and 5-position to the phenyl ring. If R8 and R9 together for a 1-2C-alkylenedioxy group the correspondent attachment preferably occurs in 3- and 4-position to the phenyl ring.

In yet a further preferred embodiment, the invention relates to a compound of formula (1) or a stereoisomer thereof, wherein A is S(O) and X, R1, R2, R3 and R7 are as defined above.

In a further preferred embodiment, the invention relates to a compound of formula (1) or a stereoisomer thereof, wherein A is S(O), X, R1, R2 and R7 are as defined above and R3 is unsubstituted phenyl.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy and R3 as well as R7 represent an unsubstituted phenyl.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy, R3 is unsubstituted phenyl and R7 is phenyl substituted either by R8 or by R8 and R9, wherein R8 and R9 are defined as above.

In a further preferred embodiment, the invention relates to a compound of formula (1) or a stereoisomer thereof, wherein A is S(O), X, R1, R2 and R7 are as defined above and R3 is phenyl substituted either by R4 and R5 or by R4, R5 and R6, wherein R4, R5 and R6 are as defined above.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy, R3 is phenyl substituted either by R4 and R5 or by R4, R5 and R6, wherein R4, R5 and R6 are as defined above and R7 represents unsub-stituted phenyl.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy, R3 is phenyl substituted either by R4 and R5 or by R4, R5 and R6 and R7 represents phenyl either substituted by R8 or by R8 and R9, wherein R4, R5, R6, R8 and R9 are as defined above.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy, R3 is preferably substituted by R4 and R5 and R7 is substituted by R8, wherein R4, R5 and R8 are as defined above.

In a further preferred embodiment, the invention relates to a compound of formula (1) or a stereoisomer thereof, wherein A is S(O), X is C(O) and is attached either in meta or para position to the phenyl, preferably in para position to the phenyl, R1 is ethoxy, R2 is methoxy, R3 is either substituted by R4 and R5 or by R4, R5 and R6 and R7 is substituted by R8 and R9, wherein R4, R5, R6, R8 and R9 are as defined above.

In yet a further preferred embodiment, the invention relates to a compound of formula (1), wherein A is $S(O)_2$ and X, R1, R2, R3 and R7 are as defined above.

In a further preferred embodiment, the invention relates to a compound of formula (1), wherein A is $S(O)_2$, X, R1, R2 and R7 are as defined above and R3 is unsubstituted phenyl.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy and R3 as well as R7 represent an unsubstituted phenyl.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy, R3 is unsubstituted phenyl and R7 is phenyl substituted either by R8 or by R8 and R9, wherein R8 and R9 are defined as above.

In a further preferred embodiment, the invention relates to a compound of formula (1), wherein A is $S(O)_2$, X, R1, R2 and R7 are as defined above and R3 is phenyl substituted either by R4 and R5 or by R4, R5 and R6, wherein R4, R5 and R6 are as defined above.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy, R3 is phenyl substituted either by R4 and R5 or by R4, R5 and R6, wherein R4, R5 and R6 are as defined above and R7 represents unsubstituted phenyl.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy, R3 is phenyl substituted either by R4 and R5 or by R4, R5 and R6 and R7 represents phenyl either substituted by R8 or by R8 and R9, wherein R4, R5, R6, R8 and R9 are as defined above.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy, R3 is preferably substituted by R4 and R5 and R7 is substituted by R8, wherein R4, R5 and R8 are as defined above.

In a further preferred embodiment, the invention relates to a compound of formula (1), wherein A is $S(O)_2$, X is C(O) and is attached either in meta or para position to the phenyl, preferably in para position to the phenyl, R1 is ethoxy, R2 is methoxy, R3 is either substituted by R4 and R5 or by R4, R5 and R6 and R7 is substituted by R8 and R9, wherein R4, R5, R6, R8 and R9 are as defined above.

In a further preferred embodiment, the invention relates to a compound of formula (1) or a stereoisomer thereof, wherein A is S, S(O) or $S(O)_2$, X is C(O) and is attached in meta or para position to the phenyl, preferably in para position to the phenyl, R3 represents phenyl either substituted by R4 and R5 or by

R4, R5 and R6, R7 represents unsubstituted phenyl and R1, R2, R4, R5 and R6 are as defined above.

In a further preferred embodiment, the invention relates to a compound of formula (1) or a stereoisomer thereof, wherein A is S, S(O) or $S(O)_2$, X is C(O) and is attached in meta or para position to the phenyl, preferably in para position to the phenyl, R3 represents phenyl either substituted by R4 and R5 or by R4, R5 and R6, R7 represents phenyl either substituted by R8 or by R8 and R9 or R8 and R9 together form a 1-2C-alkylenedioxy group, preferably a methylenedioxy group, and R1, R2, R4, R5, R6, R8 and R9 are as defined above.

The compounds of formula (1) include stereogenic centers in the positions 4a and 10b of the thiophenanthridine ring system. An additional stereogenic center can arise in position 2 of the thiophenanthridine ring system, in case A represents S(O). The absolute configuration at the stereogenic centers in positions 4a and 10b is fixed and is R in

position 4a and R in position 10b ("R" and "S" nomenclature according to the rules of Cahn, Ingold and Prelog).

Accordingly, only one stereoisomer exists in case A represents S or $S(O)_2$. The absolute configuration at the sterogenic centers 4a and 10b of this stereoisomer is R in position 4a and R in position 10b.

Two stereoisomers exist, in case A represents S(O). The absolute configuration at the stereogenic centers 2, 4a and 10b can be (2R, 4aR, 10bR) or (2S, 4aR, 10bR). The numbering of the thiophenanthridine ring system is shown in the below formula 1*.

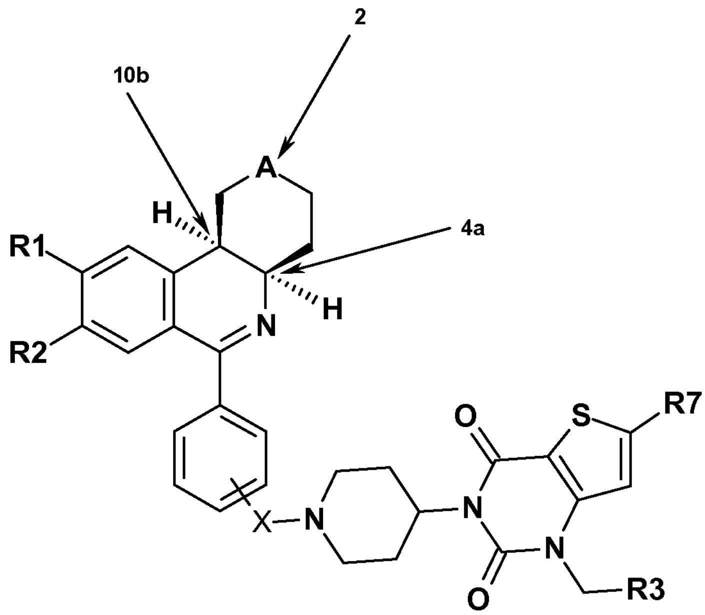

(1*)

The invention further includes the pure stereoisomers mentioned above, as well as all mixtures of the stereoisomers mentioned above, independent of the ratio.

The compounds according to the invention can be prepared according to reaction schemes 1 to 6.

As shown in reaction scheme 1 the compounds of formula 1, wherein A, R1, R2 and R3 have the above-mentioned meanings and R7 is unsubstituted phenyl, can be prepared by coupling a benzoic acid compound of formula 2, wherein A, R1 and R2 have the above-mentioned meanings, with a secondary amine of formula 3, wherein R3 has the above-mentioned meanings and R7 represents unsubstituted phenyl using any standard amide bond coupling method, such as for example the use of coupling agents like HBTU, COMU or HATU or the use of activated acid compounds, like acid anhydrides or esters. A review of suitable amide bond coupling methods can be found, for example, in C. A. G. N. Montalbetti, V. Falque, Tetrahedron, 61 (2005), 10827-10852.

The preparation of tert-butyl 4-(2,4-dioxo-6-phenyl-1,4-dihydrothieno[3,2-d]pyrimidin-3(2H)-yl)piperidine-1-carboxylate (reaction Scheme 2: Compound B2; experimental part - example B2) is described in reaction scheme 2 as well as in the experimental part of this application.

In a first step methyl 3-amino-5-phenylthiophene-2-carboxylate (6) is reacted with triphosgene followed by tert-butyl 4-aminopiperidine-1-carboxylate in an appropriate solvent such as dichloromethane, chloroform, tetrahydrofuran, acetonitrile, N,N-dimethylformamide or dimethyl sulfoxide at 0-5°C, preferably at 0°C. Treatment of the resulting tert-butyl 4-({[2-(methoxycarbonyl)-5-phenyl-3-thienyl] carbamoyl}amino)piperidine-1-carboxylate (Experimental part - example B1; reaction scheme 2: Compound B1) with sodium methoxide in an appropriate solvent such as methanol at elevated temperature, preferably under reflux conditions, yields tert-butyl 4-(2,4-dioxo-6-phenyl-1,4-dihydrothieno[3,2-d]pyrimidin-3(2H)-yl)piperidine-1-carboxylate (Experimental part - example B2; reaction Scheme 2: Compound B2).

The N-tert-butyloxycarbonyl protected compounds of formula 4, wherein R3 has the above-mentioned meanings and R7 is unsubstituted phenyl can be prepared by reacting tert-butyl 4-(2,4-dioxo-6-phenyl-1,4-dihydrothieno[3,2-d]pyrimidin-3(2H)-yl)piperidine-1-carboxylate (Experimental Part- Example B2; reaction Scheme 1: Compound B2) with a compound of formula 5, wherein R3 has the above-mentioned meanings and LG stands for a suitable leaving group, such as for example a halide, preferably chlorine or bromine, or a mesyl or tosyl group. The reaction is carried out in an appropriate solvent such as dimethyl sulfoxide or N,N-dimethylformamide in the presence of a base, such as for example potassium carbonate, sodium carbonate, diisopropylethylamine or triethylamine and preferably at elevated temperature in the range of 80 - 100°C, more preferably at 100 °C. The compounds of formula 5 are commercially available or can be prepared according to procedures known in the art or in analogy thereto.

**Reaction Scheme 1:**

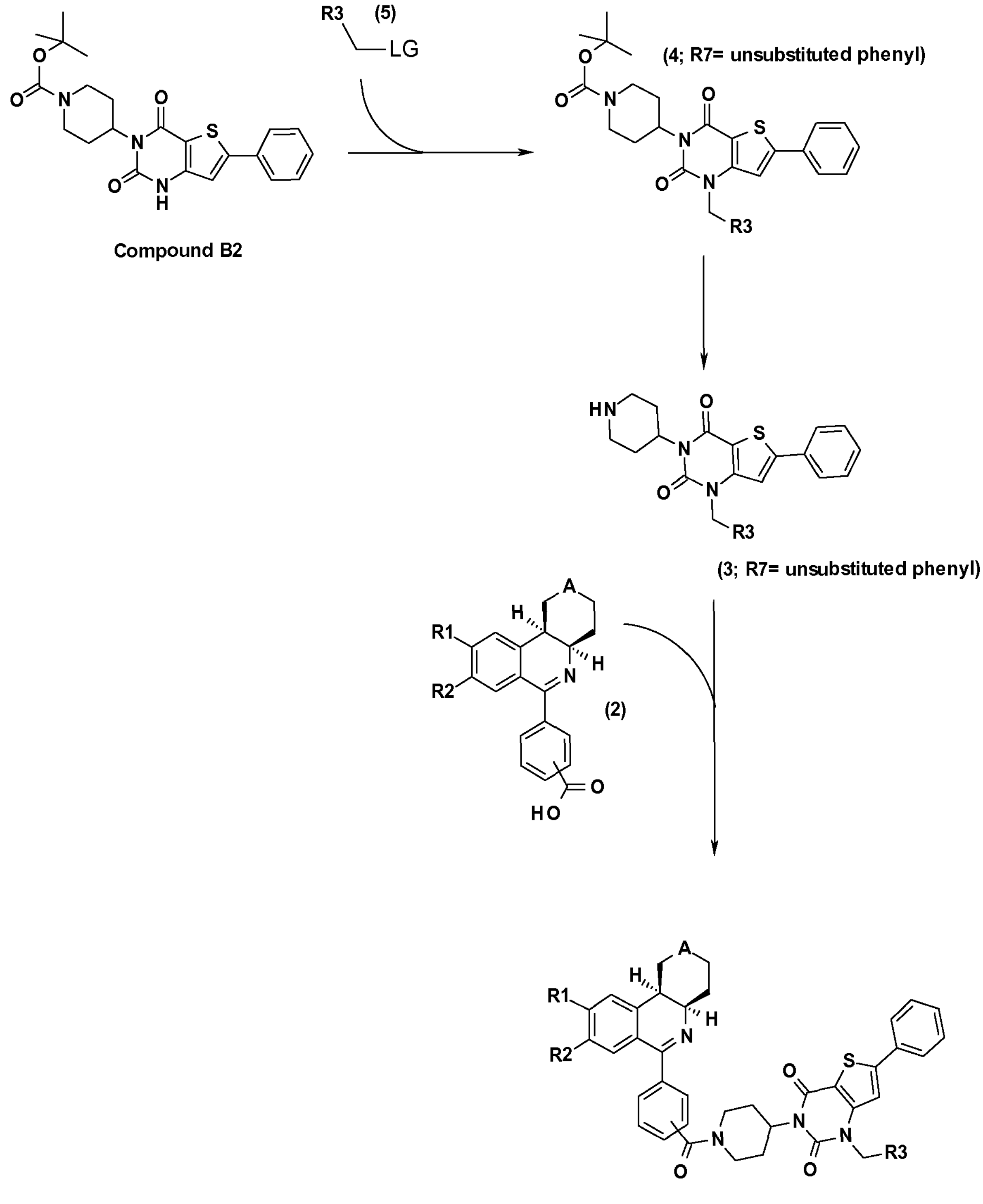

Compound B2

(5)

(4; R7= unsubstituted phenyl)

(3; R7= unsubstituted phenyl)

(2)

(1; R7= unsubstituted phenyl)

**Reaction Scheme 2:**

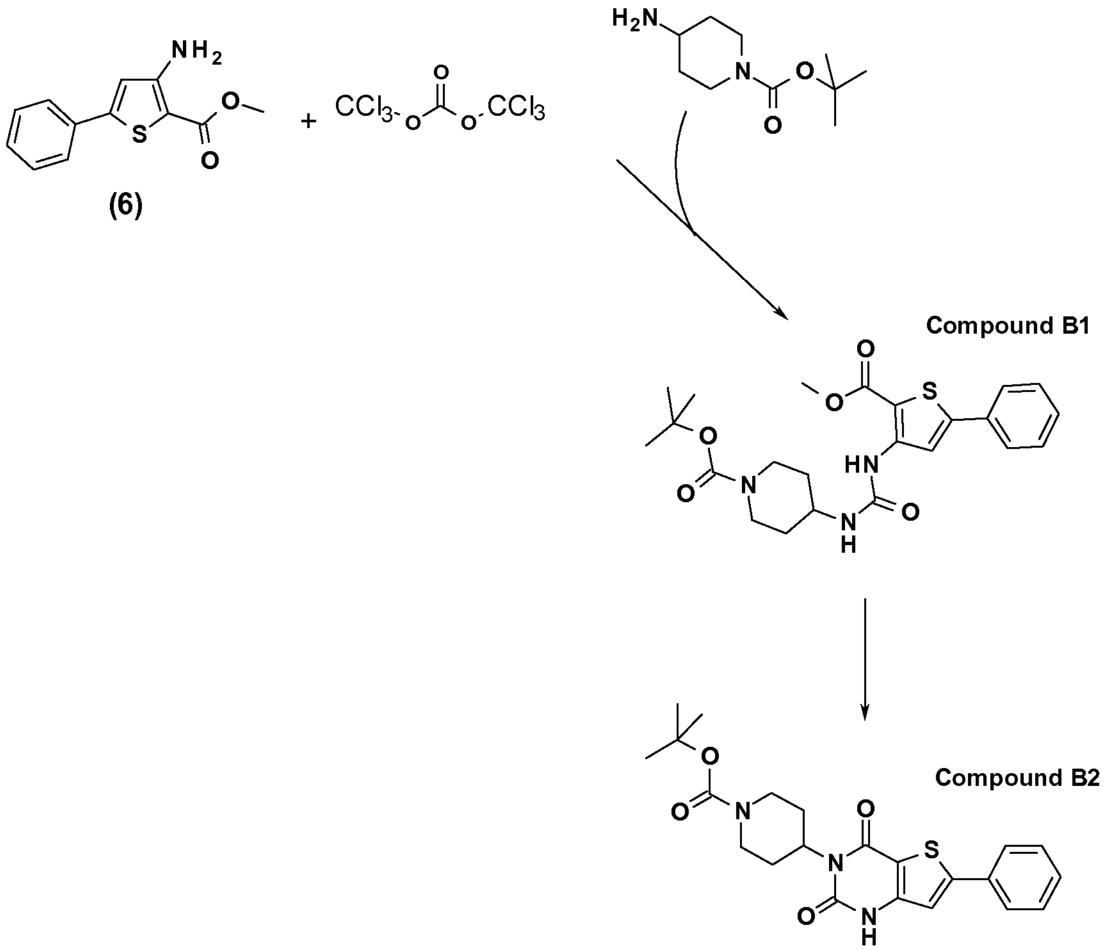

(6)

Compound B1

Compound B2

The secondary amine of formula 3, wherein R3 has the above mentioned meanings and R7 is unsubstituted phenyl can be prepared from the corresponding N-tert-butyloxycarbonyl protected compounds of formula 4 by using standard conditions for the removal of the tert-butyloxycarbonyl group, such as for example hydrogen chloride or trifluoroacetic acid in an appropriate solvent, such as dioxane or dichloromethane, and if necessary, in the presence of a cation scavenger, such as for example anisole or thiophenol. Additional alternative reaction conditions for the removal of the tert-butyloxycarbonyl group can be found, for example, in Greene and Wuts, Protective Groups in Organic Synthesis, 3rd edition, John Wiley & Sons, New York 1999.

The preparation of the benzoic acid compounds of formula 2 is described below in reaction scheme 5.

In reaction scheme 3 an alternative route of preparation for compounds of formula 1, wherein A, R1, R2 and R3 have the above-mentioned meanings and R7 is unsubstituted phenyl is described.

In addition, the route of preparation described in reaction scheme 3 is in particular suitable for the preparation of compounds of formula 1, wherein A, R1, R2 and R3 have the above-mentioned meanings and R7 is phenyl substituted by R8 or phenyl substituted by R8 and R9.

According to reaction scheme 3 compounds of formula 1, wherein A, R1, R2 R3 and R7 have the above-mentioned meanings can be prepared by coupling a benzoic acid compound of formula 2, wherein A, R1 and R2 have the above-mentioned meanings, with a secondary amine of formula 3, wherein R3 has the above-mentioned meanings and R7 is either unsubstituted phenyl or phenyl substituted by R8 or phenyl substituted by R8 and R9, using any standard amide bond coupling method, such as for example the use of coupling agents like COMU, HBTU and HATU or the use of activated acid compounds, like acid anhydrides or esters.

The preparation of the secondary amine of formula 3 wherein R3 has the above meanings and R7 is phenyl substituted by R8 or phenyl substituted by R8 and R9 can be prepared according to the above described procedure for the preparation of a secondary amine of formula 3, wherein R7 represents unsubstituted phenyl.

The N-tert-butyloxycarbonyl protected compounds of formula 4, wherein R3 and R7 have the above-mentioned meanings can be prepared, for example, by using a palladium catalyzed coupling reaction:

Compounds of formula 8, wherein R3 has the above-mentioned meanings are reacted with a phenyl boronic acid or a phenyl boronic acid ester of formula 7, wherein R7 has the above-mentioned meanings and R may be hydrogen, 1-4C-alkyl or the two R groups may form together an alkylene bridge optionally further substituted by methyl groups (for example forming a pinacol ester), in an inert solvent, such as for example 1,2-dimethoxyethane or 1,4-dioxane in presence of an aqueous solution of a base, such as for example potassium carbonate, cesium carbonate or potassium phosphate, and a palladium catalyst, such as for example dichlorobis(tricyclohexylphosphine)palladium, at a temperature in the range from 60 °C to 160 °C, preferably at about 150 °C, and additionally, under microwave irradiation.

The N-tert-butyloxycarbonyl protected compounds of formula 8 can be prepared as described in reaction scheme 1 by reacting tert-butyl 4-(6-bromo-2,4-dioxo-1,4-dihydrothieno[3,2-d]pyrimidin-3(2H)-yl)piperidine-1-carboxylate (Experimental Part - Example B5; Reaction scheme 3: Compound B5,) with a compound of formula 5, wherein R3 has the above-mentioned meanings and LG stands for a suitable leaving group, such as for example a halide, preferably chlorine or bromine, or a mesyl or tosyl group. The reaction is carried out in an appropriate solvent such as N,N-dimethylformamide in the presence of a base, such as for example potassium carbonate, sodium carbonate, diisopropylethylamine or triethylamine and preferably at elevated temperature in the range of 80-100°C, preferably at 100 °C.

**Reaction Scheme 3:**

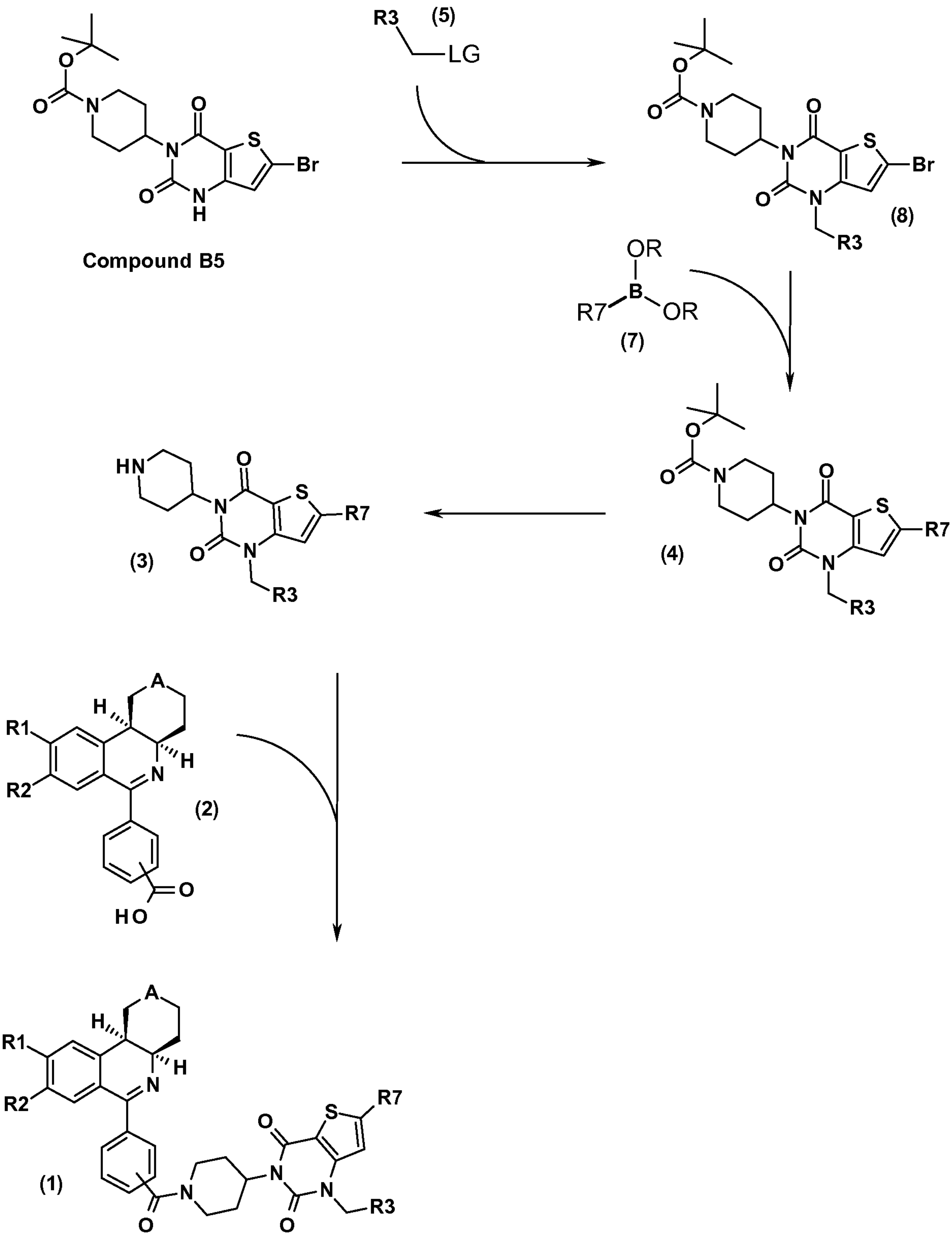

Compound B5

(5)

(8)

(7)

(4)

(3)

(2)

(1)

Not explicitly shown in reaction scheme 3 is a further alternative method for the preparation of compounds of formula (4). This alternative method also starts with compound B5, but the sequence of introduction of the R3-$CH_2$-group and the R7 group is inverted in comparison to reaction scheme 3. Suitable reaction conditions for this alternative method are described in the experimental part in the description of the preparation of compound B6.

The compounds of formulae (3) and (4) (formulae depicted in reaction scheme 3), wherein R3 either represents unsubstituted phenyl or phenyl substituted by R4, phenyl substituted by R4 and R5 or phenyl substituted by R4, R5 and R6 and R7 either represents a phenyl substituted by R8, phenyl substituted by R8 and R9 or unsubstituted phenyl and R8 and R9 have the meanings as described above are key intermediates for the synthesis of compounds according to the formula (1). It is to be understood that all (preferred) attachments of the substituents R4, R5, R6, R8 and R9 to the correspondent phenyl rings as well as their (preferred) meanings as described above for the compounds of formula (1) also applies to the compounds of formulae (3) and (4). Consequently, another aspect of the invention relates to a compound of formula (3) and salts thereof and to a compound of formula (4) and their use for the synthesis of compounds of formula (1). Examples of formulae (3) and (4), which may be mentioned, are shown in the experimental part, i.e. compounds B8 to B43:

Tert-butyl 4-[1-(3-fluoro-4-methoxybenzyl)-2,4-dioxo-6-phenyl-1,4-dihydrothieno[3,2-d]pyrimidin-3(2H)-yl]piperidine-1-carboxylate; tert-butyl 4-[1-(3,5-difluoro-4-methoxybenzyl)-2,4-dioxo-6-phenyl-1,4-dihydrothieno[3,2-d]pyrimidin-3(2H)-yl]piperidine-1-carboxylate; tert-butyl 4-[1-(2,3-difluoro-4-methoxy-benzyl)-2,4-dioxo-6-phenyl-1,4-dihydrothieno[3,2-d]pyrimidin-3(2H)-yl]piperidine-1-carboxylate; tert-butyl 4-[6-(4-fluoro-2-methoxyphenyl)-1-(3-fluoro-4-methylbenzyl)-2,4-dioxo-1,4-dihyd rothieno[3,2-d]pyrimidin-3(2H)-yl]piperidine-1-carboxylate; tert-butyl 4-[1-benzyl-6-(4-fluoro-2-methoxyphenyl)-2,4-dioxo-1,4-dihydrothieno[3,2-d]pyrimidin-3(2H)-yl]piperidine-1-carboxylate; tert-butyl 4-[1-(3-fluoro-4-methoxybenzyl)-6-(4-fluoro-2-methylphenyl)-2,4-dioxo-1,4-dihydrothieno[3,2-d]pyrimidin-3(2H)-yl] piperidine-1-carboxylate; tert-butyl 4-[1-(3-fluoro-4-methoxybenzyl)-6-(2-methoxyphenyl)-2,4-dioxo-1,4-dihydrothieno[3,2-d]pyrimidin-3(2H)-yl]piperidine-1-carboxylate; tert-butyl 4-[6-(1,3-benzodioxol-5-yl)-1-(3-fluoro-4-methoxybenzyl)-2,4-dioxo-1,4-dihydrothieno[3,2-d]pyrimidin-3(2H)-yl]piperidine-1-carboxy-late; tert-butyl 4-[1-(3-fluoro-4-methoxybenzyl)-6-(4-fluoro-2-methoxyphenyl)-2,4-dioxo-1,4-dihydro-thieno [3,2-d]pyrimidin-3(2H)-yl]piperidine-1-carboxylate; tert-butyl 4-[1-(3-fluoro-4-methoxybenzyl)-6-(5-fluoro-2-methoxyphenyl)-2,4-dioxo-1,4-dihydrothieno[3,2-d]pyrimidin-3(2H)-yl]piperidine-1-carboxylate; tert-butyl 4-[6-(2,5-dimethoxyphenyl)-1-(3-fluoro-4-methoxybenzyl)-2,4-dioxo-1,4-dihydrothieno[3,2-d]pyrimidin-3(2H)-yl]piperidine-1-carboxylate; tert-butyl 4-[1-(3-fluoro-4-methoxy-benzyl)-6-(2-fluorophenyl)-2,4-dioxo-1,4-dihydrothieno[3,2-d] pyrimidin-3(2H)-yl]piperidine-1-carboxylate; tert-butyl 4-[1-(3-fluoro-4-methoxybenzyl)-6-(4-fluorophenyl)-2,4-dioxo-1,4-dihydro-thieno[3,2-d] pyrimidin-3(2H)-yl]piperidine-1-carboxylate; 1-(3-Fluoro-4-methoxybenzyl)-6-phenyl-3-piperidin-4-ylthieno[3,2-d] pyrimidine-2,4(1H,3H)-dione hydrochloride; 1-(3,5-Difluoro-4-methoxy-benzyl)-6-phenyl-3-(piperidin-4-yl)thieno[3,2-d]pyrimidine-2,4(1H,3H)-dione hydrochloride; 1-(2,3-Difluoro-4-methoxybenzyl)-6-phenyl-3-(piperidin-4-yl)thieno[3,2-d]pyrimidine-2,4(1 H,3H)-dione hydrochloride; 6-(4-Fluoro-2-methoxy-phenyl)-1-(3-fluoro-4-methylbenzyl)-3-(piperidin-4-yl)thieno[3,2-d]pyrimidine-2,4(1H,3H)-dione hydro-chloride; 1-Benzyl-6-(4-fluoro-2-methoxyphenyl)-3-(piperidin-4-yl)thieno[3,2-d]pyrimidine-2,4(1H,3H)-dione hydrochloride; 1-(3-Fluoro-4-methoxybenzyl)-6-(4-fluoro-2-methylphenyl)-3-(piperidin-4-yl) thieno[3,2-d]pyrimidine-2,4(1H,3H)-dione hydrochloride; 1-(3-Fluoro-4-methoxybenzyl)-6-(2-methoxyphenyl)-3-(piperidin-4-yl)thieno[3,2-d]pyrimidine-2,4(1H,3H)-dione hydrochloride; 6-(1,3-Benzodioxol-5-yl)-1-(3-fluoro-4-methoxybenzyl)-3-(piperidin-4-yl)thieno[3,2-d]pyrimidine-2,4(1H,3H)-dione hydrochloride; 1-(3-Fluoro-4-methoxybenzyl)-6-(4-fluoro-2-methoxy-phenyl)-3-(piperidin-4-yl)thieno[3,2-d]pyrimidine-2,4(1H,3H)-dione hydrochloride; 1-(3-Fluoro-4-methoxybenzyl)-6-(5-fluoro-2-methoxyphenyl)-3-(piperidin-4-yl)thieno[3,2-d]pyrimidine-2,4(1H,3H)-dione hydrochloride; 6-(2,5-Dimethoxyphenyl)-1-(3-fluoro-4-methoxybenzyl)-3-(piperidin-4-yl)thieno-[3,2-d]pyrimidine-2,4(1H,3H)-dione hydrochloride; 1-(3-Fluoro-4-methoxybenzyl)-6-(2-fluorophenyl)-3-(piperidin-4-yl)thieno[3,2-d] pyrimidine-2,4(1H,3H)dione hydrochloride; 1-(3-Fluoro-4-methoxybenzyl)-6-(4-fluorophenyl)-3-(piperidin-4-yl)thieno[3,2-d]pyrimidine-2,4(1H,3H)-dione hydrochloride; 1-(2-Fluoro-4,5-dimethoxybenzyl)-6-phenyl-3-(piperidin-4-yl)thieno[3,2-d]pyrimidine-2,4(1H,3H)-dione hydrochloride; tert-butyl 4-[1-(2-fluoro-4,5-dimethoxybenzyl)-2,4-dioxo-6-phenyl-1,4-dihydrothieno[3,2-d]pyrimidin-3(2H)-yl]piperidine-1-carboxylate; 1-(3-Fluoro-4-methylbenzyl)-6-phenyl-3-(piperidin-4-yl)thieno[3,2-d] pyrimidine-2,4(1H,3H)-dione hydrochloride; tert-butyl 4-[1-(3-fluoro-4-methylbenzyl)-2,4-dioxo-6-phenyl-1,4-dihydrothieno[3,2-d]pyrimidin-3(2H)-yl]piperidine-1-carboxylate; 1-Benzyl-6-phenyl-3-(piperidin-4-yl)thieno[3,2-d]pyrimidine-2,4(1H,3H)-dione hydrochloride; tert-butyl 4-(1-benzyl-2,4-dioxo-6-phenyl-1,4-dihydrothieno[3,2-d]pyrimidin-3(2H)-yl)piperidine-1-carboxylate; tert-butyl 4-[1-(3,5-difluoro-4-methoxybenzyl)-6-(4-fluoro-2-methoxyphenyl)-2,4-dioxo-1,4-dihydro-thieno[3,2-d]-pyrimidin-3(2H)-yl]piperidine-1-carboxylate; 1-(3,5-Difluoro-4-methoxybenzyl)-6-(4-fluoro-2-methoxy-phenyl)-3-(piperidin-4-yl)thieno[3,2-d]pyrimidine-2,4(1H,3H)-dione hydrochloride; tert-butyl 4-[1-(2,3-difluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate and 1-(2,3-Difluoro-4-methoxybenzyl)-6-(4-fluoro-2-methoxyphenyl)-3-(piperidin-4-yl)thieno[3,2-d]pyrimidine-2,4(1H,3H)-dione hydrochloride.

Suitable salts for compounds of the formula 1 are all acid addition salts. Particular mention may be made of the inorganic and organic acids customarily used in pharmacy. Those suitable are watersoluble and water-insoluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, tolu-enesulphonic acid, methanesulphonic acid, 3-hydroxy-2-naphthoic acid or trifluoroacetic acid, the acids being employed in salt preparation - depending on whether a mono- or polybasic acid is concerned and depending on which salt is desired - in an equimolar quantitative ratio or one differing therefrom.

According to expert's knowledge the compounds of the invention as well as their salts may contain, e.g. when isolated in crystalline form, varying amounts of solvents. Included within the scope of the invention are therefore all solvates and in particular all hydrates of the compounds of formula I as well as all solvates and in particular all hydrates of the salts of the compounds of formula 1.

In reaction scheme 4 the synthesis of compound B5 is described. The intermediate methyl 3-amino-5-bromothiophene-2-carboxylate (compound B3) is obtained according to a procedure described in literature (Bioorganic & Medicinal Chemistry Letters 17 (2007) 2535-2539) by conversion of methyl 3-aminothiophene-2-carboxylate to methyl 3-[(trifluoroacetyl)amino]thiophene-2-carboxylate with trifluoroacetic acid anhydride followed by a lithiation / bromination sequence using n-butyllithium and 1,2-dibromoethane to give methyl 5-bromo-3-[(trifluoroacetyl)amino]thiophene-2-carboxylate, which is hydrolysed under basic conditions using potassium carbonate to obtain methyl 3-amino-5-bromothiophene-2-carboxylate (compound B3).

The synthesis of compound B5 is accomplished analogously as already described in reaction scheme 2:

Compound B3 is reacted with triphosgene followed by tert-butyl 4-aminopiperidine-1-carboxylate in an appropriate solvent such as dichloromethane, chloroform, tetrahydrofuran, acetonitrile, N,N-dimethylformamide or dimethyl sulfoxide at low temperature, preferably at 0°C. Treatment of the resulting tert-butyl 4-({[5-bromo-2-(methoxycarbonyl)thiophen-3-yl]carbamoyl}amino)piperidine-1-carboxylate (Experimental part - example B4; reaction scheme 4: Compound B4) with sodium methoxide in an appropriate solvent such as methanol at elevated temperature, preferably under reflux conditions yields tert-butyl 4-(6-bromo-2,4-dioxo-1,4-dihydrothieno[3,2-d]pyrimidin-3(2H)-yl)piperidine-1-carboxylate (Experimental part - example B5; reaction scheme 4: Compound B5).

In reaction scheme 5 the preparation of acid compounds of formula 2, wherein A is S and R1 and R2 have the above-mentioned meanings is shown.

The conversion of the tetrahydrothiopyranone derivative of formula 15, wherein R1 and R2 have the above-mentioned meanings with (1R)-1-(1-4C-alkoxy substituted or unsubstituted)arylethanamine, such as preferably (1R)-1-(4-methoxyphenyl)ethanamine or (1R)-1-phenylethanamine, is carried out according to standard procedures for condensation reactions known to the person skilled in the art, preferably in the presence of a suitable catalyst, for example p-toluenesulfonic acid, under water separation conditions in a suitable solvent, such as for example, n-hexane, benzene or toluene, at elevated temperatures, preferably at the boiling point of the solvent used.

The hydrogenation of the obtained imine/enamine of formulae 14a/14b, in which R1 and R2 have the above mentioned meanings is carried out according to standard methods known to the person skilled in the art, for example, in the presence of a platin on carbon catalyst using a suitable solvent, such as for example, methanol, ethanol, THF or 1,4-dioxane under a hydrogen pressure of about 100 mbar and at elevated temperatures, preferably between 40 and 80°C.

**Reaction Scheme 4:**

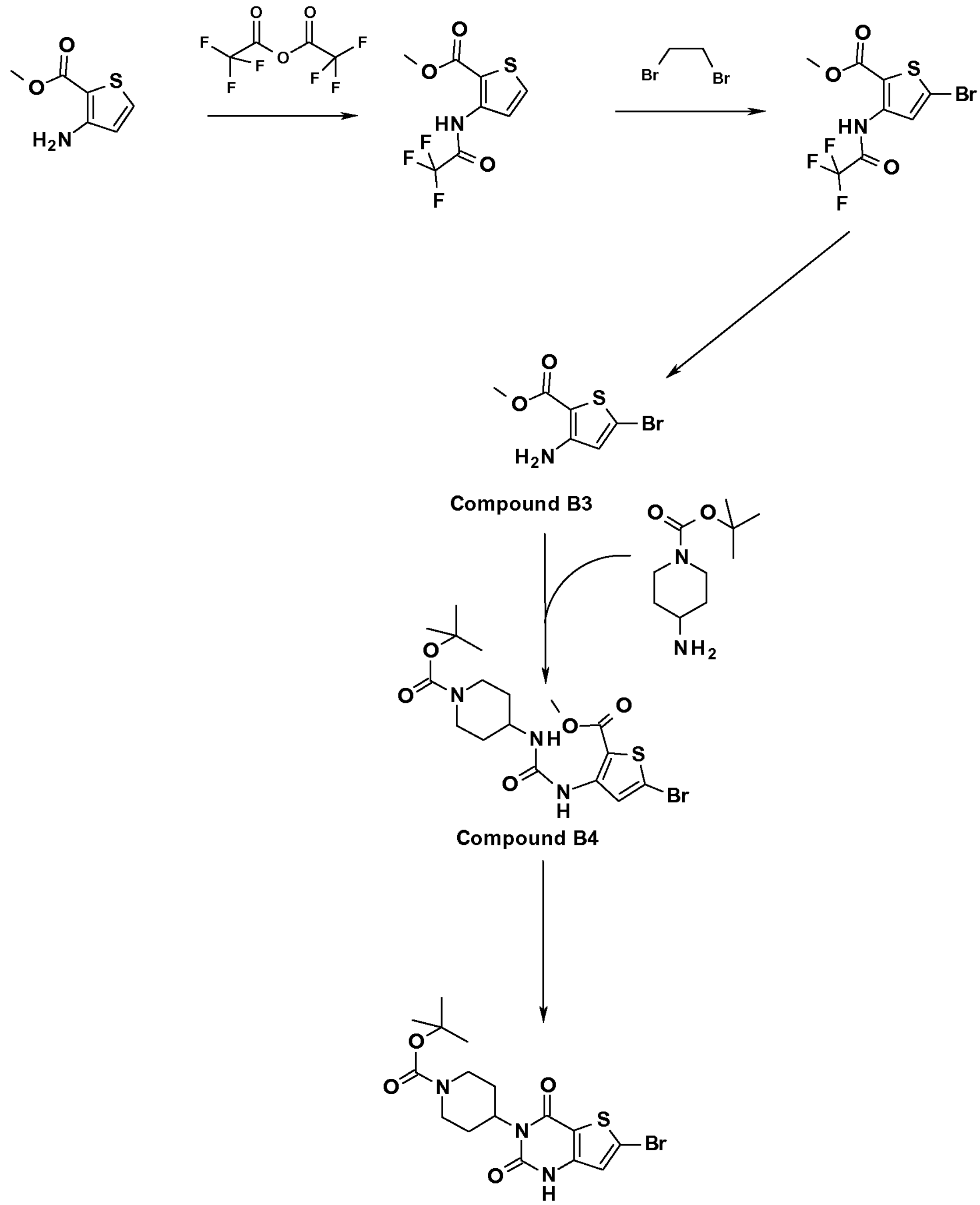


Compound B5

Alternatively, the hydrogenation of the obtained imine/enamine of formulae 14a/14b, in which R1 and R2 have the above mentioned meanings is carried out in the presence of hydrogen transfer reagents like alkali borohydride, alkali cyanoborohydride, alkali triacetoxyborohydride or alkali acyloxyborohy-drides using dichloromethane, toluene or THF as a solvent preferably at RT. The alkali acyloxyborohy-drides are prepared, for example from $NaBH_4$ and various carboxylic acids (for example 2-methyl-hexanoic acid) according to methods known to the person skilled in the art, for example, as described in Tetrahedron Letters, 37 (1996), 3977-3980.

**Reaction Scheme 5:**

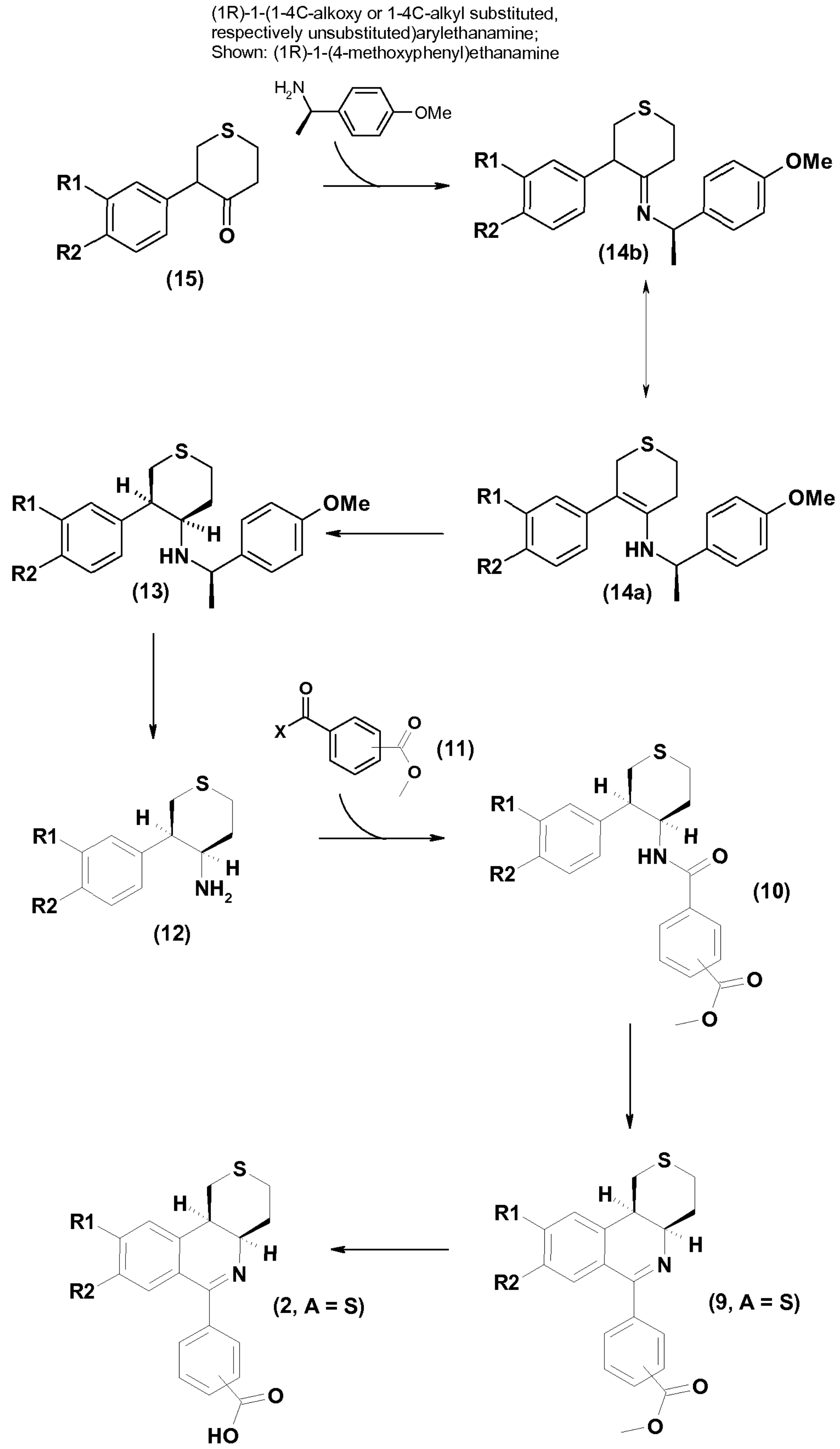


(15) (14b) (14a) (13) (12) (11) (10) (9, A = S) (2, A = S)

The cleveage of the (1R)-1-(1-4C-alkoxy substituted or unsubstituted)arylethyl group by hydrogenation from the compounds of formula 13 is also carried out according to standard methods known to the person skilled in the art, preferably in the presence of 1 to 1.2 equivalents of concentrated hydrochloric acid and a palladium on carbon catalyst using an alcohol, such as methanol or ethanol as a solvent under a hydrogen pressure of about 0.1 to 10 bar, preferably 0.1 to 1 bar, and at elevated temperatures, preferably between 40 and 60°C.

Alternatively, the separation of the (1 R)-1-(1-4C-alkoxy or unsubstituted)arylethyl group is carried out under acidic conditions using neat trifluoroacetic acid or neat formic acid at elevated temperatures, preferably between 50 and 100°C.

The resulting compounds of formula 12, in which R1 and R2 have the above-mentioned meanings are reacted with a compound of formula 11, wherein X is a suitable leaving group, for example a halide, preferably chlorine or bromine. This benzoylation is carried out, for example, according to the Einhorn process, the Schotten-Baumann variant or as described in J. Chem. Soc C, 1971, 1805-1808.

The cyclocondensation of the compounds of formula 10 is carried out in a manner known to the person skilled in the art, for example according to Bischler-Napieralski (e.g. as described in J. Chem. Soc., 1956, 4280-4282) or a Bischler-Napieralski variation (e.g. as described in Heterocycles 60 (2003), No. 12, 2707-2715) in the presence of a suitable condensing reagent, such as, for example, polyphosphoric acid, phosphorus pentachloride, phosphorus trichloride, phosphorus pentoxide, thionyl chloride or trifluoromethanesulfonic anhydride and 4-dimethylaminopyridine, in a suitable inert solvent, e.g. in a chlorinated hydrocarbon such as dichloromethane, or in a cyclic hydrocarbon such as toluene or xylene, or another inert solvent such as acetonitrile, preferably at elevated temperature, in particular at the boiling point of the solvent used.

Finally the compounds of formula 9, wherein R1 and R2 have the above-mentioned meanings are saponified to yield the corresponding acid compounds of formula 2 in the presence of a suitable base, such as, for example, lithium hydroxide, sodium hydroxide, potassium hydroxide, sodium carbonate, potassium carbonate or cesium carbonate, in a suitable solvent, e.g. water, water-methanol, water-dioxane or water-2-propanol.

Reaction scheme 6 shows the preparation of the tetrahydrothiopyranone derivative of formula 15, wherein R1 and R2 have the above-mentioned meanings.

The conversion of the racemic mixture of 3-(3-alkoxy-4-alkoxy-phenyl)-1-(1-4C-alkyl)-piperidin-4-one of formula 17 to the tetrahydrothiopyranone of formula 15 begins with a quarternization of the nitrogen atom of the piperidin-4-one ring by reaction with a suitable alkylation reagent, such as for example methyl iodide, ethyl iodide, trifluoromethansulfonic acid methylester or trifluoromethansulfonic acid ethylester in a suitable solvent, such as for example toluene, dichloromethane, diethylether and preferably 4-methyl-pentan-2-one at low temperatures, preferably between 0°C and 20°C. In the second reaction step the quarternary nitrogen atom is replaced by a sulfur atom through reaction with $Na_2S$ or one of its hydrates, such as for example the nonahydrate in the presence of sodium hydrogensulfide or one of its hydrates, preferable the monohydrate in a water/toluene, water/diethylether, water/dichloro-methan, or preferably in a water/4-methyl-pentan-2-one solvent system, at reflux temperature.

**Reaction Scheme 6:**

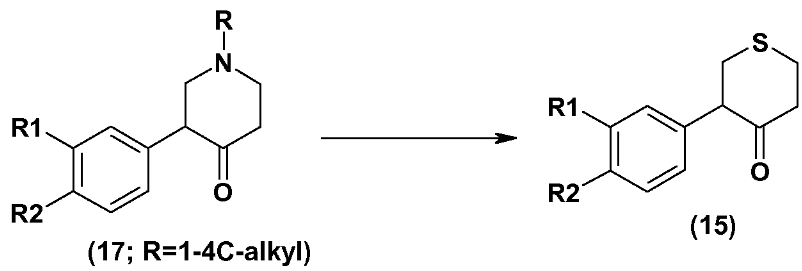

(17; R=1-4C-alkyl)

(15)

The international patent application WO2011073231 describes the preparation of compounds of formula 15, wherein

R1 and R2 independently of each other represent 1-4C-alkoxy, 3-7C-cycloalkoxy, 3-7C-cycloalkyl-methoxy or 1-4C-alkoxy predominantly or completely substituted by fluorine,

or R1 and R2 together form a 1-2C-alkylenedioxy group,

or preferably, R1 and R2 independently of each other represent 1-2C-alkoxy or 1-2C-alkoxy predomi-nantly or completely substituted by fluorine,

or R1 and R2 together form a 1-2C-alkylenedioxy group,

or more preferably, R1 is ethoxy and R2 is methoxy.

The process for the preparation of compounds of formula 15, wherein R1 and R2 have the above-mentioned

meanings is characterized in that

(a) the nitrogen ring-atom of the compound of formula 17

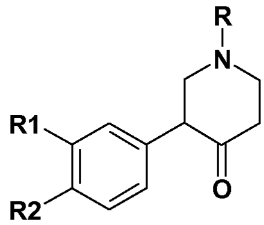

(17)

wherein R1 and R2 have the above-mentioned meanings and R is 1-4C-alkyl, preferably methyl, is quarternized by reaction with an alkylation reagent, preferably trifluoromethansulfonic acid methylester, and

(b) the quarternary ring nitrogen atom is replaced by a sulfur atom through reaction with $Na_2S$ or one of the hydrates of $Na_2S$ in the presence of sodium hydrogensulfide or one of the hydrates of sodium hydrogensulfide.

An alternative method of preparation for compounds of formula 15 is described in the international patent application WO2006027345.

Compounds of formula 17 can be prepared as described in US 3899494 or in analogy thereto.

As can be seen from reaction scheme 1 the compounds of formula 2, wherein R1, R2 and A have the above-mentioned meanings, are key intermediates. They make it possible to introduce into the compouds of formula 1 the 3,4,4a,10b-tetrahydro-1H-thiopyrano[4,3-c]isochinolin structure.

Examples of compounds of formula 2, which may be mentioned in this connection, are: 4-[(2R,4aR,10bR)-9-ethoxy-8-methoxy-2-oxido-3,4,4a,10b-tetrahydro-1H-thiopyrano[4,3-c]isoquinolin-6-yl]benzoic acid; 4-[(2S,4aR,10bR)-9-ethoxy-8-methoxy-2-oxido-3,4,4a,10b-tetrahydro-1H-thio-pyrano[4,3-c]isoquinolin-6-yl]benzoic acid; 4-[(4aR,10bR)-9-ethoxy-8-methoxy-2,2-dioxido-3,4,4a,10b-tetrahydro-1H-thiopyrano[4,3-c]isoquinolin-6-yl]benzoic acid; 3-[(4aR,10bR)-9-ethoxy-8-methoxy-3,4,4a,10b-tetrahydro-1H-thiopyrano[4,3-c]isoquinolin-6-yl]benzoic acid or 4-[(4aR,10bR)-9-ethoxy-8-methoxy-3,4,4a, 10b-tetrahydro-1H-thiopyrano[4,3-c]isoquinolin-6-yl]benzoic acid.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in T. W. Greene, Protective Groups in Organic Synthesis, John Wiley & Sons, 1999, 3rd Ed., or in P. Kocienski, Protecting Groups, Thieme Medical Publishers, 2000.

The compounds according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

As will be appreciated by persons skilled in the art, the invention is not limited to the particular embodiments described herein, but covers all modifications that are within the spirit and scope of the invention as defined by the appended claims.

The following examples illustrate the invention in greater detail, without restricting it. Further compounds according to the invention, of which the preparation is not explicitly described, can be prepared in an analogous way.

The compounds, which are mentioned in the examples, represent preferred embodiments of the invention. In one preferred embodiment, the invention relates to a compound of formula (1) wherein the compounds are selected from the group consisting of 3-(1-{4-[(4aR,10bR)-9-ethoxy-8-methoxy-3,4,4a,10b-tetrahydro-1H-thiopyrano[4,3-c]isoquinolin-6-yl]benzoyl}piperidin-4-yl)-1-(3-fluoro-4-methoxybenzyl)-6-phenyl-thieno[3,2-d]pyrimidine-2,4(1H,3H)-dione, 3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-phenylthieno[3,2-d]-pyrimidine-2,4(1H,3H)-dione, 1-(3,5-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)-piperidin-4-yl]-6-phenylthieno[3,2-d]pyrimidine-2,4(1H,3H)-dione, 1-(2,3-difluoro-4-methoxy-benzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(2-fluoro-4,5-dimethoxy-benzyl)-6-phenyl-thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methylbenzyl)-6-phenylthieno[3,2-d]pyrimidine-2,4(1H,3H)-dione, 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenyl-thieno[3,2-d]pyrimidine-2,4(1H,3H)-dione,

6-(1,3-benzodioxol-5-yl)-3-[1-({4-[(4aR,10bR)-9-ethoxy-8-methoxy-3,4,4a,10b-tetrahydro-1H-thio-pyrano[4,3-c]isoquinolin-6-yl]phenyl}carbonyl)-piperidin-4-yl]-1-(3-fluoro-4-methoxybenzyl)-thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methoxyphenyl)thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(5-fluoro-2-methoxyphenyl)thieno[3,2-d]pyrimidine-2,4(1H,3H)-dione, 6-(2,5-dimethoxyphenyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(2-fluorophenyl)thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)-piperidin-4-yl]-1-(3-fluoro-4-methoxybenzyl)-6-(4-fluorophenyl)thieno[3,2-d]pyrimidine-2,4(1H,3H)-dione, 1-(3,5-difluoro-4-methoxybenzyl)-3-[1-({4-[(4aR,10bR)-9-ethoxy-8-methoxy-3,4,4a,10b-tetra-hydro-1H-thiopyrano[4,3-c]isoquinolin-6-yl]phenyl}carbonyl)piperidin-4-yl]-6-(4-fluoro-2-methoxy-phenyl)thieno[3,2-d]pyrimidine-2,4(1H,3H)-dione, 1-(2,3-difluoro-4-methoxybenzyl)-3-[1-({4-[(4aR, 10bR)-9-ethoxy-8-methoxy-3,4,4a, 10b-tetrahydro-1H-thiopyrano[4,3-c]isoquinolin-6-yl] phenyl}-carbonyl)piperidin-4-yl]-6-(4-fluoro-2-methoxyphenyl)thieno[3,2-d]pyrimidine-2,4(1H,3H)-dione, 3-[1-({4-[(4aR, 10bR)-9-ethoxy-8-methoxy-3,4,4a, 10b-tetrahydro-1H-thiopyrano[4,3-c]isoquinolin-6-yl] phenyl}-carbonyl)piperidin-4-yl]-6-(4-fluoro-2-methoxyphenyl)-1-(3-fluoro-4-methylbenzyl)thieno[3,2-d]pyrimi-dine-2,4(1H,3H)-dione, 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxy-phenyl)thieno-[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methylphenyl)thieno[3,2-d]pyrimidine-2,4(1H,3H)-dione, 3-[1-({4-[(4aR,10bR)-9-ethoxy-8-methoxy-3,4,4a,10b-tetrahydro-1H-thiopyrano[4,3-c]isoquinolin-6-yl]phenyl}-carbonyl)piperidin-4-yl]-1-(3-fluoro-4-methoxybenzyl)-6-(2-methoxyphenyl)thieno[3,2-d]-pyrimidine-2,4(1H,3H)-dione, 6-(1,3-benzodioxol-5-yl)-3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)thieno[3,2-d]pyrimidine-2,4(1H,3H)-dione and 1-(3,5-difluoro-4-methoxybenzyl)-3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimi-dine-2,4(1H,3H)-dione.

## Examples

The following abbreviations are used:

**COMU:** (1-cyano-2-ethoxy-2-oxoethylidenaminooxy)dimethylamino-morpholino-carbenium hexafluorophosphate; **HBTU:** *O*-(benzotriazol-1-yl)-*N,N,N'N'*-tetramethyluronium hexafluorophosphate; **HATU:** *O*-(7-azabenzotriazol-1-yl)-*N,N,N',N'*-tetramethyluronium hexafluorophosphate; **Boc:** t-butoxycarbonyl; **ACN:** Acetonitril; **DIP:** diisopropyl ether; **DMF:** *N,N*-dimethylformamide; **DIPEA:** diisopropylethylamine; **CAN:** diammonium cerium(IV) nitrate; **DCM:** dichloromethane; **DME:** 1,2-dimethoxyethan; **EtOAc:** ethyl acetate; **MeOH:** methanol; **THF:** tetrahydrofuran; **RT:** room temperature; **h:** hour(s); **min:** minute(s); **d:** day(s); **calc.:** calculated; **(v/v):** (volume/volume); **(v/v/v):** (volume/volume/volume); **w/w:** weight / weight; **ESI:** electrospray ionization; **MS:** mass spectrometry; **HRMS:** high resolution mass spectrometry; **TLC:** thin layer chromatography; **HPLC:** high-performance liquid chromatography; **M.p.:** melting point.

Unless otherwise stated compound purification is achieved by flash column chromatography, preparative TLC and preparative HPLC. HPLC purifications are carried out using a Phenomenex Gemini 5 μm C18 (75 x 30 mm) or a Phenomenex Gemini 5 μm C6-Phenyl (75 x 30 mm) or a Phenomenex Gemini 5 μm C18 Axia (75 x 30 mm) column, a binary gradient (solvent A: water, solvent B: acetonitrile), a flow rate of 40 ml/min, formic acid as a buffer or a buffer system consisting of formic acid and ammonium formiate and UV detection at 240 nm.

All mass spectra are obtained using ESI technique. HRMS data of examples 1 to 21 are reported as $MH^+$.

## Final products

The chemical names have been generated using the software ACD/NAME Library DLL: NAMIPLIB.dII; Version: 11.1.0.22379.

**1. 3-(1-{4-[(4aR,10bR)-9-ethoxy-8-methoxy-3,4,4a,10b-tetrahydro-1H-thiopyrano[4,3-c]isoquinolin-6-yl]benzoyl)piperidin-4-yl)-1-(3-fluoro-4-methoxybenzyl)-6-phenyl-thieno[3,2-d]pyrimidine-2,4(1H,3H)-dione**

DIPEA (155 mg) was added to a suspension of 4-[(4aR,10bR)-9-ethoxy-8-methoxy-3,4,4a,10b-tetrahydro-1H-thiopyrano[4,3-c]isoquinolin-6-yl]benzoicacid (120 mg; compound C4), 1-(3-fluoro-4-methoxybenzyl)-6-phenyl-3-piperidin-4-ylthieno[3,2-d]pyrimidine-2,4(1H,3H)-dione hydrochloride (152 mg, compound B21) and HBTU (126 mg) in DCM (8 ml) and the mixture was stirred for 1 h at RT. Subsequently the solvent was removed under reduced pressure and the residue was purified by flash column chromatography [silica gel, eluation gradient: Cyclohexane / EtOAc / $NEt_3$, 49/49/2 to 9/89/2 (v/v/v)]. The product containing fractions were combined and the solvent was removed under vacuum. The resulting residue was treated with diethyl ether to give the title compound as a solid.

HRMS [$C_{47}H_{46}FN_4O_6S_2$]: calc.: 845.2837 found: 845.2829

**2. 3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-phenylthieno[3,2-d]pyrimidine-2,4(1H,3H)-dione**

DIPEA (0.35 ml) was added to a suspension of 3-[(4aR,10bR)-9-ethoxy-8-methoxy-3,4,4a,10b-tetrahydro-1H-thiopyrano[4,3-c]isoquinolin-6-yl]benzoic acid (200 mg; compound C2), 1-(3-fluoro-4-methoxybenzyl)-6-phenyl-3-(piperidin-4-yl)thieno[3,2-d]pyrimidine-2,4(1H,3H)-dione hydrochloride (254 mg, compound B21) and COMU (214 mg) in DCM (4 ml) and the mixture was stirred for 1 h at RT. Subsequently saturated aqueous sodium bicarbonate solution (3 ml) was added to the mixture and the organic layer was separated. The organic layer was concentrated under reduced pressure and the residue was purified by flash column chromatography [amino phase silica gel, eluation gradient: EtOAc / MeOH, 100/0 to 97/3 (v/v)] to yield the title compound as a solid.

HRMS [$C_{47}H_{46}FN_4O_6S_2$]: calc.: 845.2837 found: 845.2824

**3. 1-(3,5-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione**

DIPEA (0.28 ml) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (159 mg; compound C4), 1-(3,5-difluoro-4-methoxybenzyl)-6-phenyl-3-(piperidin-4-yl)thieno[3,2-d]pyrimidine-2,4(1H,3H)-dione hydrochloride (193 mg, compound B22) and HBTU (167 mg) in DCM (3 ml) and the mixture was stirred for 1 h at RT. Saturated aqueous sodium bicarbonate solution (2 ml) was added and the mixture was filtered using a phase separator. The organic layer was concentrated under reduced pressure and the residue was purified by flash column chromatography [amino phase silica gel, eluent: EtOAc] and afterwards by preparative HPLC to yield the title compound as a solid.

HRMS [$C_{47}H_{45}F_2N_4O_6S_2$]: calc.: 863.2743 found: 863.2731

**4. 1-(2,3-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione**

DIPEA (0.28 ml) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (159 mg; compound C4), 1-(2,3-difluoro-4-methoxybenzyl)-6-phenyl-3-(piperidin-4-yl)thieno[3,2-d]pyrimidine-2,4(1H,3H)-dione hydrochloride (193 mg, compound B23) and HBTU (167 mg) in DCM (3 ml) and the mixture was stirred for 1 h at RT. Saturated aqueous sodium bicarbonate solution (2 ml) was added and the mixture was filtered using a phase separator. The organic layer was concentrated under reduced pressure and the residue was purified by flash column chromatography [amino phase silica gel, eluent: EtOAc] and afterwards by preparative HPLC to yield the title compound as a solid.

HRMS [$C_{47}H_{45}F_2N_4O_6S_2$]: calc.: 863.2743 found: 863.2734

**5. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(2-fluoro-4,5-dimethoxybenzyl)-6-phenylthieno[3,2-d]pyrimidine-2,4(1H,3H)-dione**

DIPEA (0.28 ml) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (159 mg; compound C4), 1-(2-fluoro-4,5-dimethoxybenzyl)-6-phenyl-3-(piperidin-4-yl)thieno[3,2-d]pyrimidine-2,4(1H,3H)-dione hydrochloride (198 mg, compound B34) and HBTU (167 mg) in DCM (3 ml) and the mixture was stirred for 1 h at RT. Saturated aqueous sodium bicarbonate solution (2 ml) was added and the mixture was filtered using a phase separator. The organic layer was concentrated under reduced pressure and the residue was purified by flash column chromatography [amino phase silica gel, eluent: EtOAc] and afterwards by preparative HPLC to yield the title compound as a solid.

HRMS [$C_{48}H_{48}FN_4O_7S_2$]: calc.: 875.2943 found: 875.2941

**6. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methylbenzyl)-6-phenylthieno[3,2-d]pyrimidine-2,4(1H,3H)-dione**

DIPEA (207 mg) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (159 mg; compound C4), 1-(3-fluoro-4-methylbenzyl)-6-phenyl-3-(piperidin-4-yl)thieno[3,2-d]pyrimidine-2,4(1H,3H)-dione hydrochloride (194 mg, compound B36) and HBTU (182 mg) in DCM (10 ml) and the mixture was stirred for 75 min at RT. The solvent was removed under reduced pressure and the resulting residue was purified by flash column chromatography [amino phase silica gel, eluation gradient: Cyclohexane / EtOAc, 100/0 to 0/100 (v/v) to EtOAc / MeOH 92/8 (v/v)] to give the title compound as a solid.

HRMS [$C_{47}H_{46}FN_4O_5S_2$]: calc.: 829.2888 found: 829.2881

**7. 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione**

DIPEA (0.28 ml) was added to a suspension of 4-[(4aR,10bR)-9-ethoxy-8-methoxy-3,4,4a,10b-tetrahydro-1H-thiopyrano[4,3-c]isoquinolin-6-yl]benzoicacid (159 mg; compound C4), 1-benzyl-6-phenyl-3-(piperidin-4-yl)thieno[3,2-d]pyrimidine-2,4(1H,3H)-dione hydrochloride (167 mg, compound B38) and HBTU (167 mg) in DCM (4 ml) and the mixture was stirred for 1 h at RT. Saturated aqueous sodium bicarbonate solution (2 ml) was added and the mixture was filtered using a phase separator. The organic layer was concentrated under reduced pressure and the residue was purified by flash column chromatography [amino phase silica gel, eluent: Cyclohexane / EtOAc, 10/90 (v/v)] and afterwards by preparative HPLC to yield the title compound as a solid.

HRMS [$C_{46}H_{45}N_4O_5S_2$]: calc.: 797.2826 found: 797.2821

**8. 6-(1,3-benzodioxol-5-yl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)thieno[3,2-d]pyrimidine-2,4(1H,3H)-dione**

To a suspension of 4-[(4aR,10bR)-9-ethoxy-8-methoxy-3,4,4a,10b-tetrahydro-1H-thiopyrano[4,3-c]isoquinolin-6-yl]benzoic acid (73 mg; compound C4) and HATU (83 mg) in DCM (10 ml) was added DIPEA (0.13 ml). The mixture was stirred at RT for 40 min and then 6-(1,3-benzodioxol-5-yl)-1-(3-fluoro-4-methoxybenzyl)-3-(piperidin-4-yl)thieno[3,2-d]pyrimidine-2,4(1H,3H)-dione hydrochloride (100 mg, compound B28) was added. The reaction mixture was stirred for 12 h at RT. All volatile materials were removed in vacuo and the residue was purified by preparative HPLC to yield the title compound as a solid.

HRMS [$C_{48}H_{46}FN_4O_8S_2$]: calc.: 889.2736 found: 889.2738

**9. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methoxyphenyl)thieno[3,2-d]pyrimidine-2,4(1H,3H)-dione**

1-(3-Fluoro-4-methoxybenzyl)-6-(4-fluoro-2-methoxyphenyl)-3-(piperidin-4-yl)thieno[3,2-d]pyrimidine-2,4(1H,3H)-dione hydrochloride (100 mg, compound B29) was reacted with 4-[(4aR,10bR)-9-ethoxy-8-methoxy-3,4,4a,10b-tetrahydro-1H-thiopyrano[4,3-c]isoquinolin-6-yl]benzoic acid (72 mg; compound C4) and HATU (83 mg) in DCM (10 ml) in the presence of DIPEA (0.13 ml) according to the procedure described in example 8 to afford the title compound after purification by preparative HPLC as a solid.

HRMS [$C_{48}H_{47}F_2N_4O_7S_2$]: calc.: 893.2849 found: 893.2839

**10. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(5-fluoro-2-methoxyphenyl)thieno[3,2-d]pyrimidine-2,4(1H,3H)-dione**

1-(3-Fluoro-4-methoxybenzyl)-6-(5-fluoro-2-methoxyphenyl)-3-(piperidin-4-yl)thieno[3,2-d]pyrimidine-2,4(1H,3H)-dione hydrochloride (100 mg, compound B30) was reacted with 4-[(4aR,10bR)-9-ethoxy-8-methoxy-3,4,4a,10b-tetrahydro-1H-thiopyrano[4,3-c]isoquinolin-6-yl]benzoic acid (72 mg; compound C4) and HATU (83 mg) in DCM (10 ml) in the presence of DIPEA (0.13 ml) according to the procedure described in example 8 to afford the title compound after purification by preparative HPLC as a solid.

HRMS [$C_{48}H_{47}F_2N_4O_7S_2$]: calc.: 893.2849 found: 893.2847

**11. 6-(2,5-dimethoxyphenyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)thieno[3,2-d]pyrimidine-2,4(1H,3H)-dione**

6-(2,5-Dimethoxyphenyl)-1-(3-fluoro-4-methoxybenzyl)-3-(piperidin-4-yl)thieno[3,2-d]pyrimidine-2,4(1H,3H)-dione hydrochloride (100 mg, compound B31) was reacted with 4-[(4aR,10bR)-9-ethoxy-8-methoxy-3,4,4a,10b-tetrahydro-1H-thiopyrano[4,3-c]isoquinolin-6-yl]benzoic acid (71 mg; compound C4) and HATU (81 mg) in DCM (10 ml) in the presence of DIPEA (0.12 ml) according to the procedure described in example 8 to afford the title compound after purification by preparative HPLC as a solid.

HRMS [$C_{49}H_{50}FN_4O_8S_2$]: calc.: 905.3049 found: 905.3033

**12. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(2-fluorophenyl)thieno[3,2-d]pyrimidine-2,4(1H,3H)-dione**

1-(3-Fluoro-4-methoxybenzyl)-6-(2-fluorophenyl)-3-(piperidin-4-yl)thieno[3,2-d]pyrimidine-2,4(1H,3H)-dione hydrochloride (100 mg, compound B32) was reacted with 4-[(4aR,10bR)-9-ethoxy-8-methoxy-3,4,4a,10b-tetrahydro-1H-thiopyrano[4,3-c]isoquinolin-6-yl]benzoic acid (76 mg; compound C4) and HATU (88 mg) in DCM (10 ml) in the presence of DIPEA (0.13 ml) according to the procedure described in example 8 to afford the title compound after purification by preparative HPLC as a solid.

HRMS [$C_{47}H_{45}F_2N_4O_6S_2$]: calc.: 863.2743 found: 863.2737

**13. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluorophenyl)thieno[3,2-d]pyrimidine-2,4(1H,3H)-dione**

1-(3-Fluoro-4-methoxybenzyl)-6-(4-fluorophenyl)-3-(piperidin-4-yl)thieno[3,2-d]pyrimidine-2,4(1H,3H)-dione hydrochloride (100 mg, compound B33) was reacted with 4-[(4aR,10bR)-9-ethoxy-8-methoxy-3,4,4a,10b-tetrahydro-1H-thiopyrano[4,3-c]isoquinolin-6-yl]benzoic acid (76 mg; compound C4) and HATU (88 mg) in DCM (10 ml) in the presence of DIPEA (0.13 ml) according to the procedure described in example 8 to afford the title compound after

purification by preparative HPLC as a solid.

HRMS [$C_{47}H_{45}F_2N_4O_6S_2$]: calc.: 863.2743 found: 863.2734

**14. 1-(3,5-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)thieno[3,2-d]pyrimidine-2,4(1H,3H)-dione**

DIPEA (162 mg) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (125 mg; compound C4), 1-(3,5-difluoro-4-methoxybenzyl)-6-(4-fluoro-2-methoxyphenyl)-3-(piperidin-4-yl)thieno[3,2-d]pyrimidine-2,4(1H,3H)-dione hydrochloride (142 mg, compound B41) and HBTU (119 mg) in DCM (10 ml) and the mixture was stirred for 75 min at RT. Saturated aqueous sodium bicarbonate solution (2 ml) and DCM (5 ml) were added and the mixture was filtered using a phase separator. The organic layer was concentrated under reduced pressure and the residue was purified by flash column chromatography [amino phase silica gel, eluation gradient: cyclohexane / EtOAc, 100/0 to 50/50 to 20/80 to 0/100 v/v)] and afterwards by preparative HPLC to yield the title compound as a solid.

HRMS [$C_{48}H_{46}F_3N_4O_7S_2$]: calc.: 911.2755 found: 911.2756

**15. 1-(2,3-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)thieno[3,2-d]pyrimidine-2,4(1H,3H)-dione**

DIPEA (162 mg) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (125 mg; compound C4), 1-(2,3-difluoro-4-methoxybenzyl)-6-(4-fluoro-2-methoxyphenyl)-3-(piperidin-4-yl)thieno[3,2-d]pyrimidine-2,4(1H,3H)-dione hydrochloride (142 mg, compound B43) and HBTU (119 mg) in DCM (10 ml) and the mixture was stirred for 75 min at RT. Saturated aqueous sodium bicarbonate solution (2 ml) and DCM (5 ml) were added and the mixture was filtered using a phase separator. The organic layer was concentrated under reduced pressure and the residue was purified by flash column chromatography [amino phase silica gel, eluation gradient: cyclohexane / EtOAc, 100/0 to 50/50 to 20/80 to 0/100 v/v)] and afterwards by preparative HPLC to yield the title compound as a solid.

HRMS [$C_{48}H_{46}F_3N_4O_7S_2$]: calc.: 911.2755 found: 911.2758

**16. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)-1-(3-fluoro-4-methylbenzyl)thieno[3,2-d]pyrimidine-2,4(1H,3H)-dione**

DIPEA (175 mg) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (125 mg; compound C4), 6-(4-fluoro-2-methoxyphenyl)-1-(3-fluoro-4-methylbenzyl)-3-(piperidin-4-yl)thieno[3,2-d]pyrimidine-2,4(1H,3H)-dione hydrochloride (142 mg, compound B24) and HBTU (128 mg) in DCM (10 ml) and the mixture was stirred for 75 min at RT. Saturated aqueous sodium bicarbonate solution (2 ml) and DCM (5 ml) were added and the mixture was filtered using a phase separator. The organic layer was concentrated under reduced pressure and the residue was purified by flash column chromatography [amino phase silica gel, eluation gradient: cyclohexane / EtOAc, 100/0 to 50/50 to 20/80 to 0/100 v/v)] and afterwards by preparative HPLC to yield the title compound as a solid.

HRMS [$C_{48}H_{47}F_2N_4O_6S_2$]: calc.: 877.2900 found: 877.2900

**17. 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)thieno[3,2-d]pyrimidine-2,4(1H,3H)-dione**

DIPEA (181 mg) was added to a suspension of 4-[(4aR,10bR)-9-ethoxy-8-methoxy-3,4,4a,10b-tetrahydro-1H-thiopyrano[4,3-c]isoquinolin-6-yl]benzoic acid (139 mg; compound C4), 1-benzyl-6-(4-fluoro-2-methoxyphenyl)-3-(piperidin-4-yl)thieno[3,2-d]pyrimidine-2,4(1H,3H)-dione hydrochloride (141 mg, compound B25) and HBTU (133 mg) in DCM (10 ml) and the mixture was stirred for 75 min at RT. Saturated aqueous sodium bicarbonate solution (2 ml) and

DCM (5 ml) were added and the mixture was filtered using a phase separator. The organic layer was concentrated under reduced pressure and the residue was purified by flash column chromatography [amino phase silica gel, eluation gradient: cyclohexane/EtOAc, 100/0 to 0/100 (v/v)] and afterwards by preparative HPLC to yield the title compound as a solid.

HRMS [$C_{47}H_{46}FN_4O_6S_2$]: calc.: 845.2837 found: 845.2833

**18. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methylphenyl)thieno[3,2-d]pyrimidine-2,4(1H,3H)-dione**

1-(3-Fluoro-4-methoxybenzyl)-6-(4-fluoro-2-methylphenyl)-3-(piperidin-4-yl)thieno[3,2-d]pyrimidine-2,4(1H,3H)-dione hydrochloride (100 mg, compound B26) was reacted with 4-[(4aR,10bR)-9-ethoxy-8-methoxy-3,4,4a,10b-tetrahydro-1H-thiopyrano[4,3-c]isoquinolin-6-yl]benzoic acid (74 mg; compound C4) and HATU (85 mg) in DCM (15 ml) in the presence of DIPEA (0.13 ml) according to the procedure described in example 8 to afford the title compound after purification by preparative HPLC as a solid.

HRMS [$C_{48}H_{47}F_2N_4O_6S_2$]: calc.: 877.2900 found: 877.2912

**19. 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(2-methoxyphenyl)thieno[3,2-d]pyrimidine-2,4(1H,3H)-dione**

1-(3-Fluoro-4-methoxybenzyl)-6-(2-methoxyphenyl)-3-(piperidin-4-yl)thieno[3,2-d]pyrimidine-2,4(1H,3H)-dione hydrochloride (100 mg, compound B27) was reacted with 4-[(4aR,10bR)-9-ethoxy-8-methoxy-3,4,4a,10b-tetrahydro-1H-thiopyrano[4,3-c]isoquinolin-6-yl]benzoic acid (75 mg; compound C4) and HATU (85 mg) in DCM (15 ml) in the presence of DIPEA (0.13 ml) according to the procedure described in example 8 to afford the title compound after purification by preparative HPLC as a solid.

HRMS [$C_{48}H_{48}FN_4O_7S_2$]: calc.: 875.2943 found: 875.2954

**20. 6-(1,3-benzodioxol-5-yl)-3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)thieno[3,2-d]pyrimidine-2,4(1H,3H)-dione**

DIPEA (311 mg) was added to a suspension of 3-[(4aR,10bR)-9-ethoxy-8-methoxy-3,4,4a,10b-tetrahydro-1H-thiopyrano[4,3-c]isoquinolin-6-yl]benzoic acid (239 mg; compound C2), 6-(1,3-benzodioxol-5-yl)-1-(3-fluoro-4-methoxybenzyl)-3-(piperidin-4-yl)thieno[3,2-d]pyrimidine-2,4(1 H,3H)-dione hydrochloride (328 mg, compound B28) and HBTU (251 mg) in DCM (10 ml) and the mixture was stirred for 45 min at RT protected from light. The mixture was extracted with DCM (25 ml) and saturated aqueous sodium bicarbonate solution (10 ml). The organic phase was separated and treated with activated charcoal (0.5 g). After filtration over a plug of celite the organic layer was concentrated under reduced pressure. The resulting residue was purified twice by flash column chromatography [amino phase silica gel, eluation gradient: cyclohexane / EtOAc, 100/0 to EtOAc / MeOH, 94/6 (v/v)] to yield the title compound as a solid.

HRMS [$C_{48}H_{46}FN_4O_8S_2$]: calc.: 889.2736 found: 889.2728

**21. 1-(3,5-difluoro-4-methoxybenzyl)-3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione**

DIPEA (403 mg) was added to a suspension of 3-[(4aR,10bR)-9-ethoxy-8-methoxy-3,4,4a,10b-tetrahydro-1H-thiopyrano[4,3-c]isoquinolin-6-yl]benzoic acid (310 mg; compound C2), 1-(3,5-difluoro-4-methoxybenzyl)-6-phenyl-3-(piperidin-4-yl)thieno[3,2-d]pyrimidine-2,4(1H,3H)-dione hydrochloride (377 mg, compound B22) and HBTU (326 mg) in DCM (10 ml) and the mixture was stirred for 20 h at RT protected from light. The mixture was extracted with DCM (8 ml) and saturated aqueous sodium bicarbonate solution (2 ml). The organic phase was separated and extracted with half saturated aqueous solution of citric acid (3 ml) at first and then with saturated aqueous sodium bicarbonate solution (3 ml). The organic phase was separated, DCM (20 ml) was added and the solution was treated with activated charcoal

(0.3 g). After filtration over a plug of celite and afterwards through a phase separator the organic layer was concentrated under reduced pressure. The resulting residue was purified by flash column chromatography [amino phase silica gel, eluation gradient: cyclohexane / EtOAc, 100/0 to 0/100 (v/v)] and afterwards by preparative TLC [20x20cm TLC plates with 0.5 mm thickness, eluent: EtOAc / MeOH / $NEt_3$, 95/5/4 (v/v/v)] to yield the title compound as a solid.

HRMS [$C_{47}H_{45}F_2N_4O_6S_2$]: calc.: 863.2743 found: 863.2745

**Intermediates and Starting Compounds**

**B1. Tert-butyl 4-({[2-(methoxycarbonyl)-5-phenylthiophen-3-yl]carbamoyl}amino)piperidine-1-carboxylate**

To a solution of triphosgene (421 mg) in THF (15 ml) under nitrogen atmosphere was added at 0°C a solution of 3-amino-5-phenylthiophene-2-carboxylate (1.00 g) in THF (10 ml) within 2 h. The mixture was stirred at 0°C for 1 h and at RT for additional 14 h. The resulting suspension was cooled to 0°C and a solution of tert-butyl 4-aminopiperidine-1-carboxylate (867 mg) in THF (10 ml) was added dropwise. After 15 min at 0°C DIPEA (1.75 ml) was slowly added. The reaction mixture was stirred for 15 min at 0°C and for 2.5 h at RT and then washed with an aqueous sodium chloride solution (5 % w/w, three times with 20 ml each) and with brine (20 ml). The organic layer was dried over magnesium sulfate. All volatile materials were removed in vacuo to give the title compound as a solid.

MS: calc.: $C_{23}H_{29}N_3O_5S$ (459.56) found: [$MH^+$] = 459.98; [$MNa^+$] = 482.22

**B2. Tert-butyl 4-(2,4-dioxo-6-phenyl-1,4-dihydrothieno[3,2-d]pyrimidin-3(2H)-yl)piperidine-1-carboxylate**

To a solution of tert-butyl 4-({[2-(methoxycarbonyl)-5-phenylthiophen-3-yl]carbamoyl}amino)piperidine-1-carboxylate (2.00 g; compound B1) in MeOH (20 ml) was added sodium methoxide (300 mg) in three portions at RT. The reaction mixture was refluxed for 3 d and then allowed to come to RT. Ice cold water (30 ml) was added and the pH was adjusted to pH 4 by addition of citric acid. The resulting suspension was stirred for 2 h at RT; then the precipitate was filtered off, washed with water and dried in vacuo at 50°C. The crude title compound was taken up in MeOH (25 ml) and refluxed for 3 h. The hot suspension was filtered, and the filter cake was washed with hot MeOH three times and dried in vacuo at 45°C to afford the title compound as a solid.

MS: calc.: $C_{22}H_{25}N_3O_4S$ (427.52) found: [$MNa^+$] = 450.07; [$MH^+$ - Boc] = 328.24

**B3. Methyl 3-amino-5-bromothiophene-2-carboxylate**

The title compound can be prepared starting from commercially available methyl 3-aminothiophene-2-carboxylate as described in Bioorganic & Medicinal Chemistry Letters, 17, (2007) 2535-2539.

**B4. Tert-butyl 4-({[5-bromo-2-(methoxycarbonyl)thiophen-3-yl]carbamoyl}amino)piperidine-1-carboxylate**

Triphosgene (8.22 g) was dissolved in dry THF (345 ml) under argon atmosphere. The solution was cooled to 0°C and a solution of methyl 3-amino-5-bromothiophene-2-carboxylate (19.8 g; compound B3) in dry THF (173 ml) was added dropwise keeping the temperature below 10°C. The mixture was stirred for 14 h at RT. The reaction mixture was cooled to 0°C again and a solution of tert-butyl 4-aminopiperidine-1-carboxylate (17.0 g) in dry THF (173 ml) was added within 15 min keeping the temperature below 10°C. After 15 min at 0°C and additional 3 h at RT water (495 ml) and saturated aqueous sodium chloride solution (124 ml) were added. The organic layer was separated and the aqueous layer was extracted with THF three times (124 ml each). The combined organic layers were washed with half concentrated aqueous sodium chloride solution three times (250 ml each) and with saturated aqueous sodium chloride solution (250 ml) once. After drying over sodium sulfate all volatile materials were removed in vacuo to yield the title compound as a solid which was used without further purification.

**B5. Tert-butyl 4-(6-bromo-2,4-dioxo-1,4-dihydrothieno[3,2-d]pyrimidin-3(2H)-yl)piperidine-1-carboxylate**

To a solution of tert-butyl 4-({[5-bromo-2-(methoxycarbonyl)thiophen-3-yl]carbamoyl}amino)piperidine-1-carboxylate (37.6 g; compound B4) in dry MeOH (205 ml) under argon atmosphere was added sodium methoxide (6.16 g) at RT. The reaction mixture was refluxed for 3 h, allowed to cool to RT and then poured into a solution of citric acid (21.9

g) in ice cold water (961 ml). The precipitated solid was filtered off, washed with ice cold water and dried in vacuo. The residue was taken up in MeOH (205 ml) and the resulting suspension was refluxed for 1 h. After cooling to RT all solids were filtered off, washed with MeOH and dried in vacuo to afford the title compound as a solid.

MS: calc.: $C_{16}H_{20}BrN_3O_4S$ (430.32) found: $[MH^+]$ = 431.44; $[MH^+$- Boc] = 331.98

**B6. Tert-butyl 4-[6-(4-fluoro-2-methoxyphenyl)-2,4-dioxo-1,4-dihydrothieno[3,2-d]pyrimidin-3(2H)-yl]piperidine-1-carboxylate**

Tert-butyl 4-(6-bromo-2,4-dioxo-1,4-dihydrothieno[3,2-d]pyrimidin-3(2H)-yl)piperidine-1-carboxylate (3.00 g, compound B5), (4-fluoro-2-methoxyphenyl)boronic acid (1.30 g), dichlorobis(tricyclohexyl-phosphine)palladium (257 mg) and aqueous cesium carbonate solution (5.23 ml, 2.0 M) were partitioned between three microwave tubes and DME (12 ml each) was added. The reaction vessels were sealed and the mixtures were successively subjected to microwave irradiation at 150 °C with stirring for 20 min. The reaction mixtures were poured into ice-cold water and the mixture was extracted with DCM. The organic layer was washed with saturated aqueous sodium chloride solution and filtered using a phase separator. All volatile materials were removed in vacuo to yield the title compound as a solid.

MS: calc.: $C_{23}H_{26}FN_3O_5S$ (475.53) found: $[MH^+$- Boc] = 376.18

**B7. Tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate**

To a solution of tert-butyl 4-(6-bromo-2,4-dioxo-1,4-dihydrothieno[3,2-d]pyrimidin-3(2H)-yl)piperidine-1-carboxylate (5.00 g; compound B5) in DMF (35 ml) were added potassium carbonate (1.61 g) and 4-(chloromethyl)-2-fluoro-1-methoxybenzene (2.03 g). The mixture was stirred at 100 °C for 1.5 h, allowed to come to RT and then poured into ice-cold water. The resulting precipitate was filtered off, washed with water and dried in vacuo to give the title compound as a solid.

MS: calc.: $C_{24}H_{27}BrFN_3O_5S$ (568.46) found: $[MNa^+]$ = 589.97, 591.93

**B8. tert-butyl 4-[1-(3-fluoro-4-methoxybenzyl)-2,4-dioxo-6-phenyl-1,4-dihydrothieno[3,2-d]pyrimidin-3(2H)-yl]piperidine-1-carboxylate**

Potassium carbonate (2.49 g) and 3-fluoro-4-methoxybenzyl chloride (3.14 g) were added to a solution of tert-butyl 4-(2,4-dioxo-6-phenyl-1,4-dihydrothieno[3,2-d]pyrimidin-3(2H)-yl)piperidine-1-carboxylate (7.70 g; compound B2) in anhydrous DMF (50 ml) and the reaction mixture was stirred for 2.5 h at 100 °C under a nitrogen atmosphere. Afterwards the reaction mixture was poured into ice-cold water and the precipitated solid was filtered off and washed with water. The solid was dissolved in ethyl acetate (400 ml) at 65 °C and the solvent was slowly removed under vacuum at 40 °C to a volume of about 100 ml. The resulting precipitate was homogenized in an ultrasonic bath, filtered at room temperature and the filter cake was washed with ethyl acetate (40 ml) to give the title compound as a solid.

MS: calc.: $C_{30}H_{32}FN_3O_5S$ (565.66) found: $[MH^+]$ = 566.85; $[MH^+$ - Boc] = 466.16

**B9. Tert-butyl 4-[1-(3,5-difluoro-4-methoxybenzyl)-2,4-dioxo-6-phenyl-1,4-dihydrothieno[3,2-d]pyrimidin-3(2H)-yl]piperidine-1-carboxylate**

Potassium carbonate (323 mg) and 3,5-difluoro-4-methoxybenzyl bromide (554 mg) were added to a solution of tert-butyl 4-(2,4-dioxo-6-phenyl-1,4-dihydrothieno[3,2-d]pyrimidin-3(2H)-yl)piperidine-1-carboxylate (1.0 g; compound B2) in anhydrous DMF (10 ml) and the reaction mixture was stirred for 10 h at 100 °C under a nitrogen atmosphere. Afterwards the reaction mixture was poured into ice-cold water and the precipitated solid was filtered off, washed with water and dried under vacuum to give the title compound.

MS: calc.: $C_{30}H_{31}F_2N_3O_5S$ (583.65) found: $[MH^+]$ = 583.50; $[MH^+$ - Boc] = 484.15

**B10. Tert-butyl 4-[1-(2,3-difluoro-4-methoxybenzyl)-2,4-dioxo-6-phenyl-1,4-dihydrothieno[3,2-d]pyrimidin-3(2H)-yl]piperidine-1-carboxylate**

Potassium carbonate (323 mg) and 2,3-difluoro-4-methoxybenzyl bromide (554 mg) were added to a solution of tert-butyl 4-(2,4-dioxo-6-phenyl-1,4-dihydrothieno[3,2-d]pyrimidin-3(2H)-yl)piperidine-1-carboxylate (1.0 g; compound B2) in anhydrous DMF (10 ml) and the reaction mixture was stirred for 10 h at 100 °C under a nitrogen atmosphere. Afterwards the reaction mixture was poured into ice-cold water and the precipitated solid was filtered off, washed with water and dried under vacuum to give the title compound.

MS: calc.: $C_{30}H_{31}F_2N_3O_5S$ (583.65) found: $[MH^+]$ = 584.98; $[MH^+ - Boc]$ = 484.18

**B11. Tert-butyl 4-[6-(4-fluoro-2-methoxyphenyl)-1-(3-fluoro-4-methylbenzyl)-2,4-dioxo-1,4-dihydrothieno[3,2-d]pyrimidin-3(2H)-yl]piperidine-1-carboxylate**

To a solution of tert-butyl 4-[6-(4-fluoro-2-methoxyphenyl)-2,4-dioxo-1,4-dihydrothieno[3,2-d]pyrimidin-3(2H)-yl]piperidine-1-carboxylate (400 mg; compound B6) in DMF (10 ml) were added potassium carbonate (116 mg) and 4-(bromomethyl)-2-fluoro-1-methylbenzene (171 mg). The mixture was stirred at 80 °C for 75 min and for 12 min at RT. The mixture was poured into ice-cold water and the resulting precipitate was filtered off, washed with water and dried in vacuo to give the title compound as a solid.

MS: calc.: $C_{31}H_{33}F_2N_3O_5S$ (597.68) found: $[MNa^+]$ = 620.15

**B12. Tert-butyl 4-[1-benzyl-6-(4-fluoro-2-methoxyphenyl)-2,4-dioxo-1,4-dihydrothieno[3,2-d]pyrimidin-3(2H)-yl]piperidine-1-carboxylate**

According to the procedure described for compound B11 tert-butyl 4-[6-(4-fluoro-2-methoxyphenyl)-2,4-dioxo-1,4-dihydrothieno[3,2-d]pyrimidin-3(2H)-yl]piperidine-1-carboxylate (400 mg; compound B6) and (bromomethyl)benzene (144 mg) were reacted in the presence of potassium carbonate (116 mg) in DMF (10 ml) to yield the title compound as a solid.

MS: calc.: $C_{30}H_{32}FN_3O_5S$ (565.66) found: $[MNa^+]$ = 588.10

**B13. Tert-butyl 4-[1-(3-fluoro-4-methoxybenzyl)-6-(4-fluoro-2-methylphenyl)-2,4-dioxo-1,4-dihydrothieno[3,2-d]pyrimidin-3(2H)-yl]piperidine-1-carboxylate**

Tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7), (4-fluoro-2-methylphenyl)boronic acid (119 mg), dichlorobis(tricyclohexylphosphine)palladium (26 mg) and aqueous cesium carbonate solution (0.53 ml, 2.0 M) were placed in a microwave tube and DME (12 ml) was added. The reaction vessel was sealed and the mixture was subjected to microwave irradiation at 150 °C with stirring for 20 min. The reaction mixture was poured into ice-cold water. The resulting precipitate was filtered off and purified by flash column chromatography [silica gel, eluation gradient: DCM / MeOH, 1/0 to 1/1 (v/v)] to yield the title compound as a solid.

MS: calc.: $C_{31}H_{33}F_2N_3O_5S$ (597.68) found: $[MNa^+]$ = 620.10

**B14. Tert-butyl 4-[1-(3-fluoro-4-methoxybenzyl)-6-(2-methoxyphenyl)-2,4-dioxo-1,4-dihydrothieno[3,2-d]pyrimidin-3(2H)-yl]piperidine-1-carboxylate**

According to the procedure described for compound B13 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) and (2-methoxyphenyl)boronic acid (139 mg) were reacted in the presence of dichloro-bis(tricyclohexylphosphine)palladium (26 mg) and aqueous cesium carbonate solution (0.53 ml, 2.0 M) in DME (12 ml). The reaction mixture was concentrated in vacuo and the residue was purified by flash column chromatography [silica gel, eluation gradient: DCM / MeOH, 1/0 to 2/1 (v/v)] to yield the title compound as a solid.

MS: calc.: $C_{31}H_{34}FN_3O_6S$ (595.69) found: $[MNa^+]$ = 618.15

**B15. Tert-butyl 4-[6-(1,3-benzodioxol-5-yl)-1-(3-fluoro-4-methoxybenzyl)-2,4-dioxo-1,4-dihydrothieno[3,2-d]pyrimidin-3(2H)-yl]piperidine-1-carboxylate**

Tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7), 1,3-benzodioxol-5-ylboronic acid (117 mg), dichlorobis(tricyclohexylphosphine)palladium (26 mg) and aqueous cesium carbonate solution (0.53 ml, 2.0 M) were placed in a microwave tube and DME (12 ml) was added. The reaction vessel was sealed and the mixture was subjected to microwave irradiation at 150 °C with stirring for 20 min. DCM was added and the reaction mixture was washed with water and saturated aqueous sodium chloride solution. After filtration using a phase separator and concentration in vacuo the residue was purified by flash column chromatography [silica gel, eluation gradient: cyclohexane / EtOAc, 1/0 to 1/1 (v/v)] to yield the title compound as a solid.

MS: calc.: $C_{31}H_{32}FN_3O_7S$ (609.67) found: [$MNa^+$] = 632.15

**B16. Tert-butyl 4-[1-(3-fluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate**

According to the procedure described for compound B15 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) was reacted with (4-fluoro-2-methoxyphenyl)boronic acid (120 mg) in the presence of dichlorobis(tricyclohexylphosphine)palladium (26 mg) and aqueous cesium carbonate solution (0.53 ml, 2.0 M) in DME (10 ml). After purification by flash column chromatography [silica gel, eluation gradient: cyclohexane / EtOAc, 1/0 to 1/1 (v/v)] the title compound was obtained as a solid.

MS: calc.: $C_{31}H_{33}F_2N_3O_6S$ (613.68) found: [$MNa^+$] = 636.10

**B.17 Tert-butyl 4-[1-(3-fluoro-4-methoxybenzyl)-6-(5-fluoro-2-methoxyphenyl)-2,4-dioxo-1,4-dihydrothieno[3,2-d]pyrimidin-3(2H)-yl]piperidine-1-carboxylate**

According to the procedure described for compound B15 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) was reacted with (5-fluoro-2-methoxyphenyl)boronic acid (120 mg) in the presence of dichlorobis(tricyclohexylphosphine)palladium (26 mg) and aqueous cesium carbonate solution (0.53 ml, 2.0 M) in DME (10 ml). After purification by flash column chromatography [silica gel, eluation gradient: cyclohexane / EtOAc, 1/0 to 1/1 (v/v)] the title compound was obtained as a solid.

MS: calc.: $C_{31}H_{33}F_2N_3O_6S$ (613.68) found: [$MNa^+$] = 636.06

**B18. Tert-butyl 4-[6-(2,5-dimethoxyphenyl)-1-(3-fluoro-4-methoxybenzyl)-2,4-dioxo-1,4-dihydrothieno[3,2-d]pyrimidin-3(2H)-yl]piperidine-1-carboxylate**

According to the procedure described for compound B15 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) was reacted with (2,5-dimethoxyphenyl)boronic acid (128 mg) in the presence of dichlo-robis(tricyclohexylphosphine)palladium (26 mg) and aqueous cesium carbonate solution (0.53 ml, 2.0 M) in DME (10 ml). After purification by flash column chromatography [silica gel, eluation gradient: cyclohexane / EtOAc, 1/0 to 1/1 (v/v)] the title compound was obtained as a solid.

MS: calc.: $C_{32}H_{36}FN_3O_7S$ (625.71) found: [$MNa^+$] = 648.10

**B19. Tert-butyl 4-[1-(3-fluoro-4-methoxybenzyl)-6-(2-fluorophenyl)-2,4-dioxo-1,4-dihydrothieno[3,2-d]pyrimidin-3(2H)-yl]piperidine-1-carboxylate**

According to the procedure described for compound B15 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) was reacted with (2-fluorophenyl)boronic acid (98 mg) in the presence of dichloro-bis(tricyclohexylphosphine)palladium (26 mg) and aqueous cesium carbonate solution (0.53 ml, 2.0 M) in DME (10 ml). After purification by flash column chromatography [silica gel, eluation gradient: cyclo-hexane / EtOAc, 1/0 to 1/1 (v/v)] the title compound was obtained as a solid.

MS: calc.: $C_{30}H_{31}F_2N_3O_5S$ (583.65) found: [$MNa^+$] = 606.06

**B20. Tert-butyl 4-[1-(3-fluoro-4-methoxybenzyl)-6-(4-fluorophenyl)-2,4-dioxo-1,4-dihydrothieno[3,2-d]pyrimidin-3(2H)-yl]piperidine-1-carboxylate**

According to the procedure described for compound B15 tert-butyl 4-[6-bromo-1-(3-fluoro-4-methoxybenzyl)-2,4-dioxo-1,4-dihydrothieno[3,2-d]pyrimidin-3(2H)-yl]piperidine-1-carboxylate (400 mg; compound B7) was reacted with (4-fluorophenyl)boronic acid (98 mg) in the presence of dichlorobis (tricyclohexylphosphine)palladium (26 mg) and aqueous cesium carbonate solution (0.53 ml, 2.0 M) in DME (10 ml). After purification by flash column chromatography [silica gel, eluation gradient: cyclohexane / EtOAc, 1/0 to 1/1 (v/v)] the title compound was obtained as a solid.

MS: calc.: $C_{30}H_{31}F_2N_3O_5S$ (583.65) found: [$MNa^+$] = 606.10

**B21. 1-(3-Fluoro-4-methoxybenzyl)-6-phenyl-3-piperidin-4-ylthieno[3,2-d]pyrimidine-2,4(1H,3H)-dione hydrochloride**

To a solution of tert-butyl 4-[1-(3-fluoro-4-methoxybenzyl)-2,4-dioxo-6-phenyl-1,4-dihydrothieno[3,2-d]pyrimidin-3(2H)-yl]piperidine-1-carboxylate (8.5 g; compound B8) in DCM (180 ml) was added a solution of hydrogen chloride in 1,4-dioxane (9.4 ml, 4.0 M). The reaction mixture was stirred for 2 h at RT. The volatiles were evaporated under vacuum until a final volume of about 35 ml was reached, diethyl ether was added (10 ml), the suspension was filtered off and the filter cake was washed with diethyl ether (10 ml). The solid was dried in vacuo at 55 °C to afford the title compound.

MS: calc.: $C_{25}H_{24}FN_3O_3S$ (465.55) found: [$MH^+$] = 466.13

**B22. 1-(3,5-Difluoro-4-methoxybenzyl)-6-phenyl-3-(piperidin-4-yl)thieno[3,2-d]pyrimidine-2,4(1H,3H)-dione hydrochloride**

Tert-butyl 4-[1-(3,5-difluoro-4-methoxybenzyl)-2,4-dioxo-6-phenyl-1,4-dihydrothieno[3,2-d]pyrimidin-3(2H)-yl]piperidine-1-carboxylate (1.28 g, compound B9) was dissolved in a solution of hydrogen chloride in 1,4-dioxane (12 ml, 4.0 M) and the reaction mixture was stirred for 45 min at RT. Afterwards all volatiles were evaporated under vacuum to give the title compound as a solid.

MS: calc.: $C_{25}H_{23}F_2N_3O_3S$ (483.54) found: [$MH^+$] = 484.18

**B23. 1-(2,3-Difluoro-4-methoxybenzyl)-6-phenyl-3-(piperidin-4-yl)thieno[3,2-d]pyrimidine-2,4(1H,3H)-dione hydrochloride**

Tert-butyl 4-[1-(2,3-difluoro-4-methoxybenzyl)-2,4-dioxo-6-phenyl-1,4-dihydrothieno[3,2-d]pyrimidin-3(2H)-yl]piperidine-1-carboxylate (1.28 g, compound B10) was dissolved in a solution of hydrogen chlo-ride in 1,4-dioxane (12 ml, 4.0 M) and the reaction mixture was stirred for 45 min at RT. Afterwards all volatiles were evaporated under vacuum to give the title compound as a solid.

MS: calc.: $C_{25}H_{23}F_2N_3O_3S$ (483.54) found: [$MH^+$] = 484.15

**B24. 6-(4-Fluoro-2-methoxyphenyl)-1-(3-fluoro-4-methylbenzyl)-3-(piperidin-4-yl)thieno[3,2-d]pyrimidine-2,4(1H,3H)-dione hydrochloride**

To a solution of tert-butyl 4-[6-(4-fluoro-2-methoxyphenyl)-1-(3-fluoro-4-methylbenzyl)-2,4-dioxo-1,4-dihydrothieno[3,2-d]pyrimidin-3(2H)-yl]piperidine-1-carboxylate (246 mg; compound B11) in DCM (8 ml) was added a solution of hydrogen chloride in 1,4-dioxane (2.0 ml, 4.0 M). The reaction mixture was stirred for 14 h at RT. Diethyl ether was added and the resulting precipitate was filtered off, washed with diethyl ether and dried in vacuo to afford the title compound as a solid.

MS: calc.: $C_{26}H_{25}F_2N_3O_3S$ (497.56) found: [$MH^+$] = 498.22

**B25. 1-Benzyl-6-(4-fluoro-2-methoxyphenyl)-3-(piperidin-4-yl)thieno[3,2-d]pyrimidine-2,4(1H,3H)-dione hydrochloride**

According to the procedure described for compound B24 tert-butyl 4-[1-benzyl-6-(4-fluoro-2-methoxyphenyl)-2,4-dioxo-1,4-dihydrothieno[3,2-d]pyrimidin-3(2H)-yl]piperidine-1-carboxylate (187 mg; compound B12) in DCM (8 ml) was reacted with a solution of hydrogen chloride in 1,4-dioxane (2.0 ml, 4.0 M) to afford the title compound as a solid.

MS: calc.: $C_{25}H_{24}FN_3O_3S$ (465.55) found: [$MH^+$] = 466.18

**B26. 1-(3-Fluoro-4-methoxybenzyl)-6-(4-fluoro-2-methylphenyl)-3-(piperidin-4-yl)thieno[3,2-d]pyrimidine-2,4(1H,3H)-dione hydrochloride**

According to the procedure described for compound B24 tert-butyl 4-[1-(3-fluoro-4-methoxybenzyl)-6-(4-fluoro-2-methylphenyl)-2,4-dioxo-1,4-dihydrothieno[3,2-d]pyrimidin-3(2H)-yl]piperidine-1-carboxylate (347 mg; compound B13) in DCM (10 ml) was reacted with a solution of hydrogen chloride in 1,4-dioxane (2.0 ml, 4.0 M) to afford the title compound as a solid.

MS: calc.: $C_{26}H_{25}F_2N_3O_3S$ (497.56) found: [$MH^+$] = 498.19

**B27. 1-(3-Fluoro-4-methoxybenzyl)-6-(2-methoxyphenyl)-3-(piperidin-4-yl)thieno[3,2-d]pyrimidine-2,4(1H,3H)-dione hydrochloride**

According to the procedure described for compound B24 tert-butyl 4-[1-(3-fluoro-4-methoxybenzyl)-6-(2-methoxyphenyl)-2,4-dioxo-1,4-dihydrothieno[3,2-d]pyrimidin-3(2H)-yl]piperidine-1-carboxylate (361 mg; compound B14) in DCM (10 ml) was reacted with a solution of hydrogen chloride in 1,4-dioxane (2.0 ml, 4.0 M) to afford the title compound as a solid.

MS: calc.: $C_{26}H_{26}FN_3O_4S$ (495.57) found: [$MH^+$] = 496.18

**B28. 6-(1,3-Benzodioxol-5-yl)-1-(3-fluoro-4-methoxybenzyl)-3-(piperidin-4-yl)thieno[3,2-d]pyrimidine-2,4(1H,3H)-dione hydrochloride**

To a solution of tert-butyl 4-[6-(1,3-benzodioxol-5-yl)-1-(3-fluoro-4-methoxybenzyl)-2,4-dioxo-1,4-dihydrothieno[3,2-d]pyrimidin-3(2H)-yl]piperidine-1-carboxylate (199 mg; compound B15) in DCM (20 ml) was added a solution of hydrogen chloride in 1,4-dioxane (2.0 ml, 4.0 M). The reaction mixture was stirred for 3 d at RT. All volatile materials were removed in vacuo to afford the title compound as a solid.

MS: calc.: $C_{26}H_{24}FN_3O_5S$ (509.56) found: [$MH^+$] = 510.21

**B29. 1-(3-Fluoro-4-methoxybenzyl)-6-(4-fluoro-2-methoxyphenyl)-3-(piperidin-4-yl)thieno[3,2-d]pyrimidine-2,4(1H,3H)-dione hydrochloride**

According to the procedure described for compound B28 tert-butyl 4-[1-(3-fluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate (136 mg; compound B16) in DCM (20 ml) was reacted with a solution of hydrogen chloride in 1,4-dioxane (2.0 ml, 4.0 M) to afford the title compound as a solid.

MS: calc.: $C_{26}H_{25}F_2N_3O_4S$ (513.56) found: [$MH^+$] = 514.18

**B30. 1-(3-Fluoro-4-methoxybenzyl)-6-(5-fluoro-2-methoxyphenyl)-3-(piperidin-4-yl)thieno[3,2-d]pyrimidine-2,4(1H,3H)-dione hydrochloride**

According to the procedure described for compound B28 tert-butyl 4-[1-(3-fluoro-4-methoxybenzyl)-6-(5-fluoro-2-methoxyphenyl)-2,4-dioxo-1,4-dihydrothieno[3,2-d]pyrimidin-3(2H)-yl]piperidine-1-carboxylate (196 mg; compound B17) in DCM (20 ml) was reacted with a solution of hydrogen chloride in 1,4-dioxane (2.0 ml, 4.0 M) to afford the title compound as a solid.

MS: calc.: $C_{26}H_{25}F_2N_3O_4S$ (513.56) found: [$MH^+$] = 514.20

**B31. 6-(2,5-Dimethoxyphenyl)-1-(3-fluoro-4-methoxybenzyl)-3-(piperidin-4-yl)thieno[3,2-d]pyrimidine-2,4(1H,3H)-dione hydrochloride**

According to the procedure described for compound B28 tert-butyl 4-[6-(2,5-dimethoxyphenyl)-1-(3-fluoro-4-methoxybenzyl)-2,4-dioxo-1,4-dihydrothieno[3,2-d]pyrimidin-3(2H)-yl]piperidine-1-carboxylate (201 mg; compound B18) in DCM (20 ml) was reacted with a solution of hydrogen chloride in 1,4-dioxane (2.0 ml, 4.0 M) to afford the title compound as a solid.

MS: calc.: $C_{27}H_{28}FN_3O_5S$ (525.60) found: [$MH^+$] = 526.19

**B32. 1-(3-Fluoro-4-methoxybenzyl)-6-(2-fluorophenyl)-3-(piperidin-4-yl)thieno[3,2-d]pyrimidine-2,4(1H,3H)-dione hydrochloride**

According to the procedure described for compound B28 tert-butyl 4-[1-(3-fluoro-4-methoxybenzyl)-6-(2-fluorophenyl)-2,4-dioxo-1,4-dihydrothieno[3,2-d]pyrimidin-3(2H)-yl]piperidine-1-carboxylate (165 mg; compound B19) in DCM (20 ml) was reacted with a solution of hydrogen chloride in 1,4-dioxane (2.0 ml, 4.0 M) to afford the title compound as a solid.

MS: calc.: $C_{25}H_{23}F_2N_3O_3S$ (483.54) found: [$MH^+$] = 484.18

**B33. 1-(3-Fluoro-4-methoxybenzyl)-6-(4-fluorophenyl)-3-(piperidin-4-yl)thieno[3,2-d]pyrimidine-2,4(1H,3H)-dione hydrochloride**

According to the procedure described for compound B28 tert-butyl 4-[1-(3-fluoro-4-methoxybenzyl)-6-(4-fluorophenyl)-2,4-dioxo-1,4-dihydrothieno[3,2-d]pyrimidin-3(2H)-yl]piperidine-1-carboxylate (279 mg; compound B20) in DCM (20 ml) was reacted with a solution of hydrogen chloride in 1,4-dioxane (2.0 ml, 4.0 M) to afford the title compound as a solid.

MS: calc.: $C_{25}H_{23}F_2N_3O_3S$ (483.54) found: [$MH^+$] = 484.17

**B34. 1-(2-Fluoro-4,5-dimethoxybenzyl)-6-phenyl-3-(piperidin-4-yl)thieno[3,2-d]pyrimidine-2,4(1H,3H)-dione hydrochloride**

Tert-butyl 4-[1-(2-fluoro-4,5-dimethoxybenzyl)-2,4-dioxo-6-phenyl-1,4-dihydrothieno[3,2-d]pyrimidin-3(2H)-yl]piperidine-1-carboxylate (1.31 g; compound B35) was suspended in a solution of hydrogen chloride in 1,4-dioxane (12.0 ml, 4.0 M). The reaction mixture was stirred for 45 min at RT. All volatile materials were removed in vacuo to afford the title compound as a solid.

MS: calc.: $C_{26}H_{26}FN_3O_4S$ (495.57) found: [$MH^+$] = 496.16

**B35. Tert-butyl 4-[1-(2-fluoro-4,5-dimethoxybenzyl)-2,4-dioxo-6-phenyl-1,4-dihydroth ieno[3,2-d]pyrimidin-3(2H)-yl]piperidine-1-carboxylate**

Potassium carbonate (323 mg) and 2-fluoro-4,5-dimethoxy-benzyl chloride (479 mg) were added to a solution of tert-butyl 4-(2,4-dioxo-6-phenyl-1,4-dihydrothieno[3,2-d]pyrimidin-3(2H)-yl)piperidine-1-carboxylate (1.0 g; compound

B2) in anhydrous DMF (10 ml) and the reaction mixture was stirred for 12 h at 100 °C under a nitrogen atmosphere. Afterwards the reaction mixture was poured into ice-cold water and the precipitated solid was filtered off, washed with water and dried in vacuo to give the title compound as a solid.

MS: calc.: $C_{31}H_{34}FN_3O_6S$ (595.69) found: [$MH^+$-Boc] = 496.19; [$MNa^+$] = 618.12

**B36. 1-(3-Fluoro-4-methylbenzyl)-6-phenyl-3-(piperidin-4-yl)thieno[3,2-d]pyrimidine-2,4(1H,3H)-dione hydrochloride**

To a solution of tert-butyl 4-[1-(3-fluoro-4-methylbenzyl)-2,4-dioxo-6-phenyl-1,4-dihydrothieno[3,2-d]pyrimidin-3(2H)-yl]piperidine-1-carboxylate (1.21 g; compound B37) in dioxane was added a solution of hydrogen chloride in 1,4-dioxane (2.75 ml, 4.0 M). The reaction mixture was stirred for 75 min at RT. All volatile materials were removed in vacuo to afford the title compound as a solid.

MS: calc.: $C_{25}H_{24}FN_3O_2S$ (449.55) found: [$MH^+$] = 450.19

**B37. Tert-butyl 4-[1-(3-fluoro-4-methylbenzyl)-2,4-dioxo-6-phenyl-1,4-dihydrothieno[3,2-d]pyrimidin-3(2H)-yl]piperidine-1-carboxylate**

Potassium carbonate (323 mg) and 4-(bromomethyl)-2-fluoro-1-methylbenzene (475 mg) were added to a solution of tert-butyl 4-(2,4-dioxo-6-phenyl-1,4-dihydrothieno[3,2-d]pyrimidin-3(2H)-yl)piperidine-1-carboxylate (1.0 g; compound B2) in anhydrous DMF (15 ml) and the reaction mixture was stirred for 12 h at 100 °C under a nitrogen atmosphere. Afterwards the reaction mixture was poured into ice-cold water and the precipitated solid was filtered off, washed with ice cold water (3x10 ml) and dried in vacuo at 58 °C to give the title compound as a solid.

MS: calc.: $C_{30}H_{32}FN_3O_4S$ (549.66) found: [$MH^+$ - Boc] = 450.18; [$MNa^+$] = 572.10

**B38. 1-Benzyl-6-phenyl-3-(piperidin-4-yl)thieno[3,2-d]pyrimidine-2,4(1H,3H)-dione hydrochloride**

Tert-butyl 4-(1-benzyl-2,4-dioxo-6-phenyl-1,4-dihydrothieno[3,2-d]pyrimidin-3(2H)-yl)piperidine-1-carboxylate (1.14 g; compound B39) was suspended in a solution of hydrogen chloride in 1,4-dioxane (12.0 ml, 4.0 M). The reaction mixture was stirred for 60 min at RT. All volatile materials were removed in vacuo to afford the title compound as a solid.

MS: calc.: $C_{24}H_{23}N_3O_2S$ (417.53) found: [$MH^+$] = 418.15

**B39. Tert-butyl 4-(1-benzyl-2,4-dioxo-6-phenyl-1,4-dihydrothieno[3,2-d]pyrimidin-3(2H)-yl)piperidine-1-carboxylate**

Potassium carbonate (323 mg) and benzyl bromide (0.28 ml) were added to a solution of tert-butyl 4-(2,4-dioxo-6-phenyl-1,4-dihydrothieno[3,2-d]pyrimidin-3(2H)-yl)piperidine-1-carboxylate (1.0 g; compound B2) in anhydrous DMF (10 ml) and the reaction mixture was stirred for 12 h at 100 °C under a nitrogen atmosphere. Afterwards the reaction mixture was poured into ice-cold water and the precipitated solid was filtered off, washed with water and dried in vacuo to give the title compound as a solid.

MS: calc.: $C_{29}H_{31}N_3O_4S$ (517.65) found: [$MH^+$] = 518.79 [$MH^+$ - Boc] = 418.11

**B40. Tert-butyl 4-[1-(3,5-difluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate**

According to the procedure described for compound B11 tert-butyl 4-[6-(4-fluoro-2-methoxyphenyl)-2,4-dioxo-1,4-dihydrothieno[3,2-d]pyrimidin-3(2H)-yl]piperidine-1-carboxylate (400 mg; compound B6) and 5-(bromomethyl)-1,3-difluoro-2-methoxybenzene (199 mg) were reacted in the presence of potassium carbonate (116 mg) in DMF (10 ml) to yield the title compound as a solid.

MS: calc.: $C_{31}H_{32}F_3N_3O_6S$ (631.67) found: [$MNa^+$] = 654.05

**B41. 1-(3,5-Difluoro-4-methoxybenzyl)-6-(4-fluoro-2-methoxyphenyl)-3-(piperidin-4-yl)thieno[3,2-d]pyrimidine-2,4(1H,3H)-dione hydrochloride**

According to the procedure described for compound B24 tert-butyl 4-[1-(3,5-difluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate (210 mg; compound B40) in DCM (8 ml) was reacted with a solution of hydrogen chloride in 1,4-dioxane (2.0 ml, 4.0 M) to afford the title compound as a solid.

MS: calc.: $C_{26}H_{24}F_3N_3O_4S$ (531.55) found: [$MH^+$] = 532.19

**B42. Tert-butyl 4-[1-(2,3-difluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate**

According to the procedure described for compound B11 tert-butyl 4-[6-(4-fluoro-2-methoxyphenyl)-2,4-dioxo-1,4-dihydrothieno[3,2-d]pyrimidin-3(2H)-yl]piperidine-1-carboxylate (400 mg; compound B6) and 1-(bromomethyl)-2,3-difluoro-4-methoxybenzene (199 mg) were reacted in the presence of potassium carbonate (116 mg) in DMF (10 ml) to yield the title compound as a solid.

MS: calc.: $C_{31}H_{32}F_3N_3O_6S$ (631.67) found: [$MNa^+$] = 654.07

**B43. 1-(2,3-Difluoro-4-methoxybenzyl)-6-(4-fluoro-2-methoxyphenyl)-3-(piperidin-4-yl)thieno[3,2-d]pyrimidine-2,4(1H,3H)-dione hydrochloride**

According to the procedure described for compound B24 tert-butyl 4-[1-(2,3-difluoro-4-methoxybenzyl)-6-(4-fluoro-2-methoxyphenyl)-2,4-dioxo-1,4-dihydrothieno[3,2-d]pyrimidin-3(2H)-yl]piperidine-1-carboxylate (261 mg; compound B42) in DCM (8 ml) was reacted with a solution of hydrogen chloride in 1,4-dioxane (2.0 ml, 4.0 M) to afford the title compound as a solid.

MS: calc.: $C_{26}H_{24}F_3N_3O_4S$ (531.55) found: [$MH^+$] = 532.16

**C1. Methyl 3-[(4aR,10bR)-9-ethoxy-8-methoxy-3,4,4a,10b-tetrahydro-1H-thiopyrano[4,3-c]isoquinolin-6-yl]benzoate**

To a solution of methyl 3-{[(3R,4R)-3-(3-ethoxy-4-methoxyphenyl)tetrahydro-2H-thiopyran-4-yl]carbamoyl}benzoate (2.58 g; compound C5) and 2-chloropyridin (0.68 ml) in toluene (60 ml) was slowly added a solution of POCls (1.65 ml) in toluene (6 ml). The reaction mixture was stirred for 12 h at 100 °C. The reaction mixture was allowed to come to RT and then quenched by slow addition of water (15 ml). Additional water (30 ml) was added and the pH was adjusted to about pH 9 by addition of 5 M aqueous solution of sodium hydroxide. The organic phase was separated and the aqueous phase was extracted with toluene (30 ml). The combined organic phases were dried over sodium sulfate and the solvent was removed in vacuo. The resulting residue was purified by flash column chromatography [silica gel, eluation gradient: cyclohexane/EtOAc, 3/1 to 1/1 (v/v)] to yield the title compound as a solid.
MS: calc.: $C_{23}H_{25}NO_4S$ (411.52) found: [$MH^+$] = 411.9

**C2. 3-[(4aR,10bR)-9-ethoxy-8-methoxy-3,4,4a,10b-tetrahydro-1H-thiopyrano[4,3-c]isoquinolin-6-yl]benzoic acid**

To a solution of methyl 3-[(4aR,10bR)-9-ethoxy-8-methoxy-3,4,4a,10b-tetrahydro-1H-thiopyrano[4,3-c]isoquinolin-6-yl]benzoate (0.5 g; compound C1) in 1,4-dioxane (8 ml) was added an 2 M aqueous solution of sodium hydroxide (1.3 ml) and the reaction mixture was stirred at RT for 12 h. An aqueous solution of hydrogen chloride (1.3 ml, 2.0 M) was added under stirring. The reaction mixture was evaporated to dryness in vacuo and the resulting residue was used for the next step without further purification.

MS: calc.: $C_{22}H_{23}NO_4S$ (397.49) found: [$MH^+$] = 398.23

**C3. Methyl 4-[(4aR,10bR)-9-ethoxy-8-methoxy-3,4,4a,10b-tetrahydro-1H-thiopyrano[4,3-c]isoquinolin-6-yl]benzoate**

To a suspension of methyl 4-{[(3R,4R)-3-(3-ethoxy-4-methoxyphenyl)tetrahydro-2H-thiopyran-4-yl]carbamoyl}benzoate (20.5 g; compound C6) and potassium carbonate (6.6 g) in ACN (400 ml) was slowly added a solution of $POCl_3$(13.2 ml) in CAN (50 ml) at 0 °C. Subsequently the mixture was heated under reflux for 17 h. The reaction was quenched by addition of water (300 ml) at RT and the pH value was adjusted to pH 7.6 by addition of a 40% aqueous sodium hydroxide solution. The ace-tonitrile was removed in vacuo and diethyl ether was added to the suspension. The pH was adjusted to pH 8.6 by further addition of a 40% aqueous sodium hydroxide solution and a saturated solution of sodium hydrogen carbonate. After extraction the organic phase was separated, washed with water and dried over sodium sulfate. Filtration and evaporation of the solvent gave a solid that was purified by flash chromatography (silica gel, eluation gradient: n-hexane / isopropyl acetate, 19/1 to 12/8 (v/v)) to yield the title compound as a solid.

MS: calc.: $C_{23}H_{25}NO_4S$ (411.52) found: [$MH^+$] = 412.2

**C4. 4-[(4aR,10bR)-9-ethoxy-8-methoxy-3,4,4a,10b-tetrahydro-1H-thiopyrano[4,3-c]isoquinolin-6-yl]benzoic acid**

To a solution of methyl 4-[(4aR,10bR)-9-ethoxy-8-methoxy-3,4,4a,10b-tetrahydro-1H-thiopyrano[4,3-c]isoquinolin-6-yl]benzoate (5.0 g; compound C3) in dioxane (40 ml) was added a 2.0 M aqueous sodium hydroxide solution (18.3 ml) and the reaction mixture was stirred for 1.5 h at RT. Afterwards an aqueous hydrogen chloride solution (1.83 ml, 2.0 M) was added and all volatiles were removed under vacuo to give the title compound with parts of sodium chloride.

MS: calc.: $C_{22}H_{23}NO_4S$ (397.49) found: [$MH^+$] = 398.2

**C5. Methyl 3-{[(3R,4R)-3-(3-ethoxy-4-methoxyphenyl)tetrahydro-2H-thiopyran-4-yl]carbamoyl}benzoate**

To a suspension of (3R,4R)-3-(3-ethoxy-4-methoxyphenyl)tetrahydro-2H-thiopyran-4-amine hydrochloride (1.82 g; compound C7), 3-(methoxycarbonyl)benzoic acid (1,08 g) and HBTU (2,50 g) in DCM (50 ml) was added DIPEA (4.2 ml) and the mixture was stirred for 1 h at RT. A saturated aqueous solution of sodium hydrogen carbonate (30 ml) was added, the organic phase was separated and dried over sodium sulfate. After filtration the solvent was removed in vacuo and the resulting residue was purified by flash column chromatography [silica gel, eluation gradient: DCM / MeOH, 100 / 0 to 95 / 5 (v/v)] to give the title compound as a solid.

MS: calc.: $C_{23}H_{27}NO_5S$ (429.35) found: [$MH^+$] = 430.0

**C6. Methyl 4-{[(3R,4R)-3-(3-ethoxy-4-methoxyphenyl)tetrahydro-2H-thiopyran-4-yl]carbamoyl}benzoate**

To a suspension of 4-(methoxycarbonyl)benzoic acid (11.35 g) in DCM (300 ml) were added 5 drops of DMF and the reaction mixture was stirred for 20 min at RT under a nitrogen atmosphere. To this supension oxalyl chloride (5.67 ml) dissolved in DCM (60 ml) was slowly added at 0 °C.The ice bath was removed and the mixture was stirred for 4 h at RT. DCM (3 x 200 ml) was added to the reaction mixture and the volatiles were co-evaporated under reduced pressure (three times). Finally all volatiles were removed under vacuo to obtain an oil, which was dissolved in DCM (200 ml) and added dropwise to a solution of (3R,4R)-3-(3-ethoxy-4-methoxyphenyl)tetrahydro-2H-thiopyran-4-amine hydrochloride (18.23 g; compound C7) and DIPEA (41.1 ml) in DCM (200 ml) at 0 °C under nitrogen atmosphere. After stirring for 12 h at RT the reaction was quenched by addition of a saturated aqueous solution of sodium hydrogen carbonate. The organic phase was separated, dried over sodium sulfate, filtered and evaporated to dryness in vacuo. The resulting residue was suspended in DIP (300 ml) and heated under reflux for 3.5 h. The suspension was stirred for 2 d at RT and subsequently cooled to 0° (ice bath). The suspension was filtered and the filter cake was washed with little amounts of DIP and dried in vacuo for 12 h at 60 °C to give the title compound as a solid.

MS: calc.:$C_{23}H_{27}NO_5S$ (429.35) found: [$MH^+$] = 430.0

**C7. (3R,4R)-3-(3-Ethoxy-4-methoxy-phenyl)-tetrahydro-2H-thiopyran-4-amine hydrochloride**

A mixture of (3R,4R)-3-(3-Ethoxy-4-methoxy-phenyl)-N-[(1R)-1-(4-methoxy-phenyl)-ethyl]-tetrahydro-2H-thiopyran-4-amine hydrochloride (1.0 g; compound C8) and trifuoroacetic acid (2 ml) was stirred at reflux temperature for

30 min yielding a dark red solution. Cooled to RT the solution was evaporated, the dark viscous residue was dissolved in a mixture of diethylether (5 ml) and water (5 ml) and the pH of the solution is increased up to pH 10 by adding some drops of a 40 % aqueous solution of sodium hydroxide. The mixture was extracted with diethylether, the organic phase was washed with a saturated aqueous solution of $NaHCO_3$ and then extracted two times with a 20 % aqueous solution of citric acid. The pH of the collected aqueous acid solutions (about pH 2.4) was increased up to pH 10 by adding a 40 % NaOH solution and the basic mixture was extracted with diethylether. The organic phase was washed two times with water, dried over sodium sulfate and then evaporated to give an oily residue. This was dissolved in 2-propanol (10 ml) and to the stirred solution a 5-6 M solution of hydrogen chloride in 2-propanol (1 ml) was added dropwise at RT inducing a spontaneous crystallization. The slurry was concentrated to about half of the volume and stirred for 15 h at RT. The cyrstalls were filtered off, washed with 2-propanol (2 ml), dried in vacuo at 40 ° C to give the title compound. M. p.: 233 °C (decomposition).

MS: calc.: $C_{14}H_{21}NO_2S$ (267.85) found: [MH+] 268.0
$[d]_D^{20}$= -40.8 ° (MeOH, c = 1)

**C8. (3R,4R)-3-(3-Ethoxy-4-methoxy-phenyl)-N-[(1R)-1-(4-methoxy-phenyl)-ethyl]-tetrahydro-2H-thiopyran-4-amine hydrochloride**

3-(3-Ethoxy-4-methoxy-phenyl)-tetrahydro-4H-thiopyran-4-one (0.67 g; compound C9) and (R)-1-(4-methoxy-phenyl)-ethanamine were dissolved at RT in DCM (12.5 ml). Keeping the temperature at about 20 °C acetic acid (0.3 g) was added dropwise to the solution followed by addition of sodiumtriacetoxyborohydride (0.84 g) and the mixture was stirred for 15 h at RT. After evaporation of about 11 ml of the solvent the residue was extracted with diethylether (8 ml) and with a 20 % aqueous solution of citric acid (three times with 2.5 ml each). The collected aqueous acid solution was washed two times with diethylether (2 ml each) and the pH was increased from of about pH 2.4 up to pH 6.0 by adding a 40 % aqueous solution of sodium hydroxide.Then the solution was extracted with diethylether (three times with 4 ml each), the collected organic phase was washed two times with water (2 ml each), filtered and concentrated in vacuo to dryness yielding a solid residue. This was dissolved in 2-propanol (12 ml) at about 60 °C and a concentrated aqueous solution of hydrogen chloride (0.32 ml) was added. Keeping the temperature at about 60 °C the solution was stirred for about 1 h while a slow crystallization occured. The suspension was then heated to reflux temperature for about 1 h and then slowly cooled to RT and stirred for additional 15 h. The suspension was filtered off and the solid filter residue was washed with 2-propanol (1 ml) yielding a crystalline material. This was suspended in 2-propanol (11 ml), the suspension was heated to reflux temperature and water (1.6 ml) was added giving a clear solution. This was slowly cooled down to about 70 °C under continuous stirring inducing a spontaneous crystallization. Following further stirring at 70 °C for about 1 h the suspension was slowly cooled down to RT and stirred for additional 15 h. The crystals were filtered off, washed with 2-propanol (1 ml) and dried in vacuo at about 50 °C for 24 h yielding the title compound.
M.p.: 214 -214.5 °C (decomposition).

MS: calc.: $C_{23}H_{31}NO_3S$ (401.57) found: $[MH^+]$ 401.7
$[d]_{D20}$ = +82.5 ° (MeOH, c = 1)

**C9. 3-(3-Ethoxy-4-methoxy-phenyl)-tetrahydro-2H-thiopyran-4-one**

To a suspension of 3-(3-ethoxy-4-methoxy-phenyl)-1-methyl-piperidin-4-one (0.88 g) in 8.5 ml of 4-methyl-pentan-2-one, which was cooled to about 2 °C, trifluoro-methansulfonic acid methylester (0.6 g) was added dropwise within about 9 min. Following 20 min stirring at RT a combined solution of sodium hydrogensulfide monohydrate (0.82 g) and sodium sulfide nonahydrate (0.98 g) in 8.5 ml of water was added and the stirred reaction mixture was then heated to reflux temperature for 4 h. Cooled to RT the stirring was stopped and the building up organic layer was separated. The water phase was washed three times with 2 ml each of EtOAc. The collected organic phase was washed two times with 3 ml each of water, filtered and concentrated in vacuo giving a solid residue. This was suspended in 2-propanol (4 ml) and the suspension was heated to reflux temperature giving a clear solution. The continuously stirred solution was slowly cooled down to RT inducing a spontaneous crystallization. Following stirring for 15 h at RT, the suspension was further stirred at about 2 °C for 2 h and then the crystals were filtered of, washed with 2-propanol (1 ml) and dried in vacuo at about 60 °C for 24 h yielding the title compound.

M. p.: 108.5 - 109.5 °C
MS: calc.: $C_{14}H_{18}O_3S$ (266.36) found: $[MH^+]$ 266.2

Commercial Utility

Medical Uses

The compounds of formula (1) and the stereoisomers of the compounds of formula (1) according to the invention are hereinafter referred to as the compounds of the invention. In particular, the compounds of the invention are pharmaceutically acceptable.

The compounds of the invention have - as dual-selective type 4/type 5 phosphodiesterase (PDE4/5) inhibitors - valuable pharmaceutical properties, which make them commercially utilizable.

PDE4 inhibitors are thought to be useful in the treatment or prophylaxis of a variety of diseases and disorders. They are thought to be suitable on the one hand as bronchial therapeutics (for the treatment of airway obstructions on account of their effects to curb pulmonary inflammation, lung fibrotic remod-ling, lung parenchymal destruction, mucociliary malfunction, oxidative stress, pulmonary vascular remodelling but on the other hand especially for the treatment of disorders, in particular of an inflammatory nature, e.g. of the airways, of the skin, of the intestine, of the eyes, of the CNS and of the joints, which are mediated by mediators such as histamine, PAF (platelet-activating factor), arachidonic acid derivatives such as leukotrienes and prostaglandins, cytokines, interleukins, chemokines, alpha-, beta-and gamma-interferon, tumor necrosis factor $\alpha$ (TNF$\alpha$) or oxygen free radicals and proteases.

In particular, PDE4 inhibitors are thought to be useful in the treatment or prophylaxis of a variety of diseases and disorders, such as for example:

acute and chronic airway diseases, such as, but not limited to, chronic bronchitis, allergic bronchitis, bronchial asthma, emphysema, COPD (chronic obstructive pulmonary disease), bronchiolitis obliterans (BOS) and interstitial lung disease such as pulmonary fibrosis;

pulmonary hypertension;

diseases which are based on allergic and/or chronic, immunological false reactions in the region of the upper airways (pharynx, nose) and the adjacent regions (paranasal sinuses, eyes), such as, but not limited to, allergic rhinitis/sinusitis, chronic rhinitis/sinusitis, allergic conjunctivitis and also nasal polyps; ocular inflammatory diseases such as, but not limited to, uveitis, scleritis, keratitis, retinal vasculitis, age-related macula degeneration, diabetic nephropathy, and chronic and allergic conjunctivitis; dermatological diseases especially of proliferative, inflammatory and allergic type, such as, but not limited to psoriasis (vulgaris), toxic and allergic contact eczema, atopic dermatitis (eczema), seborrhoeic eczema, Lichen simplex, sunburn, pruritus in the anogenital area, alopecia areata, hypertrophic scars, discoid lupus erythematosus, follicular and widespread pyodermias, endogenous and exogenous acne, acne rosacea and other proliferative, inflammatory and allergic skin disorders; diseases to which excessive release of TNF$\alpha$ and leukotrienes may contribute, such as, for example, diseases of the arthritis type like rheumatoid arthritis, rheumatoid spondylitis, osteoarthritis and other arthritic conditions;

fibrotic diseases, such as, but not limited to, cystic fibrosis, pulmonary fibrosis, hepatic fibrosis renal fibrosis, myelofibrosis, retroperitoneal fibrosis, endomyocardial fibrosis, mediastinal fibrosis, nephrogenic systemic fibrosis, hypertrophic scars or toxic liver damage;

viral, alcoholic or drug-induced acute and fulminant hepatitis, hepatic steatosis (alcoholic and non-alcoholic steatio-hepatitis);

diseases of the immune system, such as, but not limited to, AIDS, multiple sclerosis, graft versus host reaction, acute allograft rejections, but also chronic graft versus host disease (CGVHD) after allogeneic hematopoietic stem-cells transplantation (HSCT);

cachexia, cancer cachexia, AIDS cachexia;

types of shock, such as, but not limited to, septic shock, endotoxin shock, gram-negative sepsis, toxic shock syndrome and ARDS (adult respiratory distress syndrome);

diseases in the gastrointestinal region, such as Crohn’s disease and ulcerative colitis;

diseases of the heart which can be treated by PDE inhibitors, such as cardiac insufficiency;

diseases which can be treated on account of the tissue-relaxant action of the PDE4 inhibitors, such as, for example, oncolytic action (to treat preterm delivery);

renal diseases such as nephritis such as glomerulonephritis, diabetic nephropathy and urinary tract infections;

diabetes insipidus, diabetes mellitus (type I and in particular type II); cancer (in particular lymphoid and myeloid leukaemia); osteoporosis;

conditions associated with cerebral metabolic inhibition, such as, but not limited to, cerebral senility, senile dementia (Alzheimer’s disease), memory impairment associated with Parkinson’s disease or multiinfarct dementia;

and also diseases of the central nervous system, such as, but not limited to, depressions, anxiety states, spinal cord injury, schizophrenia or arteriosclerotic dementia.

PDE5 inhibitors are thought to be able to influence the physiological and pathophysiological function of various cells, e.g., but not limited to, smooth muscle cells, fibroblasts, myofibroblasts and platelets, which are involved in a great variety of physiological and pathophysiological mechanisms. In particular, PDE5 inhibitors are thought to be able to effect relaxation of the vasculature, thus increasing blood flow, improve the spatial balance between blood perfusion and ventilation within the lung ("re-matching" effect) thereby reducing the amount of so-called low V/Q-areas [areas within the lung with high perfusion (Q) but no or reduced ventilation (V)] and high V/Q-areas (areas within the lung with low perfusion but high ventilation), altogether resulting in reduced shunt-flow induce neurogenesis, inhibit platelet function, such as aggregation, adhesion and mediator release and, thus, have an anti-inflammatory effect.

In particular, PDE5 inhibitors are thought to be useful in the treatment or prophylaxis of a variety of diseases and disorders, such as for example:

male and female sexual dysfunction, such as, but not limited to, male erectile dysfunction, premature ejaculation, Peyronie's disease;
acute and chronic airway diseases, such as, but not limited to, COPD (chronic obstructive pulmonary disease), bronchitis, emphysema, pulmonary vascular remodeling, interstitial lung disease such as idiopathic pulmonary lung fibrosis (IPF), asthma, cystic fibrosis, bronchiectasis, bronchiolitis obliterans,
connective tissue diseases, sarcoidosis, kyphoscoliosis, pneumoconiosis, amyotrophic lateral sclerosis, thoracoplasty, extrinsic allergic alveolitis;
pulmonary hypertension;
inflammatory diseases, such as, but not limited to, vasculature inflammation, acute respiratory distress syndrome, nephritis, mesangial glomerulonephritis, chronic inflammatory bowel disease, disseminated intravascular inflammation, allergic vasculitis, dermatoses (e.g., but not limited to, psoriasis, toxic and
allergic contact eczema, atopic eczema, seborrhoeic eczema, Lichen simplex, sunburn, pruritus in the anogenital area, alopecia areata, hypertrophic scars, discoid lupus erythematosus, follicular and
widespread pyodermias, endogenous and exogenous acne, acne rosacea), disorders of the arthritis type (e.g., but not limited to, rheumatoid arthritis, rheumatoid spondylitis, osteoarthritis), disorders of the immune system [e.g., but not limited to, AIDS (acquired immunodeficiency syndrome), multiple sclerosis], graft versus host reaction, allograft rejections, shock [e.g., but not limited to, septic shock,
endotoxin shock, gram-negative sepsis shock, toxic shock syndrome and ARDS (adult respiratory distress syndrome)], gastrointestinal inflammations (e.g., but not limited to, Crohn's disease and
ulcerative colitis); disorders which are based on allergic and/or chronic, immunological false reactions (e.g., but not limited to, allergic rhinitis, allergic sinusitis, chronic rhinitis, chronic sinusitis, allergic conjunctivitis, nasal polyps);
pain, such as, but not limited to, inflammatory pain;
right-heart failure, right heart hypertrophy (cor pulmonale), hypertension, hypercholesterolemia,
hypertriglyceridemia; diabetes mellitus (type I and type II);
ischaemic diseases, such as, but not limited to, stroke, coronary artery disease, angina (including, but
not limited to, vasospastic angina), myocardial infarction, peripheral artery disease, cerebrovascular obstruction, sleep apnea, macular ischaemia, arterial and venous occlusion, congestive heart failure;
ocular inflammatory diseases such as, but not limited to, uveitis, scleritis, keratitis, retinal vasculitis,
age-related macula degeneration, diabetic nephropathy, and chronic and allergic conjunctivitis;
diabetic gastroparesis and diseases with symptoms of gastroparesis;
diseases or conditions in which it is desirable to suppress platelet function, for example, but not limited to, after stent implantations (e.g., but not limited to, coronary stenting), after bypass operations, in pulmonary hypertension, thrombotic diseases, post-angioplasty stenosis, coronary artery disease,
infarction (e.g., but not limited to, myocardial infarction), instable angina pectoris, stroke, and arterial and venous occlusion diseases (e.g., but not limited to, claudicatio intermittens);
diseases or conditions with an impairment or dysfunction of cerebral vascular reactivity and/or
neurovascular coupling, such as, but not limited to, arteriosclerotic dementia, multi-infarct dementia,
cerebral senility;
diseases which are based on neuronal damage or degradation, such as but not limited to, stroke,
spinal cord injury, brain injury, morbus parkinson, amyotrophic lateral sclerosis, morbus alzheimer, amyloidosis, prion diseases and neuropathy;
peripheral arterial diseases, chronic renal failure, chronic heart failure, sepsis, senile dementia (Alzheimer's disease), Creutzfeld-Jacob disease, septic encephalopathy, arteriosclerotic encephalopathy, diabetes associated encephalopathy, toxic encephalopathy, vascular and neuronal dementia, Huntington's disease, Parkinson's disease, multiple sclerosis and preeclampsia;
portal hypertension, liver cirrhosis, toxic liver damage (e.g., but not limited to, alcohol-induced liver damage), hepatitis, thrombosis of the portal vein, Budd-Chiari syndrome, malformation of liver veins,

compression of liver veins (e.g., but without limitation, due to tumors), arteriovenous fistula, diseases associated with an enlarged spleen, schistosomiasis (bilharziosis), sarcoidosis and other granulomatous diseases, primary biliary cirrhosis, myeloproliferative disorders (e.g., but not limited to, chronic myeloid leukemia, osteomyelofibrosis), lymphatic systemic diseases, collagenosis (e.g., but not limited to, systemic lupus erythematodes, sclerodermia), morbus Osier (congenital arteriovenous malformations, inter alia in the liver), nodular regenerative hyperplasia, tricuspid insufficiency, pericarditis constrictiva, veno-occlusive disease (VOD), non-alcoholic steatohepatitis (NASH); fibrotic diseases, such as, but not limited to, cystic fibrosis, pulmonary fibrosis, hepatic fibrosis renal fibrosis, myelofibrosis, retroperitoneal fibrosis, endomyocardial fibrosis, mediastinal fibrosis, nephrogenic systemic fibrosis, hypertrophic scars or toxic liver damage;
benign prostatic hyperplasia;
insufficient uteroplacental blood flow in pregnancies with fetal growth restriction;
insufficient brain skills, such as but not limited to, verbal attainment, attention, concentration, deductive thinking, central auditory processing, cognition, learning, vigilance, apprehension and reagibility;
Overactive Bladder; LUTS = lower urinary tract symptoms; Raynauds syndrome/phenomenon.

In this respect, the term "pulmonary hypertension" in particular embraces

- pulmonary arterial hypertension including primary pulmonary hypertension (e.g. sporadic or familial) and pulmonary arterial hypertension related, for example, but without limitation, to collagen vascular disease, congenital systemic-to-pulmonary shunts, portal hypertension, human immunodeficiency virus infection, drugs or toxins (e.g., but not limited to, anorexigens), persistent pulmonary hypertension of the newborn;
- pulmonary venous hypertension due to, for example, but without limitation, left-sided atrial or ventricular heart disease, left-sided valvular heart disease, extrinsic compression of central pulmonary veins (e.g. fibrosing mediastinitis, adenopathy in relation to tumors), pulmonary veno-occlusive disease;
- pulmonary hypertension associated with disorders of the respiratory system or hypoxemia including, for example, but without limitation, chronic obstructive pulmonary disease (COPD), interstitial lung disease, sleep-disordered breathing, alveolar hypoventilation disorders, chronic exposure to high altitude, neonatal lung disease, alveolar-capillary dysplasia;
- pulmonary hypertension caused by chronic thrombotic or embolic diseases including thromboembolic obstruction of proximal pulmonary arteries and obstruction of distal pulmonary arteries, such as pulmonary embolism (due to thrombus, tumor, ova, parasites, or foreign material), in situ thrombosis and sickle-cell disease, in particular chronic thromboembolic pulmonary hypertension (CTEPH);
- pulmonary hypertension caused by disorders directly affecting the pulmonary vasculature including inflammatory disorders (e.g., but not limited to, schistosomiasis, sarcoidosis) and pulmonary capillary hemangiomatosis.

It is noteworthy that compounds of the invention, which are inhibitors of type 4 phosphodiesterase (PDE4) and of type 5 phosphodiesterase (PDE5), have the potential to be more effective in treatment of distinct disease identities than compounds inhibiting only one of those two enzymes, since inhibition of PDE4 and PDE5 might address diverse and different pathophysiologies occuring within one disease state as e.g. lung fibrosis.

In respect to lung fibrosis it has been described that inhibitors of type 4 phosphodiesterase inhibit TGF-P induced transition of lung fibroblasts to myofibroblasts (Dunkern et al., Eur. J. Pharmacol., 572(1): 12-22, 2007), which is a hallmark of fibrosis progression. They have further been described to inhibit matrix metalloproteinase production from lung fibroblasts (Martin-Chouly CA et al., Life Sci. 75(7): 823-40, 2004) and to prevent chemotaxis of these cells (Kohyama T et al., Am. J. Respir. Cell Mol. Biol., 26(6): 694-701, 2002), which are important pathophysiological aspects of lung fibrosis. In addition the selective type 4 phosphodiesterase inhibitor roflumilast was shown to alleviate bleomycin-induced lung fibrotic remodeling in mice and rat in preventive and therapeutic protocols outperforming glucocorticoids in the latter to inhibit fibrosis development (Cortijo J et al., Br. J. Pharmacol., 156(3): 534-44, 2009).

On the other hand it has been shown in respect to lung fibrosis that PDE5 inhibition by means of the selective PDE5 inhibitor sildenafil attenuates bleomycin-induced pulmonary fibrosis and pulmonary hypertension through inhibition of ROS generation and RhoA/Rho kinase activation (Hemnes AR, Zaiman A, Champion HC, Am. J. Physiol. Lung Cell. Mol. Physiol. 2008 Jan;294(1):L24-33. Epub 2007 Oct 26) and it has been shown in clinical human open-label trials that sildenafil improves lung hemodynamic parameters (vascular resistance and ventilation/perfusion matching) and increases exercise tolerance in patients with pulmonary fibrosis (Ghofrani et al., Lancet 360, 895-900, 2002; Collard et al., Chest 131, 897-899, 2007).

Accordingly, the invention further relates to the compounds of the invention for use in the treatment or prophylaxis of diseases, especially diseases alleviated by inhibition of type 4 and type 5 phosphodiesterase, in particular the diseases exemplified above.

Preferably, the invention relates to the compounds of the invention for use in the treatment or prophylaxis of

the following diseases:

acute and chronic airway diseases, such as interstitial lung disease such as pulmonary fibrosis, cystic fibrosis, bronchial asthma, chronic bronchitis, allergic bronchitis, allergic rhinitis, emphysema, chronic obstructive pulmonary disease (COPD) and COPD associated with pulmonary hypertension;
pulmonary hypertension, in particular thromboembolic pulmonary hypertension;
dermatological diseases, such as psoriasis and atopic dermatitis (eczema);
ocular diseases, such as uveitis, scleritis, keratitis, retinal vasculitis, age-related macula degeneration, diabetic nephropathy, and chronic and allergic conjunctivitis;
rheumatoid arthritis; and
inflammations in the gastrointestinal region, such as Crohn's disease and ulcerative colitis.

The invention also relates to the use of a compound of the invention in the manufacture of a pharmaceutical composition inhibiting the type 4 and type 5 phosphodiesterase, in particular a pharmaceutical composition for the treatment or prophylaxis of diseases alleviated by inhibition of type 4 and type 5 phosphodiesterase, preferably, a pharmaceutical composition for the treatment or prophylaxis of the diseases exemplified above.

In particular, the invention relates to the use of a compound of the invention in the manufacture of a pharmaceutical composition for the treatment or prophylaxis of an acute or chronic airway disease, such as, but not limited to, interstitial lung disease, pulmonary fibrosis, cystic fibrosis, bronchial asthma, chronic bronchitis, emphysema, chronic obstructive pulmonary disease (COPD) or COPD associated with pulmonary hypertension.

The invention also relates to the use of a compound of the invention in the manufacture of a pharmaceutical composition for the treatment or prophylaxis of pulmonary hypertension or thromboembolic pulmonary hypertension.

The invention relates also to the use of a compound of the invention in the manufacture of a pharmaceutical composition for the treatment or prophylaxis of allergic rhinitis or allergic asthma.

Furthermore, the invention relates to the use of a compound of the invention in the manufacture of a pharmaceutical composition for the treatment or prophylaxis of dermatological diseases, such as, but not limited to, psoriasis or atopic dermatitis (eczema).

In addition, the invention relates to the use of a compound of the invention in the manufacture of a pharmaceutical composition for the treatment or prophylaxis of ocular diseases, such as, but not limited to uveitis, scleritis, keratitis, retinal vasculitis, age-related macula degeneration, diabetic nephropathy, or chronic or allergic conjunctivitis.

The invention relates as well to the use of a compound of the invention in the manufacture of a pharmaceutical composition for the treatment or prophylaxis of rheumatoid arthritis.

Additionally, the invention relates to the use of a compound of the invention in the manufacture of a pharmaceutical composition for the treatment or prophylaxis of inflammations in the gastrointestinal region, such as, but not limited to, Crohn's disease or ulcerative colitis.

In a particularly preferred embodiment of the invention, in the above-mentioned uses the compound of the invention is a compound of the examples according to the invention.

The disclosure further relates to a method of treating or preventing a disease comprising administering to a patient in need thereof a therapeutically effective amount of at least one of the compounds of the invention.

In particular, the disclosure relates to a method of treating or preventing one of the above mentioned diseases comprising administering to a patient in need thereof a therapeutically effective amount of at least one of the compounds of the invention.

Especially, the disclosure relates to a method of treating or preventing a disease, which is alleviated by inhibition of the type 4 and type 5 phosphodiesterase comprising administering to a patient in need thereof a therapeutically effective amount of at least one of the compounds of the invention.

Preferably, the disclosure relates to a method of treating or preventing an acute or chronic airway disease, for example, but not limited to interstitial lung disease, pulmonary fibrosis, cystic fibrosis, bronchial asthma, chronic bronchitis, emphysema, chronic obstructive pulmonary disease (COPD) or COPD associated with pulmonary hypertension comprising administering to a patient in need thereof a therapeutically effective amount of at least one of the compounds of the invention.

The disclosure relates also to a method of treating or preventing pulmonary hypertension or thromboembolic pulmonary hypertension comprising administering to a patient in need thereof a therapeutically effective amount of at least one of the compounds of the invention.

The disclosure relates also to a method of treating or preventing allergic rhinitis or allergic asthma comprising administering to a patient in need thereof a therapeutically effective amount of at least one of the compounds of the invention.

Furthermore, the disclosure preferably relates to a method of treating or preventing dermatological diseases, such as, but not limited to, psoriasis or atopic dermatitis (eczema) comprising administering to a patient in need thereof

a therapeutically effective amount of at least one of the compounds of the invention.

In addition, the disclosure preferably relates to a method of treating or preventing diseases of the eye, such as, but not limited to, uveitis, scleritis, keratitis, retinal vasculitis, age-related macula degeneration, diabetic nephropathy or chronic or allergic conjunctivitis comprising administering to a patient in need thereof a therapeutically effective amount of at least one of the compounds of the invention.

The disclosure relates as well to a method of treating or preventing rheumatoid arthritis comprising administering to a patient in need thereof a therapeutically effective amount of at least one of the compounds of the invention.

Additionally, the disclosure preferably relates to a method of treating or preventing diseases in the gastrointestinal region, such as, but not limited to, Crohn's disease or ulcerative colitis comprising administering to a patient in need thereof a therapeutically effective amount of at least one of the compounds of the invention.

In the above methods, the patient is preferably a mammal, more preferably a human. Furthermore, in the above methods, at least one of the compounds of the invention can be used. Preferably, one or two of the compounds of the invention are used, more preferably, one of the compounds of the invention is used.

In a particularly preferred embodiment of the invention, the above methods of treating or preventing one of the above mentioned diseases comprise administering to a patient in need thereof a therapeutically effective amount of a compound of the examples according to the present invention.

Pharmaceutical compositions

The invention furthermore relates to a pharmaceutical composition, which comprises at least one of the compounds of the invention together with at least one pharmaceutically acceptable auxiliary.

Preferably, the pharmaceutical composition comprises one or two of the compounds of the invention. More preferably, the pharmaceutical composition comprises one of the compounds of the invention.

In a particularly preferred embodiment of the invention, the pharmaceutical composition comprises a compound of the examples according to the present invention together with at least one pharmaceutically acceptable auxiliary.

The invention furthermore relates to a pharmaceutical composition according to the invention inhibiting the type 4 and type 5 phosphodiesterase, especially for (use in) the treatment or prophylaxis of diseases alleviated by inhibition of type 4 and type 5 phosphodiesterase, in particular for the treatment or prophylaxis of the diseases exemplified above.

The invention encompasses pharmaceutical compositions according to the invention, as defined above, in particular for (use in) the treatment or prophylaxis of one or more of the following diseases: interstitial lung disease such as pulmonary fibrosis, cystic fibrosis, bronchial asthma, chronic bronchitis, allergic bronchitis, allergic rhinitis, emphysema, chronic obstructive pulmonary disease (COPD) and COPD associated with pulmonary hypertension;
pulmonary hypertension, in particular thromboembolic pulmonary hypertension;
dermatological diseases, such as psoriasis and atopic dermatitis (eczema);
ocular diseases, such as uveitis, scleritis, keratitis, retinal vasculitis, age-related macula degeneration, diabetic nephropathy, and chronic and allergic conjunctivitis
rheumatoid arthritis; and
inflammations in the gastrointestinal region, such as Crohn's disease and ulcerative colitis.

Although the compounds of the invention may be administered orally, oral administration is not presently thought to be a preferred route of administration. This is because, without intending to be bound by this data, preliminary tests appear to indicate low systemic exposure after oral administration of the compounds of the invention in rats at a dose level of about 10 $\mu$mol/kg of the compound of the invention per kg bodyweight when formulated in aqueous suspension with polyethylenglycol 400 (1.3%) and hypromellose (4%).

The compounds of the invention respectively the pharmaceutical compositions comprising the compounds of the invention therefore preferably may be administered, for example, by external topical (i.e. through the skin/ transdermal or via the eye), parenteral (e.g. intravenous, subcutaneous, intraarterial, intraperitoneal, intraarticular, or intramuscular), inhaled or nasal administration. The compounds may also be administered via the rectal route, for example in form of a suppository or a foam.

Accordingly, the pharmaceutical composition can be suitable for (e.g. adapted for) external topical (i.e. through the skin / transdermal or via the eye), parenteral (e.g. intravenous, subcutaneous, intraarterial, intraperitoneal, intraarticular, or intramuscular), inhaled or nasal administration. The pharmaceutical composition is preferably suitable for inhaled administration. Inhaled administration involves topical administration to the lung e.g. by aerosol or dry powder composition.

Inhalable and intranasal pharmaceutical compositions

Formulations for inhalation include powder compositions, which will preferably contain lactose, and spray compositions which may be formulated, for example, as aqueous solutions or suspensions or as aerosols delivered from

pressurised packs, with the use of a suitable propellant, e. g. 1, 1, 1, 2-tetrafluorethane, 1, 1, 1, 2, 3, 3, 3-heptafluoropropane, carbon dioxide or other suitable gas.

A class of propellants, which is believed to have minimal ozone-depleting effects in comparison to conventional chlorofluorocarbons, comprise hydrofluorocarbons and a number of medicinal aerosol formulations using such propellant systems are disclosed in, for example, EP 0372777, WO91/04011, WO91/11173, WO91/11495, WO91/14422, WO93/11743, and EP-0553298. These applications are all concerned with the preparation of pressurised aerosols for the administration of medicaments and seek to overcome problems associated with the use of this new class of propellants, in particular the problems of stability associated with the pharmaceutical formulations prepared. The applications propose, for example, the addition of one or more of excipients such as polar cosolvents or wetting agents (e.g. alcohols such as ethanol), alkanes, dimethyl ether, surfactants (including fluorinated and non-fluorinated surfactants, carboxylic acids such as oleic acid, polyethoxylates etc.) or bulking agents such as a sugar (see for example WO02/30394) and vehicles such as cromoglicic acid and/or nedocromil, which are contained at concentrations, which are not therapeutically and prophylactically active (see WO00/07567). The aerosol dosage form can also take the form of a pump-atomizer.

For suspension aerosols, the compound of the invention should be micronised so as to permit inhalation of substantially all of the compound of the invention into the lungs upon administration of the aerosol formulation, thus the compound of the invention will have a mean particle size of less than 100 μm, desirably less than 20 μm, and preferably in the range of 1 to 10 μm (D50 value, e.g. as measured using laser diffraction).

Dry powder inhalable compositions: For pharmaceutical compositions suitable (e.g. adapted for) inhaled administration, the pharmaceutical composition may for example be a dry powder inhalable composition. The dry powder comprises finely divided compound of the invention optionally together with a finely divided pharmaceutically acceptable carrier, which is preferably present and may be one or more materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose, particularly in the form of the monohydrate.

The dry powder may be in capsules of gelatine or plastic, or in blisters, for use in a dry powder inhalation device, preferably in dosage units of the compound of the invention together with the carrier in amounts to bring the total weight of powder in each capsule to from 5mg to 50mg. Alternatively the dry powder may be contained in a reservoir of a multi-dose dry powder inhalation device. Capsules and cartridges of for example gelatin, or blisters of for example laminated aluminium foil, for use in an inhaler or insulator may be formulated containing a powder mix of the compounds of the invention and a suitable powder base such as lactose or starch, preferably lactose. In this aspect, the compound of the invention is suitably micronised so as to permit inhalation of substantially all of the compound of the invention into the lungs upon administration of the dry powder formulation, thus the compound of the invention will have a particle size of less than 100μm, desirably less than 20μm, and preferably in the range 1 to 10μm (D50 value, e.g. as measured using laser diffraction). The solid carrier, where present, generally has a maximum particle diameter of 300μm, preferably 200μm, and conveniently has a mean particle diameter of 40 to 100μm, preferably 50 to 75μm. The particle size of the compound of the invention and that of a solid carrier where present in dry powder compositions, can be reduced to the desired level by conventional methods, for example by grinding in an air-jet mill, ball mill or vibrator mill, microprecipitation, spray drying, lyophilisation or recrystallisation from supercritical media.

Where the inhalable form of the composition of the invention is the finely divided particulate form, the inhalation device may be, for example a dry powder inhalation device adapted to deliver dry powder from a capsule or blister containing a dosage unit of the dry powder or a multi-dose dry powder inhalation device. Such dry powder inhalation devices are known in the art. Examples which may be mentioned are Cyclohaler®, Diskhaler®, Rotadisk®, Turbohaler®, Novolizer®, Easyhaler®, Jethaler®, Clickhaler® or the dry powder inhalation devices disclosed in EP 0 505 321, EP 407028, EP 650410, EP 691865 or EP 725725 (Ultrahaler®).

Formulations for inhalation by nebulization may be formulated with an aqueous vehicle with the addition of agents such as acid or alkali, buffer salts, isotonicity adjusting agents or antimicrobials. They may be sterilised by filtration or heating in an autoclave. Suitable technologies for this type of administration are known in the art. As an example the Mystic® technology is to be mentioned (see for example US6397838, US6454193 and US6302331).

Preferred unit dosage formulations are those containing a pharmaceutical effective dose, as hereinbelow recited, or an appropriate fraction thereof, of the active ingredient. Thus, in the case of formulations designed for delivery by metered dose pressurised aerosols, one actuation of the aerosol may deliver half of the therapeutical effective amount such that two actuations are necessary to deliver the therapeutically effective dose.

In the dry powder inhalable composition, the compound of the invention can for example be present in about 0.1 % to about 70% (e.g. about 1% to about 50%, e.g. about 5% to about 40%, e.g. about 20 to about 30%) by weight of the composition.

In case of intranasal administration, for example, sprays and solutions to be applied in drop form are preferred formulations. Intranasal sprays or nasal drops may be formulated with aqueous or nonaqueous vehicles with or without the addition of agents such as thickening agents, buffer salts or acid or alkali to adjust the pH, isotonicity adjusting

agents, preservatives or anti-oxidants.

Pharmaceutical compositions suitable for external topical administration

"External topical" administration means topical administration to an external body part (i.e. excluding, for example, the lung or mouth, but including the lips or the eye). External topical adminstration (e.g. through the skin / transdermal) can for example be to those parts of the skin affected by or susceptible to a dermatological disease, such as for example, atopic dermatitis or psoriasis.

In case of external topical administration (i.e. through the skin / transdermal), suitable pharmaceutical formulations are, for example, ointments, creams (usually an oil-in-water or water-in-oil pharmaceutical composition, usually an emulsion), lotions, pastes, gels, powders, solutions, emulsions, suspensions, oils, sprays and patches (e.g., but not limited to, transdermal therapeutic systems).

In an external-topical pharmaceutical composition, e.g. an ointment or an oil-in-water or water-in-oil composition, the compound of the invention is suitably present in 0.05 to 10%, preferably 0.1 to 5%, more preferably 0.1 to 3%, still more preferably 0.5 to about 2.5 %, by weight of the composition (w/w).

External topical adminstration (e.g. via the eye) can for example be to the eye affected by or susceptible to an ocular disease, such as for example, uveitis, scleritis, keratitis, retinal vasculitis, age-related macula degeneration, diabetic nephropathy, and chronic and allergic conjunctivitis.

Examples, which may be mentioned in connection with pharmaceutical formulations for the eye are eyebaths or eye lotions, eye inserts, eye ointments, eye sprays, eye drops, preparations for intraocular application [e.g. intravitreale application, intraocular injection] and eyelid ointments.

Pharmaceutical compositions for oral or parenteral administration

For parenteral modes of administration such as, for example, intravenous, subcutaneous or intramuscular administration, preferably solutions (e.g., but not limited to, sterile solutions, isotonic solutions) are used. They are preferably administered by injection or infusion techniques.

A pharmaceutical composition suitable for parenteral (e.g. intravenous, subcutaneous or intramuscular) administration can comprise a solution or suspension of the compound of the invention in a sterile parenterally acceptable carrier (e.g. sterile water) or parenterally acceptable oil. Alternatively, the solution can be lyophilised. A lyophilised pharmaceutical composition suitable for parenteral administration may, in use, optionally be reconstituted with a suitable solvent, e.g. sterile water or a sterile parenterally acceptable aqueous solution, just prior to administration.

Oral administration is not preferred, as described above. However, a pharmaceutical composition for oral administration may be liquid or solid; for example, it may be a syrup, suspension or emulsion; as well it may be, for example, a tablet, coated tablet (dragee), pill, cachet, capsule (caplet), or in form of granules.

A liquid formulation may optionally consist of a suspension or solution of the compound of the invention in a pharmaceutically acceptable liquid carrier, for example an aqueous solvent such as water, ethanol or glycerine, or a non-aqueous solvent, such as polyethylene glycol or an oil. The formulation may contain in addition, a suspending agent, a preservative, flavouring and/or a colouring agent.

A pharmaceutical composition for oral administration being a tablet, though not preferred, may comprise one or more pharmaceutically acceptable auxiliaries (for example, carriers and/or excipients) suitable for preparing tablet formulations. The carrier may, for example, be or include lactose, cellulose or mannitol. The tablet may also, or instead, contain one or more pharmaceutically acceptable excipients, for example, a binding agent, a lubricant and/or a tablet disintegrant.

The pharmaceutical compositions according to the invention for oral or parenteral administration preferably contain the compound or compounds of the invention in a total amount of from 0.1 to 99.9%, more preferably 5 to 95%, in particular 20 to 80% by weight of the composition (w/w).

In general, as pharmaceutically acceptable auxiliaries, any auxiliaries known to be suitable for preparing a particular pharmaceutical composition can be used. Examples thereof include, but are not limited to, solvents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The pharmaceutical compositions/formulations can be manufactured in a manner known to a person skilled in the art, e.g. by dissolving, mixing, granulating, dragee-making, levigating, emulsifying, encapsulating, entrapping or lyophilizing processes.

Dosages

Generally, the pharmaceutical compositions according to the invention can be administered such that the dose of the compound of the invention is in the range customary for type 4 phosphodiesterase inhibitors.

The pharmaceutically acceptable compounds of the invention are preferably administered in a daily dose (for an adult patient) of, for example an oral or parenteral dose of 0.01 mg to 250 mg per day, preferably 0.05 mg to 100 mg per day, more preferably 0.05 mg to 10 mg per day, or a nasal or inhaled dose of 0.001 mg to 30 mg per day, preferably 0.01 mg to 10 mg per day, more preferably 0.1 mg to 4 mg per day, of the compound of the invention, calculated as the free compound (= the unsolvated, unhydrated, non-salt form of the compound).

In this respect, it is to be noted that the dose is dependent, for example, on the specific compound used, the species treated, age, body weight, general health, sex and diet of the subject treated, mode and time of administration, rate of excretion, severity of the disease to be treated and drug combination.

The pharmaceutical compositions of the invention can be administered in a single dose per day or in multiple subdoses, for example, 2 to 4 doses per day. A single dose unit of the pharmaceutical composition can contain, in case of inhalative administration e.g. from 0.001 mg to 10 mg, preferably 0.01 mg to 7.5 mg, more preferably 0.1mg to 4 mg of the compound of the invention. Administration of the pharmaceutical composition in a single dose per day is preferred.

Combinations

Depending on the particular disease to be treated or prevented, additionally therapeutic agents, which are normally administered to treat or prevent that disease, may optionally be co-administered with the compounds of the invention.

In a preferred embodiment, at least one of the compounds of the invention is co-administered with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides.

In this respect, the "therapeutic agent" includes the corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides in form of the free compounds, the pharmaceutically acceptable salts thereof, the pharmaceutically acceptable derivatives thereof (e.g., but not limited to, ester derivatives, N-oxides etc.), the solvates (hydrates) thereof and the stereoisomers of the compounds, salts, derivatives and solvates.

Co-administration of at least one of the compounds of the invention with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides can take place in form of a fixed combination, a non-fixed combination or a kit of parts.

A "fixed combination" is defined as a combination wherein the compound of the invention and the therapeutic agent intended for co-administration are present in one dosing unit or in a single entity. One example of a fixed combination is a pharmaceutical composition wherein the compound of the invention and the therapeutic agent are present in admixture for simultaneous administration. Another example of a fixed combination is a pharmaceutical composition wherein the compound of the invention and the therapeutic compound are present in one dosing unit without being in admixture.

A "non-fixed combination" or "kit of parts" is defined as a combination wherein the compound of the invention and the therapeutic agent are present in more than one dosing unit. In a non-fixed combination or a kit of parts the compound of the invention and the therapeutic agent are provided as separate formulations. They might be packaged and presented together as separate components of a combination pack for simultaneous, sequential or separate use in combination therapy. Simultaneous or sequential administration of the compound of the invention and the therapeutic agent are preferred. In case of sequential or separate administration of the compound of the invention and the therapeutic agent, the compound of the invention can be administered before or after administration of the therapeutic agent.

Sequential administration encompasses a short time period between the administration of the compound of the invention and the therapeutic agent or vice versa (for example, the time that is needed to swallow one tablet after the other).

Separate administration encompasses longer time periods between the administration of the compound of the invention and the therapeutic agent. In a preferred embodiment of the invention, the compound of the invention is administered while the therapeutic agent (or vice versa) still has an therapeutic effect on the patient being treated.

In a particularly preferred embodiment of the invention the co-administration of at least one of the compounds of the invention with at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylyl-cyclase activators/stimulators, tetrahydrobiopterin and tetrahydrobiopterin derivatives, anticoagulants, diuretics, pirfenidone and digitalis glycosides leads to a therapeutic effect that is greater than the sum of the therapeutic effects that will be achieved in case the compound of the invention respectively the additional therapeutic agent are given alone.

The type of formulation of the compound of the invention and the therapeutic agent of a non-fixed combination or a kit of parts can be identical, i.e. both, the compound of the invention and the therapeutic agent are formulated, for example, as powder, solution or suspension suitable for inhalative administration, or can be different, i.e. suited for different administration forms, such as e.g. the compound of the invention is formulated as powder, solution or suspension suitable for inhalative administration and the therapeutic agent is formulated as tablet or capsule for oral administration.

Accordingly, the invention additionally relates to a pharmaceutical composition presented either as a fixed combination, a non-fixed combination or kit of parts comprising at least one of the compounds of the invention, at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, angiotensin II-receptor antagonists, lung surfactants, antibiotics, guanylylcyclase activators/stimulators, tetrahydrobiopterin and tetrahydrobiopterin derivatives, anticoagulants, diuretics, pirfenidone and digitalis glycosides, and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a $\beta_2$-adrenoreceptor agonist and at least one pharmaceutically acceptable auxiliary. In a particularly preferred embodiment, the above-mentioned fixed combination, non-fixed combination or kit of parts comprise:

a compound of the invention and salbutamol,
a compound of the invention and milveterol,
a compound of the invention and indacaterol,
a compound of the invention and carmoterol,
a compound of the invention and salmeterol,
a compound of the invention and formoterol,
a compound of the invention and vilanterol, or
a compound of the invention and olodaterol,
and at least one pharmaceutically acceptable auxiliary.

In a preferred embodiment, the pharmaceutically acceptable salt of salbutamol is salbutamol sulfate. In a preferred embodiment, the pharmaceutically acceptable salt of milveterol is milveterol hydrochloride. In a preferred embodiment, the pharmaceutically acceptable salt of carmoterol is carmoterol hydrochloride. In a preferred embodiment, the pharmaceutically acceptable salt of salmeterol is salmeterol xinafoate. In another preferred embodiment, the pharmaceutically acceptable salt of formoterol is formoterol hemifumarate monohydrate. In another preferred embodiment, the stereoisomer of formoterol is R,R-formoterol. In another preferred embodiment, the pharmaceutically acceptable salt of R,R-formoterol is R,R-formoterol L-tartrate. In a preferred embodiment, the pharmaceutically acceptable salt of vilanterol is vilanterol trifenatate. In another preferred embodiment, the pharmaceutically acceptable salt of vilanterol is vilanterol $\alpha$-phenyl cinnamate. In a preferred embodiment, the pharmaceutically acceptable salt of olodaterol is olodaterol hydrochloride.

Preferably the $\beta 2$-adrenoreceptor agonist is a long-acting $\beta 2$-adrenoreceptor agonist; particularly preferred in this respect are those $\beta 2$-adrenoreceptor agonists having a therapeutic effect over a 12-24 hours period. Furthermore, the $\beta 2$-adrenoreceptor agonist is preferably for inhaled administration, for once daily administration and for simultaneous inhaled administration.

Preferably, the combination comprising a compound of the invention and a β2-adrenoreceptor agonist is for the treatment or prophylaxis of bronchial asthma and COPD.

In a preferred embodiment, the above-mentioned fixed combination, non-fixed combination or kit of parts comprises a compound of the invention (in particular the compound of the invention is one of the examples of the invention), a corticosteroid and at least one pharmaceutically acceptable auxiliary. In a particularly preferred embodiment, the above-mentioned fixed combination, non-fixed combination or kit of parts comprise:

a compound of the invention and budesonide,
a compound of the invention and fluticasone,
a compound of the invention and beclometasone,
a compound of the invention and mometasone,
a compound of the invention and triamcinolone acetonide, or
a compound of the invention and ciclesonide,
and at least one pharmaceutically acceptable auxiliary.

In a preferred embodiment, the pharmaceutically acceptable derivative of fluticasone is fluticasone-17-propionate. In another preferred embodiment, the pharmaceutically acceptable derivative of fluticasone is fluticasone-17-furoate. In another preferred embodiment, the pharmaceutically acceptable derivative of beclometasone is beclometasone 17, 21-dipropionate ester. In a preferred embodiment, the pharmaceutically acceptable derivative of mometasone is mometasone furoate.

The combination comprising a compound of the invention and a corticosteroid preferably is for the treatment and prophylaxis of bronchial asthma, COPD, allergic rhinitis or a dermatological disease, such as for example atopic dermatitis. Preferably the corticosteroid is used for external topical, intranasal or inhaled administration; in severe cases, the corticosteroid may also be used orally.

In a preferred embodiment, the above-mentioned fixed combination, non-fixed combination or kit of parts comprises a compound of the invention (in particular the compound of the invention is one of the examples of the invention), an anticholinergic and at least one pharmaceutically acceptable auxiliary. In a particularly preferred embodiment, the above-mentioned fixed combination, non-fixed combination or kit of parts comprise:

a compound of the invention and glycopyrronium bromide,
a compound of the invention and aclidinium bromide,
a compound of the invention and tiotropium bromide,
a compound of the invention and ipratropium bromide, or
a compound of the invention and darotropium bromide,
and at least one pharmaceutically acceptable auxiliary.

In a preferred embodiment, the stereoisomer of glycopyrronium bromide is (R,R)-glycopyrronium bromide. In a preferred embodiment, tiotropium bromide is used in form of its monohydrate.

Preferably, the anticholinergic is for inhaled administration. The combination comprising a compound of the invention and an anticholinergic is preferably for the treatment or prophylaxis of COPD.

In a preferred embodiment, the above-mentioned fixed combination, non-fixed combination or kit of parts comprises a compound of the invention (in particular the compound of the invention is one of the examples of the invention), a H1 receptor antagonist and at least one pharmaceutically acceptable auxiliary. In a particularly preferred embodiment, the above-mentioned fixed combination, non-fixed combination or kit of parts comprise:

a compound of the invention and azelastine,
a compound of the invention and olopatadine,
a compound of the invention and loratadine,
a compound of the invention and desloratadine, or
a compound of the invention and cetirizine,
and at least one pharmaceutically acceptable auxiliary.

In a preferred embodiment, the pharmaceutically acceptable salt of azelastine is is azelastine hydrochloride. In a preferred embodiment, the pharmaceutically acceptable salt of olapatadine is olapatadine hydrochloride. In a preferred embodiment, the pharmaceutically acceptable salt of cetirizine is cetirizine dihydrochloride. In a preferred embodiment, the stereoisomer of cetirizine is levocetirizine. In another preferred embodiment, the pharmaceutically acceptable salt of levocetirizine is levocetirizine dihydrochloride.

The combination comprising a compound of the invention and a H1 receptor agonist is preferably for the

treatment or prophylaxis of allergic rhinitis.

In a preferred embodiment, the above-mentioned fixed combination, non-fixed combination or kit of parts comprises a compound of the invention (in particular the compound of the invention is one of the examples of the invention), a leukotriene receptor antagonist and at least one pharmaceutically acceptable auxiliary. In a particularly preferred embodiment, the above-mentioned fixed combination, non-fixed combination or kit of parts comprise:

a compound of the invention and montelukast,
a compound of the invention and pranlukast, or
a compound of the invention and zafirlukast,
and at least one pharmaceutically acceptable auxiliary.

In a preferred embodiment, the pharmaceutically acceptable salt of montelukast is montelukast sodium. In another preferred embodiment, pranlukast is used in form of its monohydrate.

The combination comprising a compound of the invention and a leukotriene receptor antagonist is preferably for the treatment or prophylaxis of bronchial asthma.

In a preferred embodiment, the above-mentioned fixed combination, non-fixed combination or kit of parts comprises a compound of the invention (in particular the compound of the invention is one of the examples of the invention), a 5-lipoxygenase inhibitor and at least one pharmaceutically acceptable auxiliary. In a particularly preferred embodiment, the above-mentioned fixed combination, non-fixed combination or kit of parts comprise:

a compound of the invention and zileuton,
and at least one pharmaceutically acceptable auxiliary.

The combination comprising a compound of the invention and a 5-lipoxygenase inhibitor is preferably for the treatment or prophylaxis of bronchial asthma.

In a preferred embodiment, the above-mentioned fixed combination, non-fixed combination or kit of parts comprises a compound of the invention (in particular the compound of the invention is one of the examples of the invention), an endothelin receptor antagonist and at least one pharmaceutically acceptable auxiliary. In a particularly preferred embodiment, the above-mentioned fixed combination, non-fixed combination or kit of parts comprise:

a compound of the invention and bosentan,
a compound of the invention and ambrisentan,
a compound of the invention and atrasentan,
a compound of the invention and darusentan,
a compound of the invention and clazosentan, or
a compound of the invention and avosentan,
and at least one pharmaceutically acceptable auxiliary.

In another preferred embodiment, bosentan is used in form of its monohydrate. In another preferred embodiment the pharmaceutically acceptable salt of clazosentan is the disodium salt of clazosentan. In another preferred embodiment the pharmaceutically acceptable salts of atrasentan are atrasentan hydrochloride or the sodium salt of atrasentan. In another preferred embodiment the R-enantiomer of atrasentan is used. In another preferred embodiment the S-enantiomer of darusentan is used.

The combination comprising a compound of the invention and an endothelin antagonist is preferably for the treatment or prophylaxis of pulmonary hypertension and COPD.

In a preferred embodiment, the above-mentioned fixed combination, non-fixed combination or kit of parts comprises a compound of the invention invention (in particular the compound of the invention is one of the examples of the invention), a prostacyclin and at least one pharmaceutically acceptable auxiliary. In a particularly alternative embodiment, the above-mentioned fixed combination, non-fixed combination or kit of parts comprise:

a compound of the invention and iloprost,
a compound of the invention and epoprostenol, or
a compound of the invention and triprostinil,
and at least one pharmaceutically acceptable auxiliary.

The combination comprising a compound of the invention and a prostacyclin is preferably for the treatment or prophylaxis of pulmonary hypertension.

In a preferred embodiment, the above-mentioned fixed combination, non-fixed combination or kit of parts comprises a compound of the invention invention (in particular the compound of the invention is one of the examples of the invention), a calcium channel blocker and at least one pharmaceutically acceptable auxiliary. In a particularly alternative embodiment, the above-mentioned fixed combination, non-fixed combination or kit of parts comprise:

a compound of the invention and amlodipine,
a compound of the invention and nifedipine,
a compound of the invention and diltiazem,
a compound of the invention and verapamil, or
a compound of the invention and felodipine,
and at least one pharmaceutically acceptable auxiliary.

In a preferred embodiment, the above-mentioned fixed combination, non-fixed combination or kit of parts comprises a compound of the invention invention (in particular the compound of the invention is one of the examples of the invention), a beta-blocker and at least one pharmaceutically acceptable auxiliary. In a particularly preferred embodiment, the above-mentioned fixed combination, non-fixed combination or kit of parts comprise:

a compound of the invention and bisoprolol,
a compound of the invention and nebivolol,
a compound of the invention and metoprolol,
a compound of the invention and carvedilol,
a compound of the invention and atenolol, or
a compound of the invention and nadolol,
and at least one pharmaceutically acceptable auxiliary.

In a preferred embodiment, the above-mentioned fixed combination, non-fixed combination or kit of parts comprises a compound of the invention invention (in particular the compound of the invention is one of the examples of the invention), a type 4 phosphodiesterase inhibitor and at least one pharmaceutically acceptable auxiliary. In a particularly preferred embodiment, the above-mentioned fixed combination, non-fixed combination or kit of parts comprise:

a compound of the invention and roflumilast,
a compound of the invention and roflumilast N-oxide,
a compound of the invention and apremilast,
a compound of the invention and oglemilast,
a compound of the invention and revamilast, or
a compound of the invention and 6-({3- [(dimethylamino) carbonyl]phenyl)sulfonyl)-8-methyl-4-{ [3-methyloxy)phenyl]amino}-3-quinolinecarboxamide (GSK256066)
and at least one pharmaceutically acceptable auxiliary.

The combination comprising a compound of the invention and an additional PDE4 inhibitor is preferably for the treatment or prophylaxis of pulmonary hypertension and COPD.

In a preferred embodiment, the above-mentioned fixed combination, non-fixed combination or kit of parts comprises a compound of the invention (in particular the compound of the invention is one of the examples of the invention), a type 5 phosphodiesterase inhibitor and at least one pharmaceutically acceptable auxiliary. In a particularly preferred embodiment, the above-mentioned fixed combination, non-fixed combination or kit of parts comprise:

a compound of the invention and sildenafil,
a compound of the invention and vardenafil,
a compound of the invention and tadalafil,
a compound of the invention and udenafil, or
a compound of the invention and avanafil,
and at least one pharmaceutically acceptable auxiliary.

In another preferred embodiment, the pharmaceutically acceptable salts of sildenafil are sildenafil hemi-citrate, sildenafil citrate and sildenafil mesilate; particularly preferred is the citrate salt of sildenafil. In another preferred embodiment, the pharmaceutically acceptable salts of vardenafil are vardenafil hydrochloride or vardenafil dihyrochloride. In another preferred embodiment, the pharmaceutically acceptable salt of avanafil is avanafil besilate.

The combination comprising a compound of the invention and an additional PDE5 inhibitor is preferably for the treatment or prophylaxis of pulmonary hypertension and COPD.

In a preferred embodiment, the above-mentioned fixed combination, non-fixed combination or kit of parts comprises a compound of the invention (in particular the compound of the invention is one of the examples of the invention), a guanyl-cyclase activator/stimulator and at least one pharmaceutically acceptable auxiliary. In a particularly preferred embodiment, the above-mentioned fixed combination, non-fixed combination or kit of parts comprise:

a compound of the present subject matter and BAY63-2521 (Riociguat), or
a compound of the present subject matter and Ataciguat,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), tetrahydrobiopterin or a tetrahydrobiopterin derivative and at least one pharmaceutically acceptable auxiliary. In a particularly preferred embodiment, the above-mentioned fixed combination, non-fixed combination or kit of parts comprise:

a compound of the invention and (6R)-L-erythro-5,6,7,8-tetrahydrobiopterin,
a compound of the invention and (6R,S)-5,6,7,8-tetrahydrobiopterin,
a compound of the invention and 1',2'-diacetyl-5,6,7,8-tetrahydrobiopterin,
a compound of the invention and sepiapterin,
a compound of the invention and 6-methyl-5,6,7,8-tetrahydropterin,
a compound of the invention and 6-hydroxymethyl-5,6,7,8-tetrahydropterin, or
a compound of the invention and 6-phenyl-5,6,7,8-tetrahydropterin,
and at least one pharmaceutically acceptable auxiliary.

In a preferred embodiment, the pharmaceutically acceptable derivative of (6R)-L-erythro-5,6,7,8-tetrahydrobiopterin is (6R)-L-erythro-5,6,7,8-tetrahydrobiopterin dihydrochloride.

In a preferred embodiment, the above-mentioned fixed combination, non-fixed combination or kit of parts comprises a compound of the invention (in particular the compound of the invention is one of the examples of the invention), a HMG-CoA reductase inhibitor and at least one pharmaceutically acceptable auxiliary. In a particularly preferred embodiment, the above-mentioned fixed combination, non-fixed combination or kit of parts comprise:

a compound of the invention and lovastatin,
a compound of the invention and pravastatin,
a compound of the invention and simvastatin,
a compound of the invention and atorvastatin,
a compound of the invention and fluvastatin,
a compound of the invention and rosuvastatin,
a compound of the invention and pitavastatin,
a compound of the invention and bervastatin,
a compound of the invention and dalvastatin, or
a compound of the invention and glenvastatin,
and at least one pharmaceutically acceptable auxiliary.

In a preferred embodiment the pharmaceutically acceptable salts of pravastatin are the potassium, lithium, sodium and hemi-calcium salt of pravastatin. A particularly preferred pharmaceutically acceptable salt of pravastatin is the sodium salt of pravastatin. In a preferred embodiment the pharmaceutically acceptable salt of simvastatin is the sodium salt of simvastatin. In a preferred embodiment the pharmaceutically acceptable salts of atorvastatin are the potassium, sodium and the hemi-calcium salt of atorvastatin. A particularly preferred pharmaceutically acceptable salt of atorvastatin is the hemi-calcium salt of atorvastatin. As an example for a hydrate of atorvastatin may be mentioned the trihydrate and the sesqui-hydrate of the hemi-calcium salt of atorvastatin. In a preferred embodiment of the pharmaceutically acceptable salt of fluvastatin is the sodium salt of fluvastatin. In a preferred embodiment the pharmaceutically acceptable salts of rosuvastatin are the potassium, lithium, sodium, hemimagnesium and the hemi-calcium salt of rosuvastatin. A particularly preferred pharmaceutically acceptable salt of rosuvastatin is the hemi-calcium salt of rosuvastatin. Another particularly preferred pharmaceutically acceptable salt of rosuvastatin is the sodium salt of rosuvastatin. In a preferred embodiment the pharmaceutically acceptable salts of pitavastatin are the potassium, sodium and the hemi-calcium salt of pitavastatin. A particularly preferred pharmaceutically acceptable salt of pitavastatin is the hemi-calcium salt of pitavastatin.

The combination comprising a compound of the invention and a HMG-CoA reductase inhibitor is preferably for the treatment or prophylaxis of COPD.

In a preferred embodiment, the above-mentioned fixed combination, non-fixed combination or kit of parts comprises a compound of the invention (in particular the compound of the invention is one of the examples of the invention), a PPARγ agonist and at least one pharmaceutically acceptable auxiliary. In a particularly preferred embodiment, the above-mentioned fixed combination, non-fixed combination or kit of parts comprise:

a compound of the invention and pioglitazone,
a compound of the invention and rosiglitazone,
a compound of the invention and troglitazone,
a compound of the invention and rivoglitazone, or
a compound of the invention and ciglitazone,
and at least one pharmaceutically acceptable auxiliary.

The combination comprising a compound of the invention and an additional PPARγ agonist is preferably for the treatment or prophylaxis of COPD and comorbidities.

In a preferred embodiment, the above-mentioned fixed combination, non-fixed combination or kit of parts comprises a compound of the invention (in particular the compound of the invention is one of the examples of the invention), an ACE inhibitor and at least one pharmaceutically acceptable auxiliary. In a particularly preferred embodiment, the above-mentioned fixed combination, non-fixed combination or kit of parts comprise:

a compound of the invention and captopril,
a compound of the invention and enalapril,
a compound of the invention and fosinopril,
a compound of the invention and lisinopril,
a compound of the invention and moexipril,
a compound of the invention and benazepril,
a compound of the invention and perindopril
a compound of the invention and ramipril
a compound of the invention and trandolapril, or
a compound of the invention and quinapril,
and at least one pharmaceutically acceptable auxiliary.

The combination comprising a compound of the invention and an additional ACE inhibitor is preferably for the treatment or prophylaxis of COPD and comorbidities.

In a preferred embodiment, the above-mentioned fixed combination, non-fixed combination or kit of parts comprises a compound of the invention (in particular the compound of the invention is one of the examples of the invention), a angiotensin II receptor antagonist and at least one pharmaceutically acceptable auxiliary. In a particularly preferred embodiment, the above-mentioned fixed combination, non-fixed combination or kit of parts comprise:

a compound of the invention and losartan,
a compound of the invention and azilsartan,
a compound of the invention and valsartan,
a compound of the invention and olemsartan,
a compound of the invention and telmisartan, or
a compound of the invention and irbesartan,
and at least one pharmaceutically acceptable auxiliary.

The combination comprising a compound of the invention and an additional angiotensin II receptor antagonist is preferably for the treatment or prophylaxis of COPD and comorbidities.

In a preferred embodiment, the above-mentioned fixed combination, non-fixed combination or kit of parts comprise a compound of the invention (in particular the compound of the invention is one of the examples of the invention), a lung surfactant and at least one pharmaceutically acceptable auxiliary. In a particularly preferred embodiment, the above-mentioned fixed combination, non-fixed combination or kit of parts comprise:

a compound of the invention and lusupultide,
a compound of the invention and poracant alfa,
a compound of the invention and sinapultide,
a compound of the invention and beracant,
a compound of the invention and bovacant,

a compound of the invention and colfosceril palmitate,
a compound of the invention and surfactant-TA, or
a compound of the invention and calfacant,
and at least one pharmaceutically acceptable auxiliary.

The combination comprising a compound of the invention and a lung surfactant is preferably for the treatment or prophylaxis of bronchial asthma or COPD.

In a preferred embodiment, the above-mentioned fixed combination, non-fixed combination or kit of parts comprises a compound of the invention (in particular the compound of the invention is one of the examples of the invention), an antibiotic and at least one pharmaceutically acceptable auxiliary. In a particularly preferred embodiment, the above-mentioned fixed combination, non-fixed combination or kit of parts comprise:

a compound of the invention and amoxicillin,
a compound of the invention and ampicillin,
a compound of the invention and levofloxacin,
a compound of the invention and clarithromycin,
a compound of the invention and ciprofloxacin,
a compound of the invention and telithromycin, or
a compound of the invention and azithromycin,
and at least one pharmaceutically acceptable auxiliary.

In a preferred embodiment, amoxicillin is used in form of its trihydrate. In another preferred embodiment, ampicillin is used in form of its trihydrate. In another preferred embodiment, the pharmaceutically acceptable salt of ampicillin is ampicillin natrium. In another preferred embodiment levofloxacin is used in form of its hemi hydrate. In another preferred embodiment, the pharmaceutically acceptable salt of ciprofloxacin is ciprofloxacin hydrochloride monohydrate. In another preferred embodiment, azithromycin is used in form of its monohydrate.

The combination comprising a compound of the invention and an antibiotic is preferably for the treatment or prophylaxis of exacerbations associated with bronchial asthma and COPD.

In a preferred embodiment, the above-mentioned fixed combination, non-fixed combination or kit of parts comprises a compound of the invention (in particular the compound of the invention is one of the examples of the invention), an anticoagulant and at least one pharmaceutically acceptable auxiliary. In a particularly preferred embodiment, the above-mentioned fixed combination, non-fixed combination or kit of parts comprise:

a compound of the invention and clopidogrel,
a compound of the invention and enoxaparin,
a compound of the invention and cilostazol,
a compound of the invention and nadroparin,
a compound of the invention and warfarin, or
a compound of the invention and abciximab,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a diuretic and at least one pharmaceutically acceptable auxiliary. In a particularly preferred embodiment, the above-mentioned fixed combination, non-fixed combination or kit of parts comprise:

a compound of the invention and furosemide,
a compound of the invention and bumetanide, or
a compound of the invention and torsemide,
and at least one pharmaceutically acceptable auxiliary.

The combination comprising a compound of the invention and a diuretic preferably is for the treatment and prophylaxis of cystic fibrosis.

In a preferred embodiment, the above-mentioned fixed combination, non-fixed combination or kit of parts comprises a compound of the invention (in particular the compound of the invention is one of the examples of the invention), pirfenidone and at least one pharmaceutically acceptable auxiliary. In a particularly preferred embodiment, the above-mentioned fixed combination, non-fixed combination or kit of parts comprise:

a compound of the invention and pirfenidone,
and at least one pharmaceutically acceptable auxiliary.

The combination comprising a compound of the invention and pirfenidone preferably is for the treatment and prophylaxis of lung fibrosis.

In a preferred embodiment, the above-mentioned fixed combination, non-fixed combination or kit of parts comprises a compound of the invention (in particular the compound of the invention is one of the examples of the invention), a digitalis glycoside and at least one pharmaceutically acceptable auxiliary. In a particularly preferred embodiment, the above-mentioned fixed combination, non-fixed combination or kit of parts comprise:

a compound of the invention and digoxin, or
a compound of the invention and digitoxin,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a corticosteroid, a $\beta_2$-adrenoreceptor agonist and at least one pharmaceutically acceptable auxiliary. In a particularly preferred embodiment, the above-mentioned fixed combination, non-fixed combination or kit of parts comprise:

a compound of the invention, budesonide and salbutamol,
a compound of the invention, budesonide and milveterol,
a compound of the invention, budesonide and indacaterol,
a compound of the invention, budesonide and carmoterol,
a compound of the invention, budesonide and salmeterol,
a compound of the invention, budesonide and formoterol,
a compound of the invention, budesonide and vilanterol,
a compound of the invention, budesonide and olodaterol,
a compound of the invention, fluticasone and salbutamol,
a compound of the invention, fluticasone and milveterol,
a compound of the invention, fluticasone and indacaterol,
a compound of the invention, fluticasone and carmoterol,
a compound of the invention, fluticasone and salmeterol,
a compound of the invention, fluticasone and formoterol,
a compound of the invention, fluticasone and vilanterol,
a compound of the invention, fluticasone and olodaterol,
a compound of the invention, beclometasone and salbutamol,
a compound of the invention, beclometasone and milveterol,
a compound of the invention, beclometasone and indacaterol,
a compound of the invention, beclometasone and carmoterol,
a compound of the invention, beclometasone and salmeterol,
a compound of the invention, beclometasone and formoterol,
a compound of the invention, beclometasone and vilanterol,
a compound of the invention, beclometasone and olodaterol,
a compound of the invention, mometasone and salbutamol,
a compound of the invention, mometasone and milveterol,
a compound of the invention, mometasone and indacaterol,
a compound of the invention, mometasone and carmoterol,
a compound of the invention, mometasone and salmeterol,
a compound of the invention, mometasone and formoterol,
a compound of the invention, mometasone and vilanterol,
a compound of the invention, mometasone and olodaterol,
a compound of the invention, triamcinolone acetonide and salbutamol,
a compound of the invention, triamcinolone acetonide and milveterol,
a compound of the invention, triamcinolone acetonide and indacaterol,
a compound of the invention, triamcinolone acetonide and carmoterol,
a compound of the invention, triamcinolone acetonide and salmeterol,
a compound of the invention, triamcinolone acetonide and formoterol,
a compound of the invention, triamcinolone and vilanterol,

a compound of the invention, triamcinolone and olodaterol,
a compound of the invention, ciclesonide and salbutamol,
a compound of the invention, ciclesonide and milveterol,
a compound of the invention, ciclesonide and indacaterol,
a compound of the invention, ciclesonide and carmoterol,
a compound of the invention, ciclesonide and salmeterol,
a compound of the invention, ciclesonide and formoterol,
a compound of the invention, ciclesonide and vilanterol, or
a compound of the invention, ciclesonide and olodaterol,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a $\beta_2$-adrenoreceptor agonist, an anticholinergic and at least one pharmaceutically acceptable auxiliary. In a particularly preferred embodiment, the above-mentioned fixed combination, non-fixed combination or kit of parts comprise:

a compound of the invention, salbutamol and glycopyrronium bromide,
a compound of the invention, salbutamol and aclidinium bromide,
a compound of the invention, salbutamol and tiotropium bromide,
a compound of the invention, salbutamol and ipratropium bromide,
a compound of the invention, salbutamol and darotropium bromide,
a compound of the invention, milveterol and glycopyrronium bromide,
a compound of the invention, milveterol and aclidinium bromide,
a compound of the invention, milveterol and tiotropium bromide,
a compound of the invention, milveterol and ipratropium bromide,
a compound of the invention, milveterol and darotropium bromide,
a compound of the invention, salmeterol and glycopyrronium bromide,
a compound of the invention, salmeterol and aclidinium bromide,
a compound of the invention, salmeterol and tiotropium bromide,
a compound of the invention, salmeterol and ipratropium bromide,
a compound of the invention, salmeterol and darotropium bromide,
a compound of the invention, formoterol and glycopyrronium bromide,
a compound of the invention, formoterol and aclidinium bromide,
a compound of the invention, formoterol and tiotropium bromide,
a compound of the invention, formoterol and ipratropium bromide,
a compound of the invention, formoterol and darotropium bromide,
a compound of the invention, indacaterol and glycopyrronium bromide,
a compound of the invention, indacaterol and aclidinium bromide,
a compound of the invention, indacaterol and tiotropium bromide,
a compound of the invention, indacaterol and ipratropium bromide,
a compound of the invention, indacaterol and darotropium bromide,
a compound of the invention, carmoterol and glycopyrronium bromide,
a compound of the invention, carmoterol and aclidinium bromide,
a compound of the invention, carmoterol and tiotropium bromide,
a compound of the invention, carmoterol and ipratropium bromide,
a compound of the invention, carmoterol and darotropium bromide,
a compound of the invention, vilanterol and glycopyrronium bromide,
a compound of the invention, vilanterol and aclidinium bromide,
a compound of the invention, vilanterol and tiotropium bromide,
a compound of the invention, vilanterol and ipratropium bromide,
a compound of the invention, vilanterol and darotropium bromide,
a compound of the invention, olodaterol and glycopyrronium bromide,
a compound of the invention, olodaterol and aclidinium bromide,
a compound of the invention, olodaterol and tiotropium bromide,
a compound of the invention, olodaterol and ipratropium bromide, or
a compound of the invention, olodaterol and darotropium bromide,
and at least one pharmaceutically acceptable auxiliary.

In a preferred embodiment, the above-mentioned fixed combination, non-fixed combination or kit of parts comprises a compound of the invention (in particular the compound of the invention is one of the examples of the invention), a corticosteroid, an anticholinergic and at least one pharmaceutically acceptable auxiliary. In a particularly preferred embodiment, the above-mentioned fixed combination, non-fixed combination or kit of parts comprise:

a compound of the invention, budesonide and glycopyrronium bromide,
a compound of the invention, budesonide and aclidinium bromide,
a compound of the invention, budesonide and tiotropium bromide,
a compound of the invention, budesonide and ipratropium bromide,
a compound of the invention, budesonide and darotropium bromide,
a compound of the invention, fluticasone and glycopyrronium bromide,
a compound of the invention, fluticasone and aclidinium bromide,
a compound of the invention, fluticasone and tiotropium bromide,
a compound of the invention, fluticasone and ipratropium bromide,
a compound of the invention, fluticasone and darotropium bromide,
a compound of the invention, beclometasone and glycopyrronium bromide,
a compound of the invention, beclometasone and aclidinium bromide,
a compound of the invention, beclometasone and tiotropium bromide,
a compound of the invention, beclometasone and ipratropium bromide,
a compound of the invention, beclometasone and darotropium bromide,
a compound of the invention, mometasone and glycopyrronium bromide,
a compound of the invention, mometasone and aclidinium bromide,
a compound of the invention, mometasone and tiotropium bromide,
a compound of the invention, mometasone and ipratropium bromide,
a compound of the invention, mometasone and darotropium bromide,
a compound of the invention, triamcinolone acetonide and glycopyrronium bromide,
a compound of the invention, triamcinolone acetonide and aclidinium bromide,
a compound of the invention, triamcinolone acetonide and tiotropium bromide,
a compound of the invention, triamcinolone acetonide and ipratropium bromide,
a compound of the invention, triamcinolone acetonide and darotropium bromide,
a compound of the invention, ciclesonide and glycopyrronium bromide,
a compound of the invention, ciclesonide and aclidinium bromide,
a compound of the invention, ciclesonide and tiotropium bromide,
a compound of the invention, ciclesonide and ipratropium bromide, or
a compound of the invention, ciclesonide and darotropium bromide,
and at least one pharmaceutically acceptable auxiliary.

The above-mentioned triple combinations may preferably be used in the treatment or prophylaxis of bronchial asthma or COPD.

Exemplary combinations, in particular for external topical administration (for example versus atopic dermatitis or psoriasis), may include a compound of the invention and an immunosuppressant, for example a calcineurin inhibitor, such as pimecrolimus or tacrolimus.

Therefore, in another preferred embodiment, the above-mentioned fixed combination, non-fixed combination or kit of parts comprise a compound of the invention (in particular the compound of the invention is one of the examples of the invention or a pharmaceutically acceptable salt thereof), an immunosuppressant and at least one pharmaceutically acceptable auxiliary. In a particularly preferred embodiment, the above mentioned fixed combination, non-fixed combination or kit of parts comprises:

a compound of the invention and pimecrolimus,
a compound of the invention and tacrolimus,
a compound of the invention and methotrexate,
a compound of the invention and ascomycin, or
a compound of the invention and cyclosporin A,
and at least one pharmaceutically acceptable auxiliary.

The externally topically administrable immunosuppressant can be administered or administrable in a external-topical composition separately from the compound of the invention (non-fixed combination or kit of parts) or it can be contained with the compound of the invention in a combined externally-topically administrable composition (fixed combination). In a preferred embodiment the externally topically administrable composition is a cream containing pimecrolimus at ca. 1 % w/w concentration. In another preferred embodiment the externally topically administrable composition is an ointment containing tacrolimus at from about 0.03% to about 0.1 % w/w concentration).

Other combinations for external topical adminstration, in particular for the treatment or prophylaxis of atopic dermatitis and psoriasis, may include a compound of the invention and a corticosteroid. Beside the corticosteroid combinations mentioned above also the following corticosteroid combinations may be useful.

In another preferred embodiment, the above-mentioned fixed combination, non-fixed combination or kit of parts comprise a compound of the invention (in particular the compound of the invention is one of the examples of the invention or a pharmaceutically acceptable salt thereof), a corticosteroid and at least one pharmaceutically acceptable auxiliary. In a particularly preferred embodiment, the above mentioned fixed combination, non-fixed combination or kit of parts comprises:

a compound of the invention and prednisolone,
a compound of the invention and dexamethasone,
a compound of the invention and betamethasone, or
a compound of the invention and hydrocortisone,
and at least one pharmaceutically acceptable auxiliary.

In another preferred embodiment, the above-mentioned corticosteroids are used in form of an ester, such as, for example, prednisolone valerate acetate, hydrocortisone butyrate, hydrocortisone acetate, dexamethasone valerate, dexamethasone propionate, dexamethasone dipropionate, betamethasone butyrate propionate or prednisolone valerate acetate.

Further combinations for external topical combination, in particular for the treatment of psoriasis, may include a compound of the invention and a vitamin D analogue.

Therefore, in another preferred embodiment the above-mentioned fixed combination, non-fixed combination or kit of parts comprise a compound of the invention (in particular the compound of the invention is one of the examples of the invention or a pharmaceutically acceptable salt thereof), a vitamin D analogue and at least one pharmaceutically acceptable auxiliary. In a particularly preferred embodiment, the above mentioned fixed combination, non-fixed combination or kit of parts comprises:

a compound of the invention and calcitriol,
a compound of the invention and calcipotriol, or
a compound of the invention and tacalcitol,
and at least one pharmaceutically acceptable auxiliary.

In case, both (or all) combination partners - the compound of the invention as well as the therapeutic agent(s) -of the above-defined combinations are both (or all) suitable for inhalative administration, a preferred embodiment of the invention is the simultaneous inhaled administration of both (or all) combination partners by use of a combination inhalation device. Such a combination inhalation device can comprise a combined pharmaceutical composition for simultaneous inhaled administration, the composition comprising both (or all) individual compounds of the particular combination.

In an alternative, the combination inhalation device can be such that the individual compounds of the particular combination are administrable simultaneously but are stored separately (or wholly or partly separated for triple combinations), for example in separate pharmaceutical compositions.

In case of non-fixed combinations or kit of parts comprising at least one of the compounds of the invention and at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, the compound of the invention and the therapeutic agent may be administered by the same route, e.g., without limitation, by inhalation (or external topical), or by different routes, e.g., without limitation, the compound of the invention may be, for example, administered by inhalation and the therapeutic agent may be administered orally.

In case of co-administration of at least one compound of the invention with at least one therapeutic agent selected from the group consisting of corticosteroids, anticholinergics, $P_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, lung surfactants,

antibiotics, guanylyl-cyclase activators/stimulators, tetrahydrobiopterin and tetrahydrobiopterin derivatives, anticoagulants, diuretics, pirfenidone and digitalis glycosides, in form of a fixed combination, non-fixed combination or kit of parts the dose of the compound of the invention as well as the dose of the therapeutic agent will be in a range customary for the mono-therapy, it more likely being possible, on account of the individual action, which are mutually positively influencing and reinforcing, to reduce the respective doses in case of co-administration of the compound(s) of the invention and the therapeutic agent.

In case of co-administration of at least one compound of the invention and at least one therapeutic compound selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immunosuppressants, vitamin D analogues, HMG-CoA reductase-inhibitors, PPAR$\gamma$ agonists, ACE inhibitors, lung surfactants, antibiotics, guanylyl-cyclase activators/stimulators, tetrahydrobiopterin and tetrahydrobiopterin derivatives, anticoagulants, diuretics, pirfenidone and digitalis glycosides, in form of a fixed combination, a non-fixed combination or a kit of parts a single dose unit of the respective pharmaceutical composition/formulation can contain, in case of oral or parenteral administration 0.01 mg to 250 mg, preferably 0.05 mg to 100 mg, more preferably 0.05 mg to 10 mg, or in case of nasal or inhalative administration 0.001 mg to 10 mg, preferably 0.01 mg to 7.5 mg, more preferably 0.1 mg to 4 mg of the compound of the invention and from 0.01 mg to 4000 mg, preferably 0.1 mg to 2000 mg, more preferably 0.5 mg to 1000 mg, most preferably 1 mg to 500 mg, of the therapeutic agent, depending on the therapeutic agent being used the disease to be treated and the administration route selected. Preferably, the at least one compound of the invention and the at least one therapeutic agent are present in the pharmaceutical compositions/formulations in a weight ratio of from 1000:1 to 1:1000, more preferably in a weight ratio of from 100:1 to 1:100, even more preferably in a weight ratio of from 25:1 to 1:25.

## Biological investigations

### Method for measuring inhibition of PDE4 activity

The PDE4B1 (GB no. L20966) was a gift of Prof. M. Conti (Stanford University, USA). It was amplified from the original plasmid (pCMV5) via PCR with primers Rb18 (5'- CAGACATCCTAAGAGGGGAT -3') and Rb10 (5'- AGAGGGGGATTATGTATCCAC -3') and cloned into the pCR-Bac vector (Invitrogen, Groningen, NL).

The recombinant baculovirus was prepared by means of homologous recombination in SF9 insect cells. The expression plasmids were cotransfected with Baculo-Gold DNA (Pharmingen, Hamburg) using a standard protocol (Pharmingen, Hamburg). Wt virus-free recombinant virus supernatants were selected using plaque assay methods. After that, high-titre virus supernatants were prepared by amplifying 3 times. PDE4B1 was expressed in SF21 cells by infecting 2 x $10^6$ cells/ml with an MOI (multiplicity of infection) between 1 and 10 in the serum-free SF900 medium (GIBCO Life Technologies, Karlsruhe, Germany ). The cells were cultured at 28°C for 48 - 72 hours, after which they were pelleted for 5-10 min at 1000xg and 4°C.

The SF21 insect cells were resuspended, at a concentration of approx. $10^7$ cells/ml, in ice-cold (4°C) homogenization buffer (20 mM Tris, pH 8.2, containing the following additions: 140 mM NaCl, 3.8 mM KCl, 1 mM EGTA, 1 mM $MgCl_2$, 10 mM $\beta$-mercaptoethanol, 2 mM benzamidine, 0.4 mM Pefablock, 10 $\mu$M leupeptin, 10 $\mu$M pepstatin A, 5 $\mu$M trypsin inhibitor) and disrupted by ultrasonication. The homogenate was then centrifuged for 10 min at 1000$\times$g and the supernatant was stored at -80°C until subsequent use (see below). The protein content was determined by the Bradford method (BioRad, Munich) using BSA (Bovine serum albumin) as the standard.

PDE4B1 activities were measured in a 96-well platform using SPA (scintillation proximity assay) yttrium silicate beads (RPNQ1050 from GE Healthcare). In a first step the PDE activity operated hydrolysis of either [$^3$H] cAMP (substrate) into [$^3$H] 5'AMP. In a second step substrate and product were distinguished following addition of SPA yttrium silicate beads. Indeed, in the presence of zinc sulphate the linear [$^3$H] 5'AMP bound to the beads while the cyclic [$^3$H] cAMP did not. Close proximity of bound [$^3$H] 5'AMP then allowed radiation from the tritium to the scintillant within the beads resulting in a measureable signal while the unbound, hence distant [$^3$H] cAMP did not generate this signal. The test volume was 100 $\mu$l and finally contained 20 mM Tris buffer (pH 7.4), 0.1 mg /ml of BSA, 5 mM $Mg^{2+}$, 0.5 $\mu$M cAMP (including about 50,000 cpm of [3H]cAMP) as substrate, 1 $\mu$l of the respective substance dilution in DMSO and sufficient recombinant PDE (1000xg supernatant, see above) to ensure that 10-20% of the cAMP was converted under the said experimental conditions. The final concentration of DMSO in the assays (1 % v/v) did not substantially affect the activity of the PDE investigated. After a preincubation of 5 min at 37°C, the reaction was commenced by adding the substrate (cAMP) and the assays were incubated for a further 15 min. Reactions were terminated by adding SPA beads (50 $\mu$l). In agreement with the manufacturer's instructions, the SPA beads had previously been resuspended in water, but were then diluted 1:3 (v/v) in water. This diluted solution further contained 3 mM IBMX (isobutylmethylxanthine) to ensure a complete block of PDE activity. Once the beads were sedimented (> 30 min), the MTP's (microtiter plate) were analyzed

in commercially available luminescence detection devices. The corresponding $IC_{50}$ values of the compounds for inhibition of PDE4B1 activity were determined from the concentration-effect curves by means of non-linear regression.

For the following compounds PDE4B1 inhibitory values [measured as $-logIC_{50}$ (mol/l)] below 8 and between 8 and 9 have been determined. The numbers of the compounds correspond to the numbers of the examples.

| PDE4B1 inhibitory values measured as -logIC50 (mol/l) | |
|---|---|
| Below 8 | Between 8 and 9 |
| Example 6, 14, 18, 20, 21 | Example 1, 2, 3, 4, 5, 7, 8, 9, 10, 11, 12, 13, 15, 16, 17, 19 |

**Method for measuring inhibition of PDE5 activity.**

As a source for human PDE5, platelets were used. For that purpose, 150 ml fresh blood from human donors anticoagulated with citrate [final concentration 0.3% (w/v)] was centrifuged at 200 g for 10 min to obtain the so-called platelet-rich-plasma (PRP) as a supernatant. 1/10 volume of ACD solution (85 mM $Na_3$-citrate, 111 mM D-glucose, 71 mM citric acid, pH 4.4) was added to 9/10 volume of PRP. After centrifugation (1,400 g, 10 min) the cell pellet was resuspended in 3 ml homogenization buffer (NaCl 140 mM, KCl 3.8 mM, EGTA (ethylene glycol tetraacetic acid) 1 mM, $MgCl_2$ 1 mM, Tris-HCl 20 mM, beta-mercaptoethanol 1 mM, pH 8.2) plus protease-inhibitor mix resulting in final concentrations of 0.5 mM Pefablock (Roche), 10 μM Leupeptin, 5 μM Trypsine inhibitor, 2 mM Benzamidin and 10 μM Pepstatin A. The suspension was sonicated and thereafter centrifuged for 15 min at 10,000 g. The resulting supernatant (platelet lysate) was used for enzymatic assays.

PDE5 activities were measured in a 96-well platform using SPA (scintillation proximity assay) yttrium silicate beads (RPNQ1050 from GE Healthcare). In a first step the PDE activity operated hydrolysis of either [$^3$H] cGMP (substrate) into [$^3$H] 5'GMP. In a second step substrate and product were distinguished following addition of SPA yttrium silicate beads. Indeed, in the presence of zinc sulphate the linear [$^3$H] 5'GMP bound to the beads while the cyclic [$^3$H] cGMP did not. Close proximity of bound [$^3$H] 5'GMP then allowed radiation from the tritium to the scintillant within the beads resulting in a measureable signal while the unbound, hence distant [$^3$H] cGMP did not generate this signal. The test volume was 100 μl and contained 20 mM Tris buffer (pH 7.4), 0.1 mg of BSA (bovine serum albumin)/ml, 5 mM $Mg^{2+}$, 1 μM motapizone (PDE3 Inhibitor), 10 nM PDE2 inhibitor 2-(3,4-dimethoxybenzyl)-7-[(1*R*,2*R*)-2-hydroxy-1-(2-phenylethyl)propyl]-5-methylimidazo[5,1-*f*][1,2,4]triazin-4(3*H*)-one, 0.5 μM cGMP (cyclic guanosine monophosphate) (including about 50,000 cpm of [3H]cGMP as a tracer) (substrate), 1 μl of the respective compound dilution in dimethylsulfoxide (DMSO) and sufficient PDE5-containing platelet lysat (10,000xg supernatant, see above) to ensure that 10-20% of the cGMP was converted under the said experimental conditions. The final concentration of DMSO in the assay (1% v/v) did not substantially affect the activity of the PDE investigated. After a preincubation of 5 min at 37°C, the reaction was commenced by adding the substrate (cGMP) and the assay was incubated for a further 15 min. The reaction was terminated by adding SPA beads (50 μl). In agreement with the manufacturer's instructions, the SPA beads had previously been resuspended in water, but were then diluted 1:3 (v/v) in water. This diluted solution also contained 3 mM 8-methoxymethyl-3-isobutyl-1-methylxanthine (IBMX) to ensure a complete block of PDE activity. Once the beads were sedimented (> 30 min), the MTP's were analyzed in commercially available luminescence detection devices. The corresponding $IC_{50}$ values of the compounds for inhibition of PDE activity were determined from the concentration-effect curves by means of non-linear regression.

For the following compounds PDE5 inhibitory values [measured as $-logIC_{50}$ (mol/l)] below 8 and between 8 and 9 have been determined. The numbers of the compounds correspond to the numbers of the examples.

| PDE5 inhibitory values measured as -logIC50 (mol/l) | |
|---|---|
| Below 8 | Between 8 and 9 |
| Example 15, 17 | Example 1, 2, 3, 4, 5, 6, 7, 8, 9, 10, 11, 12, 13, 14, 16, 18, 19, 20, 21 |

**In Vivo Assay: LPS-induced pulmonary inflammation model in rats (Method B)**

**Introduction**

Exposure of rats to aerosolized lipopolysaccharide (LPS) causes a pulmonary mainly neutrophilic inflammation, which can be assessed by bronchoalveolar lavage (BAL). LPS-induced pulmonary inflammation models are robust and are commonly used for the evaluation of test compounds modulating the immediate immune response. Selective phosphodiesterase-4 inhibitors are administered by intratracheal instillation 1 h prior nose-only LPS challenge in rats. The anti-inflammatory activity of the selective phosphodiesterase inhibitors is assessed based on pulmonary total leukocyte and neutrophil counts in the bronchoalveolar lavage fluid 4 h after LPS exposure.

**Materials and Methods**

**Animals**

Male Sprague Dawley rats weighing 200 - 300 g were used. Rats were delivered 1 weak prior to the experiments and had free access to water and food.

**Intratracheal compound instillation**

**Compound preparation**

The test compound was suspended in Aqua ad injectabilia (Braun, Melsungen, Germany) or 0.9 % NaCl (Saline) (Braun, Melsungen, Germany) supplemented with 0.02% Tween20 (Sigma-Aldrich, Schnelldorf, Germany) for intratracheal instillation. Suspensions of test compound were treated in an ultrasonic bath or a Covaris S2x high-energetic ultrasonic bath (KBiosciences, Hoddesdon Herts, UK) to obtain a homogenous suspension. The aimed doses were prepared by dilution series from the stock suspension, which was prepared for the administration of the highest dose in each experiment.

**Compound instillation technique**

The compound suspension was administered intratracheally. The intubation was guided by sight and was done under a short time isoflurane anesthesia. The compound suspension was administered to the lungs by liquid instillation. Therefore, the trachea was intubated with a device consisting of a catheter which contained a blunted cannula (size 14G, Dispomed, Gelnhausen, Germany). The length of the catheter was adjusted to avoid disruption of the tracheal bifurcation. A 1 ml syringe, filled with the compound suspension and air, was connected to the intubation device via the Luer Lock adapter and the whole content of the syringe was directly administered to the lungs.

**Compound dosing**

The administered volume of the compound suspension is 0.5 - 1ml/kg. Control animals received drug-free Aqua/Tween20 or NaCl/Tween20 solution as placebo. Test compounds and placebo were administered 1 h prior to LPS challenge.

**LPS challenge**

Conscious and restrained animals were connected to a nose-only exposure system (CR equipment SA, Tannay, Switzerland) and were exposed to the LPS aerosol for 30 min. The LPS-containing aerosol was generated using a compressed air driven medication nebulizer device (Pari master in combination with Pari LC Sprint Star, Pari GmbH, Starnberg, Germany). The LPS solution (E. coli, Serotype 055B5, Art.# L2880, Lot# L048K4126 or 109K4075, Sigma-Aldrich, Germany, 1 mg/ml - 3 mg/ml diluted in Phosphate-Buffered Saline (PBS)) was prepared 30 minutes in advance. The aerosol was dispersed and transported to the exposure tower by a sheath air flow of 600 l/h. All rats except negative controls were exposed to LPS.

**Bronchoalveolar lavage**

4 hours after LPS challenge animals were anesthetized by isoflurane and sacrificed by cervical dislocation. BALs were performed. For the BAL the trachea was exposed and cannulated, followed by gently lavage of the lungs two times in situ with 5 ml PBS buffer supplemented with 0.5% Bovine Serum Albumin (Serva, Darmstadt, Germany).

**Total and differential cell counts**

Determination of total leukocyte and neutrophil counts in BAL fluid was performed with an automated haemocytometer (XT-2000iV, Sysmex, Norderstedt, Germany).

**Data Analysis**

The baseline correction was done for each sample according to the formula:

*Baseline-corrected cell count value = cell count - Median (negative control group)*

All further calculations were performed with the baseline-corrected values.

The effect of a compound on LPS-induced total cell and neutrophil influx into the lungs was calculated in % using the median of the cell count of each treatment group in relation to the median of the control group according to the formula:

$$\% \text{ effect} = (Y-K)/K*100$$

With defining:

Y= Median of the baseline-corrected cell count value of compound-treated group
K= Median of the baseline-corrected cell count value of placebo-treated group

Statistical analysis was performed on the primary cell count data using one-way ANOVA and Dunnett's multiple comparison post test vs. positive control. The Grubbs test was used to detect statistical outliers.

**Exemplary Results for compounds tested using Method B** (the numbers of the compounds correspond to the numbers of the examples):

The **compounds 3, 8 and 12** showed at a dosage of 1 mg/kg a reduction in the range of 28 to 47 % of the total cell count, respectively a reduction in the range of 25 to 45 % of neutrophils in comparison to the placebo group.

## Claims

**1.** A compound of formula (1)

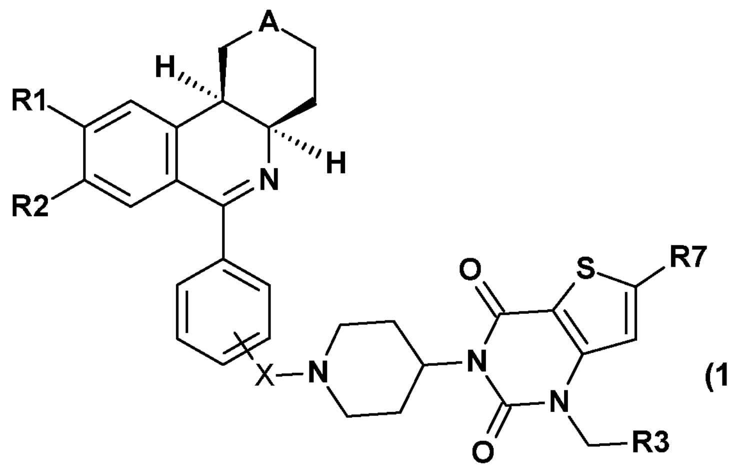

(1)

wherein

A is S, S(O) or $S(O)_2$,
X is C(O) and is attached either in ortho, meta or para position to the phenyl,
R1 is 1-4C-alkoxy, 3-7C-cycloalkoxy, 3-7C-cycloalkylmethoxy or 1-4C-alkoxy predominantly or completely substituted by fluorine,
R2 is 1-4C-alkoxy, 3-7C-cycloalkoxy, 3-7C-cycloalkylmethoxy, or 1-4C-alkoxy predominantly or completely substituted by fluorine, or
R1 and R2 together form a 1-2C-alkylenedioxy group,

R3 is unsubstituted phenyl, phenyl substituted by R4 and R5 or phenyl substituted by R4, R5 and R6, wherein R4 is 1-4C-alkyl, 1-4C-alkoxy or halogen, R5 is 1-4C-alkyl, 1-4C-alkoxy or halogen and R6 is 1-4Calkyl, 1-4C-alkoxy or halogen,
R7 is unsubstituted phenyl, phenyl substituted by R8 or phenyl substsituted by R8 and R9, wherein R8 is 1-4C-alkyl, 1-4C-alkoxy or halogen and R9 is 1-4C-alkyl, 1-4C-alkoxy or halogen or R8 and R9 together form a 1-2C-alkylenedioxy group,

or a stereoisomer of the compound.

**2.** A compound of formula (1) according to claim 1, wherein

A is S, S(O) or $S(O)_2$,
X is C(O) and is attached either in meta or para position to the phenyl,
R1 is 1-4C-alkoxy or 1-4C-alkoxy predominantly or completely substituted by fluorine,
R2 is 1-4C-alkoxy or 1-4C-alkoxy predominantly or completely substituted by fluorine, or
R1 and R2 together form a 1-2C-alkylenedioxy group,
R3 is unsubstituted phenyl, phenyl substituted by R4 and R5 or phenyl substituted by R4, R5 and R6, wherein R4 is 1-4C-alkyl, 1-4C-alkoxy or halogen, R5 is 1-4C-alkoxy or halogen and R6 is 1-4C-alkoxy or halogen,
R7 is unsubstituted phenyl, phenyl substituted by R8 or phenyl substsituted by R8 and R9, wherein R8 is 1-4C-alkoxy or halogen and R9 is 1-4C-alkyl, 1-4C-alkoxy or halogen, or R8 and R9 together form a 1-2C-alkylenedioxy group,

or a stereoisomer of the compound.

**3.** A compound of formula (1) according to claim 1 or 2, wherein

A is S, S(O) or $S(O)_2$,
X is C(O) and is attached either in meta or para position to the phenyl,
R1 is 1-2C-alkoxy or 1-2C-alkoxy predominantly or completely substituted by fluorine,
R2 is 1-2C-alkoxy or 1-2C-alkoxy predominantly or completely substituted by fluorine, or
R1 and R2 together form a 1-2C-alkylenedioxy group,
R3 is unsubstituted phenyl, phenyl substituted by R4 and R5 or phenyl substituted by R4, R5 and R6, wherein R4 is 1-4C-alkyl, 1-4C-alkoxy or halogen, R5 is 1-4C-alkoxy or halogen and R6 is 1-4C-alkoxy or halogen,
R7 is unsubstituted phenyl, phenyl substituted by R8 or phenyl substsituted by R8 and R9, wherein R8 is 1-2C-alkoxy, fluorine, chlorine or bromine and R9 is 1-2C-alkyl, 1-2C-alkoxy, fluorine, chlorine or bromine, or R8 and R9 together form a 1-2C-alkylenedioxy group,

or a stereoisomer of the compound.

**4.** A compound of formula (1) according to claim 3, wherein

A is S, S(O) or $S(O)_2$,
X is C(O) and is attached either in meta or para position to the phenyl,
R1 is ethoxy,
R2 is methoxy,
R3 is unsubstituted phenyl, phenyl substituted by R4 and R5 or phenyl substituted by R4, R5 and R6, wherein R4 is 1-2C-alkyl, 1-2C-alkoxy, fluorine, chlorine or bromine, R5 is 1-2C-alkoxy, fluorine, chlorine or bromine and R6 is 1-2C-alkoxy, fluorine, chlorine or bromine,
R7 is unsubstituted phenyl, phenyl substituted by R8 or phenyl substsituted by R8 and R9,
wherein
R8 is methoxy or fluorine and
R9 is methyl, methoxy or fluorine
or
R8 and R9 together form a methylenedioxy group,
or a stereoisomer of the compound.

**5.** A compound of formula (1) according to claim 4, wherein

A is S, S(O) or $S(O)_2$,
X is C(O) and is attached either in meta or para position to the phenyl,
R1 is ethoxy,
R2 is methoxy,
R3 is phenyl substituted by R4 and R5 or phenyl substituted by R4, R5 and R6, wherein R4 is methyl, methoxy or fluorine, R5 is methoxy or fluorine and R6 is methoxy or fluorine,
R7 is unsubstituted phenyl, phenyl substituted by R8 or phenyl substsituted by R8 and R9, wherein R8 is methoxy or fluorine, R9 is methyl, methoxy or fluorine, or R8 and R9 together form a methylenedioxy group,

or a stereoisomer of the compound.

**6.** A compound of formula (1) according to claim 5, which is selected from the group consisting of 3-(1-{4-[(4aR,10bR)-9-ethoxy-8-methoxy-3,4,4a,10b-tetrahydro-1H-thiopyrano[4,3-c]isoquinolin-6-yl]benzoyl}piperidin-4-yl)-1-(3-fluoro-4-methoxybenzyl)-6-phenylthieno[3,2-d]pyrimidine-2,4(1H,3H)-dione; 3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-phenylthieno[3,2-d]pyrimidine-2,4(1H,3H)-dione; 1-(3,5-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione; 1-(2,3-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione; 3-[1-({4-[(4aR,10bR)-9-ethoxy-8-methoxy-3,4,4a,10b-tetrahydro-1H-thio-pyrano[4,3-c]isoquinolin-6-yl]phenyl}carbonyl)piperidin-4-yl]-1-(2-fluoro-4,5-dimethoxy-benzyl)-6-phenylthieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methylbenzyl)-6-phenylthieno[3,2-d]pyrimidine-2,4(1H,3H)-dione; 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-phenylthieno[3,2-d]pyrimidine-2,4(1H,3H)-dione; 6-(1,3-benzodioxol-5-yl)-3-[1-({4-[(4aR, 10bR)-9-ethoxy-8-methoxy-3,4,4a, 10b-tetrahydro-1H-thio-pyrano[4,3-c]isoquinolin-6-yl]phenyl}carbonyl)piperidin-4-yl]-1-(3-fluoro-4-methoxybenzyl) thieno-[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methoxyphenyl) thieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]-phenyl}carbonyl)piperidin-4-yl]-1-(3-fluoro-4-methoxybenzyl)-6-(5-fluoro-2-methoxyphenyl)-thieno[3,2-d]pyrimidine-2,4(1H,3H)-dione; 6-(2,5-dimethoxyphenyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)-piperidin-4-yl]-1-(3-fluoro-4-methoxybenzyl) thieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]-phenyl}carbonyl)piperidin-4-yl]-1-(3-fluoro-4-methoxybenzyl)-6-(2-fluorophenyl)thieno[3,2-d]-pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxy-benzyl)-6-(4-fluorophenyl)thieno[3,2-d]pyrimidine-2,4(1H,3H)-dione; 1-(3,5-difluoro-4-methoxy-benzyl)-3-[1-({4-[(4aR,10bR)-9-ethoxy-8-methoxy-3,4,4a,10b-tetrahydro-1H-thiopyrano[4,3-c]-isoquinolin-6-yl]phenyl}carbonyl)piperidin-4-yl]-6-(4-fluoro-2-methoxyphenyl)thieno[3,2-d]-pyrimidine-2,4(1H,3H)-dione; 1-(2,3-difluoro-4-methoxybenzyl)-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)thieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)-piperidin-4-yl]-6-(4-fluoro-2-methoxyphenyl)-1-(3-fluoro-4-methylbenzyl) thieno[3,2-d]pyrimidine-2,4(1H,3H)-dione, 1-benzyl-3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-6-(4-fluoro-2-methoxyphenyl)-thieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxybenzyl)-6-(4-fluoro-2-methylphenyl) thieno[3,2-d]pyrimidine-2,4(1H,3H)-dione; 3-[1-({4-[(4aR,10bR)-9-ethoxy-8-methoxy-3,4,4a,10b-tetrahydro-1H-thiopyrano[4,3-c]isoquinolin-6-yl]-phenyl}carbonyl)piperidin-4-yl]-1-(3-fluoro-4-methoxybenzyl)-6-(2-methoxyphenyl)thieno[3,2-d]-pyrimidine-2,4(1H,3H)-dione, 6-(1,3-benzodioxol-5-yl)-3-[1-({3-[(4aR,10bR)-9-ethoxy-8-methoxy-3,4,4a,10b-tetrahydro-1H-thiopyrano[4,3-c]isoquinolin-6-yl]phenyl}carbonyl)piperidin-4-yl]-1-(3-fluoro-4-methoxy-benzyl)thieno[3,2-d]pyrimidine-2,4(1H,3H)-dione and 1-(3,5-difluoro-4-methoxy-benzyl)-3-[1-({3-[(4aR,10bR)-9-ethoxy-8-methoxy-3,4,4a,10b-tetrahydro-1H-thiopyrano[4,3-c]-isoquinolin-6-yl]phenyl}carbonyl)piperidin-4-yl]-6-phenylthieno[3,2-d]pyrimidine-2,4(1H,3H)-dione.

**7.** A compound of formula (1) according to any one of claims 1 to 6 for use in the treatment or prophylaxis of diseases.

**8.** Pharmaceutical composition comprising at least one of the compounds of formula (1) or a stereoisomer thereof according to any one of claims 1 to 6 together with at least one pharmaceutically acceptable auxiliary.

**9.** Fixed combination, non-fixed combination or kit of parts comprising at least one compound of formula (1) or a stereoisomer thereof according to any one of claims 1 to 6, at least one therapeutic agent selected from the group consisting of corticosteroids, anticholinergics, $\beta_2$-adrenoreceptor agonists, H1 receptor antagonists, leukotriene receptor antagonists, 5-lipoxygenase inhibitors, endothelin receptor antagonists, prostacyclines, calcium channel blockers, beta-blockers, type 4 phosphodiesterase inhibitors, type 5 phosphodiesterase inhibitors, immuno-suppressants, vitamin D analogues, HMG-CoA reductase-inhibitors, PPAR$\gamma$ agonists, ACE inhibitors, angiotensin II-receptor antagonists , lung surfactants, antibiotics, guanylyl-cyclase activators/stimulators, tetrahydrobiopterin and tetrahydrobiopterin derivatives, anticoagulants, diuretics, pirfenidone and digitalis glycosides, and at least one pharmaceutically acceptable auxiliary.

**10.** Use of a compound of formula (1) or a stereoisomer thereof according to any one of claims 1 to 6 in the manufacture of a pharmaceutical composition for the treatment or prophylaxis of an acute or chronic airway disease.

**11.** Use according to claim 10, wherein the acute or chronic airway disease is selected from the group consisting of interstitial lung disease, pulmonary fibrosis, cystic fibrosis, bronchial asthma, chronic bronchitis, emphysema, chronic obstructive pulmonary disease (COPD) and COPD associated with pulmonary hypertension.

**12.** A compound of formula (1) or a stereoisomer thereof according to any one of claims 1 to 6 for use in the treatment or prophylaxis of an acute or chronic airway disease.

**13.** A compound of formula (1) or a stereoisomer thereof according to any one of claims 1 to 6 for use in the treatment or prophylaxis of an acute or chronic airway diseases selected from the group consisting of interstitial lung disease, pulmonary fibrosis, cystic fibrosis, bronchial asthma, chronic bronchitis, emphysema, chronic obstructive pulmonary disease (COPD) and COPD associated with pulmonary hypertension.

## Patentansprüche

**1.** Verbindung der Formel (1)

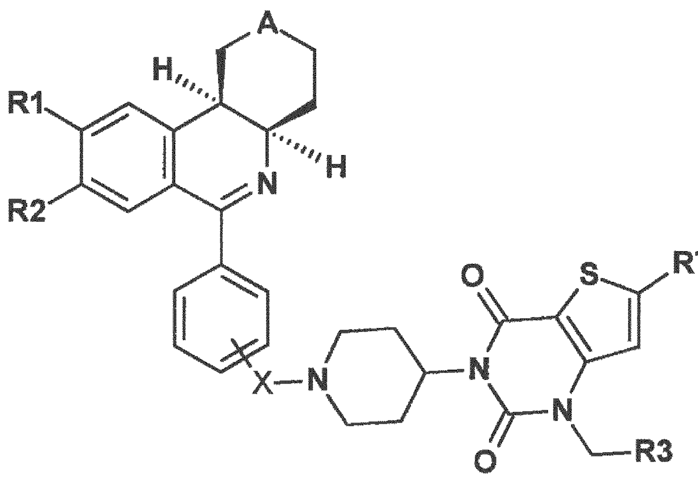

(1)

worin

A S, S(O) oder $S(O)_2$ bedeutet,
X C(O) bedeutet und entweder in ortho-, in meta- oder in para-Stellung an das Phenyl gebunden ist,
R1 1-4C-Alkoxy, 3-7C-Cycloalkoxy, 3-7C-Cycloalkyl-methoxy oder vorwiegend oder vollständig durch Fluor substituiertes 1-4C-Alkoxy bedeutet,
R2 1-4C-Alkoxy, 3-7C-Cycloalkoxy, 3-7C-Cycloalkyl-methoxy oder vorwiegend oder vollständig durch Fluor substituiertes 1-4C-Alkoxy bedeutet, oder

R1 und R2 zusammen eine 1-2C-Alkylendioxygruppe bilden,
R3 unsubstituiertes Phenyl, Phenyl, das durch R4 und R5 substituiert ist, oder Phenyl, das durch R4, R5 und R6 substituiert ist, bedeutet, wobei R4 1-4C-Alkyl, 1-4C-Alkoxy oder Halogen bedeutet, R5 1-4C-Alkyl, 1-4C-Alkoxy oder Halogen bedeutet und R6 1-4C-Alkyl, 1-4C-Alkoxy oder Halogen bedeutet,
R7 unsubstituiertes Phenyl, Phenyl, das durch R8 substituiert ist, oder Phenyl, das durch R8 und R9 substituiert ist, bedeutet, wobei R8 1-4C-Alkyl, 1-4C-Alkoxy oder Halogen bedeutet und R9 1-4C-Alkyl, 1-4C-Alkoxy oder Halogen bedeutet, oder R8 und R9 zusammen eine 1-2C-Alkylendioxygruppe bilden, oder ein Stereoisomer der Verbindung.

**2.** Verbindung der Formel (1) nach Anspruch 1, worin

A S, S(O) oder $S(O)_2$ bedeutet,
X C(O) bedeutet und entweder in meta- oder in para-Stellung an das Phenyl gebunden ist,
R1 1-4C-Alkoxy oder vorwiegend oder vollständig durch Fluor substituiertes 1-4C-Alkoxy bedeutet,
R2 1-4C-Alkoxy oder vorwiegend oder vollständig durch Fluor substituiertes 1-4C-Alkoxy bedeutet, oder
R1 und R2 zusammen eine 1-2C-Alkylendioxygruppe bilden,
R3 unsubstituiertes Phenyl, Phenyl, das durch R4 und R5 substituiert ist, oder Phenyl, das durch R4, R5 und R6 substituiert ist, bedeutet, wobei R4 1-4C-Alkyl, 1-4C-Alkoxy oder Halogen bedeutet, R5 1-4C-Alkoxy oder Halogen bedeutet und R6 1-4C-Alkoxy oder Halogen bedeutet,
R7 unsubstituiertes Phenyl, Phenyl, das durch R8 substituiert ist, oder Phenyl, das durch R8 und R9 substituiert ist, bedeutet, wobei R8 1-4C-Alkoxy oder Halogen bedeutet und R9 1-4C-Alkyl, 1-4C-Alkoxy oder Halogen bedeutet, oder R8 und R9 zusammen eine 1-2C-Alkylendioxygruppe bilden,

oder ein Stereoisomer der Verbindung.

**3.** Verbindung der Formel (1) nach Anspruch 1 oder 2, worin

A S, S(O) oder $S(O)_2$ bedeutet,
X C(O) bedeutet und entweder in meta- oder in para-Stellung an das Phenyl gebunden ist,
R1 1-2C-Alkoxy oder vorwiegend oder vollständig durch Fluor substituiertes 1-2C-Alkoxy bedeutet,
R2 1-2C-Alkoxy oder vorwiegend oder vollständig durch Fluor substituiertes 1-2C-Alkoxy bedeutet, oder
R1 und R2 zusammen eine 1-2C-Alkylendioxygruppe bilden,
R3 unsubstituiertes Phenyl, Phenyl, das durch R4 und R5 substituiert ist, oder Phenyl, das durch R4, R5 und R6 substituiert ist, bedeutet, wobei R4 1-4C-Alkyl, 1-4C-Alkoxy oder Halogen bedeutet, R5 1-4C-Alkoxy oder Halogen bedeutet und R6 1-4C-Alkoxy oder Halogen bedeutet,
R7 unsubstituiertes Phenyl, Phenyl, das durch R8 substituiert ist, oder Phenyl, das durch R8 und R9 substituiert ist, bedeutet, wobei R8 1-2C-Alkoxy, Fluor, Chlor oder Brom bedeutet und R9 1-2C-Alkyl, 1-2C-Alkoxy, Fluor, Chlor oder Brom bedeutet, oder R8 und R9 zusammen eine 1-2C-Alkylendioxygruppe bilden,

oder ein Stereoisomer der Verbindung.

**4.** Verbindung der Formel (1) nach Anspruch 3, worin

A S, S(O) oder $S(O)_2$ bedeutet,
X C(O) bedeutet und entweder in meta- oder in para-Stellung an das Phenyl gebunden ist,
R1 Ethoxy bedeutet,
R2 Methoxy bedeutet,
R3 unsubstituiertes Phenyl, Phenyl, das durch R4 und R5 substituiert ist, oder Phenyl, das durch R4, R5 und R6 substituiert ist, bedeutet, wobei R4 1-2C-Alkyl, 1-2C-Alkoxy, Fluor, Chlor oder Brom bedeutet, R5 1-2C-Alkoxy, Fluor, Chlor oder Brom bedeutet und R6 1-2C-Alkoxy, Fluor, Chlor oder Brom bedeutet,
R7 unsubstituiertes Phenyl, Phenyl, das durch R8 substituiert ist, oder Phenyl, das durch R8 und R9 substituiert ist, bedeutet, wobei R8 Methoxy oder Fluor bedeutet und R9 Methyl, Methoxy oder Fluor bedeutet, oder R8 und R9 zusammen eine Methylendioxygruppe bilden,

oder ein Stereoisomer der Verbindung.

**5.** Verbindung der Formel (1) nach Anspruch 4, worin

A S, S(O) oder $S(O)_2$ bedeutet,
X C(O) bedeutet und entweder in meta- oder in para-Stellung an das Phenyl gebunden ist,
R1 Ethoxy bedeutet,
R2 Methoxy bedeutet,
R3 Phenyl, das durch R4 und R5 substituiert ist, oder Phenyl, das durch R4, R5 und R6 substituiert ist, bedeutet, wobei R4 Methyl, Methoxy oder Fluor bedeutet, R5 Methoxy oder Fluor bedeutet und R6 Methoxy oder Fluor bedeutet,
R7 unsubstituiertes Phenyl, Phenyl, das durch R8 substituiert ist, oder Phenyl, das durch R8 und R9 substituiert ist, bedeutet, wobei R8 Methoxy oder Fluor bedeutet, R9 Methyl, Methoxy oder Fluor bedeutet, oder R8 und R9 zusammen eine Methylendioxygruppe bilden, oder ein Stereoisomer der Verbindung.

**6.** Verbindung der Formel (1) nach Anspruch 5, ausgewählt aus der Gruppe bestehend aus 3-(1-{4-[(4aR,10bR)-9-Ethoxy-8-methoxy-3,4,4a,10b-tetra-hydro-1H-thiopyrano[4,3-c]isochinolin-6-yl]-benzoyl}piperidin-4-yl)-1-(3-fluor-4-methoxy-benzyl)-6-phenylthieno[3,2-d]pyrimidin-2,4(1H,3H)-dion; 3-[1-({3-[(4aR,10bR)-9-Ethoxy-8-methoxy-3,4,4a,10b-tetrahydro-1H-thiopyrano[4,3-c]isochinolin-6-yl]phenyl}carbonyl)piperidin-4-yl]-1-(3-fluor-4-methoxy-benzyl)-6-phenylthieno-[3,2-d]pyrimidin-2,4(1H,3H)-dion; 1-(3,5-Difluor-4-methoxybenzyl)-3-[1-({4-[(4aR,10bR)-9-ethoxy-8-methoxy-3,4,4a,10b-tetrahydro-1H-thiopyrano[4,3-c]isochinolin-6-yl]phenyl}carbonyl)piperidin-4-yl]-6-phenylthieno[3,2-d]pyrimidin-2,4-(1H,3H)-dion; 1-(2,3-Difluor-4-methoxybenzyl)-3-[1-({4-[(4aR,10bR)-9-ethoxy-8-methoxy-3,4,4a,10b-tetra-hydro-1H-thiopyrano[4,3-c]isochinolin-6-yl]-phenyl}carbonyl)piperidin-4-yl]-6-phenylthieno-[3,2-d]pyrimidin-2,4(1H,3H)-dion; 3-[1-({4-[(4aR,10bR)-9-Ethoxy-8-methoxy-3,4,4a,10b-tetra-hydro-1H-thiopyrano[4,3-c]isochinolin-6-yl]-phenyl}carbonyl)piperidin-4-yl]-1-(2-fluor-4,5-dimethoxybenzyl)-6-phenylthieno[3,2-d]pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]isochinolin-6-yl]phenyl}carbonyl)piperidin-4-yl]-1-(3-fluor-4-methylbenzyl)-6-phenylthieno[3,2-d]pyrimidin-2,4(1H,3H)-dion; 1-Benzyl-3-[1-({4-[(4aR,10bR)-9-ethoxy-8-methoxy-3,4,4a,10b-tetra-hydro-1H-thiopyrano[4,3-c]isochinolin-6-yl]-phenyl}carbonyl)piperidin-4-yl]-6-phenylthieno-[3,2-d]pyrimidin-2,4(1H,3H)-dion; 6-(1,3-Benzo-dioxol-5-yl)-3-[1-({4-[(4aR,10bR)-9-ethoxy-8-methoxy-3,4,4a,10b-tetrahydro-1H-thiopyrano[4,3-c]isochinolin-6-yl]phenyl}carbonyl)piperidin-4-yl]-1-(3-fluor-4-methoxybenzyl)thieno-[3,2-d]-pyrimidin-2,4(1,3H)-dion; 3-[1({4-[(4aR,10bR)-9-Ethoxy-8-methoxy-3,4,4a,10b-tetrahydro-1H-thio-pyrano[4,3-c]isochinolin-6-yl]phenyl}carbonyl)-piperidin-4-yl]-1-(3-fluor-4-methoxybenzyl)-6-(4-fluor-2-methoxyphenyl)thieno[3,2-d]pyrimidin-2,4(1H,3H)-dion; 3-[1-(14-[(4aR,10bR)-9-Ethoxy-8-methoxy-3,4,4a,10b-tetrahydro-1H-thiopyrano[4,3-c]isochinolin-6-yl]phenyl}carbonyl)piperidin-4-yl]-1-(3-fluor-4-methoxybenzyl)-6-(5-fluor-2-methoxyphenyl)thieno[3,2-d]pyrimidin-2,4(1H,3H)-dion; 6-(2,5-Dimethoxyphenyl)-3-[1-({4-[(4aR,10bR)-9-ethoxy-8-methoxy-3,4,4a,10b-tetra-hydro-1H-thiopyrano[4,3-c]isochinolin-6-yl]-phenyl}carbonyl)piperidin-4-yl]-1-(3-fluor-4-methoxybenzyl)thieno[3,2-d]pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]-isochinolin-6-yl]phenyl}carbonyl)piperidin-4-yl]-1-(3-fluor-4-methoxybenzyl)-6-(2-fluorphenyl)-thieno[3,2-d]pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]isochinolin-6-yl]-phenyl}carbonyl)piperidin-4-yl]-1-(3-fluor-4-methoxybenzyl)-6-(4-fluorphenyl)thieno[3,2-d]-pyrimidin-2,4(1H,3H)-dion; 1-(3,5-Difluor-4-methoxybenzyl)-3-[1-({4-[(4aR,10bR)-9-ethoxy-8-methoxy-3,4,4a,10b-tetrahydro-1H-thiopyrano[4,3-c]isochinolin-6-yl]phenyl}carbonyl)piperidin-4-yl]-6-(4-fluor-2-methoxyphenyl)thieno[3,2-d]-pyrimidin-2,4(1H,3H)-dion; 1-(2,3-Difluor-4-methoxybenzyl)-3-[1-({4-[(4aR,10bR)-9-ethoxy-8-methoxy-3,4,4a,10b-tetrahydro-1H-thiopyrano[4,3-c]isochinolin-6-yl]phenyl}carbonyl)piperidin-4-yl]-6-(4-fluor-2-methoxy-phenyl)thieno[3,2-d]-pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]isochinolin-6-yl]phenyl}-carbonyl)piperidin-4-yl]-6-(4-fluor-2-methoxy-phenyl)-1-(3-fluor-4-methylbenzyl)thieno[3,2-d]-pyrimidin-2,4(1H,3H)-dion, 1-Benzyl-3-[1-({4-[(4aR,10bR)-9-ethoxy-8-methoxy-3,4,4a,10b-tetra-hydro-1H-thiopyrano[4,3-c]isochinolin-6-yl]-phenyl}carbonyl)piperidin-4-yl]-6-(4-fluor-2-methoxy-phenyl)thieno[3,2-d]pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]iso-chinolin-6-yl]phenyl}carbonyl)piperidin-4-yl]-1-(3-fluor-4-methoxybenzyl)-6-(4-fluor-2-methylphenyl)thieno[3,2-d]pyrimidin-2,4(1H,3H)-dion; 3-[1-({4-[(4aR,10bR)-9-Ethoxy-8-methoxy-3,4,4a,10b-tetrahydro-1H-thiopyrano[4,3-c]isochinolin-6-yl]-phenyl}carbonyl)piperidin-4-yl]-1-(3-fluor-4-methoxybenzyl)-6-(2-methoxyphenyl)thieno[3,2-d]-pyrimidin-2,4(1H,3H)-dion, 6-(1,3-Benzodioxol-5-yl)-3-[1-({3-[(4aR,10bR)-9-ethoxy-8-methoxy-3,4,4a,10b-tetrahydro-1H-thiopyrano[4,3-c]iso-chinolin-6-yl]phenyl}carbonyl)piperidin-4-yl]-1-(3-fluor-4-methoxybenzyl)thieno[3,2-d]pyrimidin-2,4(1H,3H)-dion und 1-(3,5-Difluor-4-methoxy-benzyl)-3-[1-({3-[(4aR,10bR)-9-ethoxy-8-methoxy-3,4,4a,10b-tetrahydro-1H-thiopyrano[4,3-c]-iso-chinolin-6-yl]phenyl}carbonyl)piperidin-4-yl]-6-phenylthieno[3,2-d]pyrimidin-2,4(1H,3H)-dion.

**7.** Verbindung der Formel (1) nach einem der Ansprüche 1 bis 6 zur Verwendung bei der Behandlung oder Prophylaxe von Erkrankungen.

8. Pharmazeutische Zusammensetzung, enthaltend mindestens eine der Verbindungen der Formel (1) oder ein Stereoisomer davon nach einem der Ansprüche 1 bis 6 zusammen mit mindestens einem pharmazeutisch unbedenklichen Hilfsstoff.

9. Fixe Kombination, nichtfixe Kombination oder Kit von Teilen, umfassend mindestens eine Verbindung der Formel (1) oder ein Stereoisomer davon nach einem der Ansprüche 1 bis 6, mindestens ein therapeutisches Mittel ausgewählt aus der Gruppe bestehend aus Corticosteroiden, Anticholinergika, $\beta_2$-Adrenorezeptor-Agonisten, H1-Rezeptor-Antagonisten, Leukotrienrezeptor-Antagonisten, 5-Lipoxygenase-Inhibitoren, Endothelinrezeptor-Antagonisten, Prostacyclinen, Calciumkanalblockern, Beta-Blockern, Typ-4-Phosphodiesterase-Inhibitoren, Typ-5-Phosphodiesterase-Inhibitoren, Immunsuppressiva, Vitamin-D-Analoga, HMG-CoA-Reductase-Inhibitoren, PPAR$\gamma$-Agonisten, ACE-Inhibitoren, Angiotensin-II-Rezeptor-Antagonisten, Lungen-Surfactants, Antibiotika, Aktivatoren/Stimulatoren der Guanylylcyclase, Tetrahydrobiopterin und Tetrahydrobiopterinderivaten, Antikoagulantien, Diuretika, Pirfenidon und Digitalis-Glycosiden und mindestens einen pharmazeutisch unbedenklichen Hilfsstoff.

10. Verwendung einer Verbindung der Formel (1) oder eines Stereoisomers davon nach einem der Ansprüche 1 bis 6 bei der Herstellung einer pharmazeutischen Zusammensetzung zur Behandlung oder Prophylaxe einer akuten oder chronischen Atemwegserkrankung.

11. Verwendung nach Anspruch 10, wobei die akute oder chronische Atemwegserkrankung aus der Gruppe bestehend aus interstitieller Lungenerkrankung, Lungenfibrose, zystischer Fibrose, Bronchial-asthma, chronischer Bronchitis, Emphysem, chronisch-obstruktiver Lungenerkrankung (COPD) und mit pulmonaler Hypertonie assoziierter COPD ausgewählt ist.

12. Verbindung der Formel (1) oder ein Stereoisomer davon nach einem der Ansprüche 1 bis 6 zur Verwendung bei der Behandlung oder Prophylaxe einer akuten oder chronischen Atemwegserkrankung.

13. Verbindung der Formel (1) oder ein Stereoisomer davon nach einem der Ansprüche 1 bis 6 zur Verwendung bei der Behandlung oder Prophylaxe einer akuten oder chronischen Atemwegserkrankung ausgewählt aus der Gruppe bestehend aus interstitieller Lungenerkrankung, Lungenfibrose, zystischer Fibrose, Bronchialasthma, chronischer Bronchitis, Emphysem, chronisch-obstruktiver Lungenerkrankung (COPD) und mit pulmonaler Hypertonie assoziierter COPD.

**Revendications**

1. Composé de formule (1)

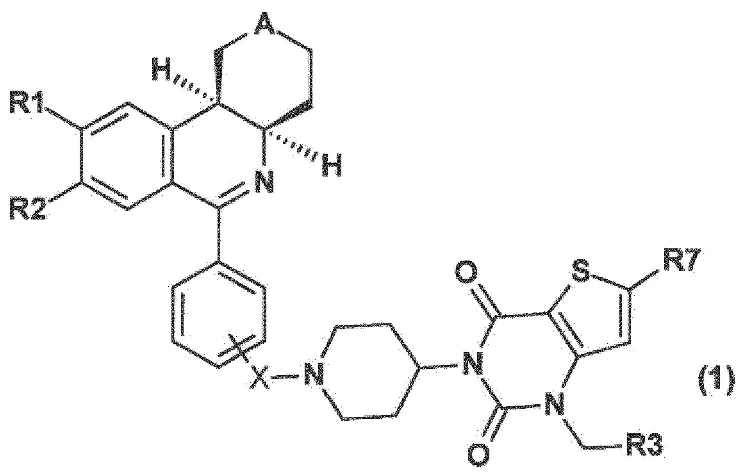

(1)

où

A représente S, S(O) or $S(O)_2$,
X représente C(O) et est lié au groupement phényle en position ortho, méta ou para,

R1 représente un groupement alkoxy en C1-4, cycloalkoxy en C3-7, (cycloalkyle en C3-7)-méthoxy ou alkoxy en C1-4 entièrement ou majoritairement fluoré,
R2 représente un groupement alkoxy en C1-4, cycloalkoxy en C3-7, (cycloalkyle en C3-7)-méthoxy ou alkoxy en C1-4 entièrement ou majoritairement fluoré, ou
R1 et R2 forment ensemble un groupement alkylènedioxy en C1-2,
R3 représente un groupement phényle non substitué, phényle substitué par R4 et R5 ou phényle substitué par R4, R5 et R6, où
R4 représente un groupement alkyle en C1-4, alkoxy en C1-4 ou halogène,
R5 représente un groupement alkyle en C1-4, alkoxy en C1-4 ou halogène, et
R6 représente un groupement alkyle en C1-4, alkoxy en C1-4 ou halogène,
R7 représente un groupement phényle non substitué, phényle substitué par R8 ou phényle substitué par R8 et R9, où
R8 représente un groupement alkyle en C1-4, alkoxy en C1-4 ou halogène, et
R9 représente un groupement alkyle en C1-4, alkoxy en C1-4 ou halogène,
ou R8 et R9 forment ensemble un groupement alkylènedioxy en C1-2, ou l'un des stéréoisomères du composé.

**2.** Composé de formule (1) selon la revendication 1,
où
A représente S, S(O) or $S(O)_2$,
X représente C(O) et est lié au groupement phényle en position méta ou para,
R1 représente un groupement alkoxy en C1-4 ou alkoxy en C1-4 entièrement ou majoritairement fluoré,
R2 représente un groupement alkoxy en C1-4 ou alkoxy en C1-4 entièrement ou majoritairement fluoré, ou
R1 et R2 forment ensemble un groupement alkylènedioxy en C1-2,
R3 représente un groupement phényle non substitué, phényle substitué par R4 et R5 ou phényle substitué par R4, R5 et R6, où
R4 représente un groupement alkyle en C1-4, alkoxy en C1-4 ou halogène,
R5 représente un groupement alkoxy en C1-4 ou halogène, et
R6 représente un groupement alkoxy en C1-4 ou halogène, R7 représente un groupement phényle non substitué, phényle substitué par R8 ou phényle substitué par R8 et R9, où
R8 représente un groupement alkoxy en C1-4 ou halogène, et
R9 représente un groupement alkyle en C1-4, alkoxy en C1-4 ou halogène,
ou
R8 et R9 forment ensemble un groupement alkylènedioxy en C1-2,
ou l'un des stéréoisomères du composé.

**3.** Composé de formule (1) selon la revendication 1 ou 2, où
A représente S, S(O) or $S(O)_2$,
X représente C(O) et est lié au groupement phényle en position méta ou para,
R1 représente un groupement alkoxy en C1-2 ou alkoxy en C1-2 entièrement ou majoritairement fluoré,
R2 représente un groupement alkoxy en C1-2 ou alkoxy en C1-2 entièrement ou majoritairement fluoré,
ou
R1 et R2 forment ensemble un groupement alkylènedioxy en C1-2,
R3 représente un groupement phényle non substitué, phényle substitué par R4 et R5 ou phényle substitué par R4, R5 et R6, où
R4 représente un groupement alkyle en C1-4, alkoxy en C1-4 ou halogène,
R5 représente un groupement alkoxy en C1-4 ou halogène, et
R6 représente un groupement alkoxy en C1-4 ou halogène, R7 représente un groupement phényle non substitué, phényle substitué par R8 ou phényle substitué par R8 et R9, où
R8 représente un groupement alkoxy en C1-2, le fluor, le chlore ou le brome, et
R9 représente un groupement alkyle en C1-2, alkoxy en C1-2, le fluor, le chlore ou le brome,
ou
R8 et R9 forment ensemble un groupement alkylènedioxy en C1-2,
ou l'un des stéréoisomères du composé.

**4.** Composé de formule (1) selon la revendication 3, où
A représente S, S(O) or $S(O)_2$,
X représente C(O) et est lié au groupement phényle en position méta ou para,
R1 représente un groupement éthoxy,

R2 représente un groupement méthoxy,
R3 représente un groupement phényle non substitué, phényle substitué par R4 et R5 ou phényle substitué par R4, R5 et R6, où
R4 représente un groupement alkyle en C1-2, alkoxy en C1-2, le fluor, le chlore ou le brome,
R5 représente un groupement alkoxy en C1-2, le fluor, le chlore ou le brome, et
R6 représente un groupement alkoxy en C1-2, le fluor, le chlore ou le brome,
R7 représente un groupement phényle non substitué, phényle substitué par R8 ou phényle substitué par R8 et R9, où
R8 représente un groupement méthoxy ou fluor et
R9 représente un groupement méthyle, méthoxy ou fluor, ou
R8 et R9 forment ensemble un groupement méthylènedioxy, ou l'un des stéréoisomères du composé.

**5.** Composé de formule (1) selon la revendication 4, où
A représente S, S(O) or $S(O)_2$,
X représente C(O) et est lié au groupement phényle en position méta ou para,
R1 représente un groupement éthoxy,
R2 représente un groupement méthoxy,
R3 représente un groupement phényle substitué par R4 et R5 ou un groupement phényle substitué par R4, R5 et R6, où
R4 représente un groupement méthyle, méthoxy ou fluor, R5 représente un groupement méthoxy ou fluor et
R6 représente un groupement méthoxy ou fluor,
R7 représente un groupement phényle non substitué, phényle substitué par R8 ou phényle substitué par R8 et R9, où
R8 représente un groupement méthoxy ou fluor,
R9 représente un groupement méthyle, méthoxy ou fluor, ou
R8 et R9 forment ensemble un groupement méthylènedioxy, ou l'un des stéréoisomères du composé.

**6.** Composé de formule (1) selon la revendication 5, qui est choisi dans le groupe constitué par les suivantes :

3-(1-{4-[(4aR,10bR)-9-éthoxy-8-méthoxy-3,4,4a,10b-tétrahydro-1H-thiopyrano[4,3-c]isoquinoléin-6-yl]benzoyl}pipéridin-4-yl)-1-(3-fluoro-4-méthoxybenzyl)-6-phénylthiéno[3,2-d]pyrimidine-2,4(1H,3H)-dione ; 3-[1-({3-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-1-(3-fluoro-4-méthoxybenzyl)-6-phénylthiéno[3,2-d]pyrimidine-2,4(1H,3H)-dione ; 1-(3,5-difluoro-4-méthoxybenzyl)-3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-6-phénylthiéno[3,2-d]pyrimidine-2,4(1H,3H)-dione ; 1-(2,3-difluoro-4-méthoxybenzyl)-3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-6-phénylthiéno[3,2-d]pyrimidine-2,4(1H,3H)-dione ; 3-[1-({4-[(4aR,10bR)-9-éthoxy-8-méthoxy-3,4,4a,10b-tétrahydro-1H-thio-pyrano[4,3-c]isoquinoléin-6-yl]phényl}carbonyl)pipéridin-4-yl]-1-(2-fluoro-4,5-diméthoxy-benzyl)-6-phénylthiéno[3,2- d]pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-1-(3-fluoro-4- méthyl benzyl)-6-phénylthiéno[3,2-d]pyrimidine-2,4(1H,3H)-dione ; 1-benzyl-3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-6-phénylthiéno[3,2-d]pyrimidine-2,4(1H,3H)-dione ; 6-(1,3-benzodioxol-5-yl)-3-t1-({4-[(4aR,10bR)-9-éthoxy-8-méthoxy-3,4,4a,10b-tétrahydro-1H-thio-pyrano[4,3-c]isoquinoléin-6-yl]phényl}carbonyl)pipéridin-4-yl]-1-(3-fluoro-4-méthoxybenzyl)thiéno-[3,2-d]pyrimidine-2,4(1H,3H)-dione ; 3-[1-({4-[(4aR,10bR)-9-éthoxy-8-méthoxy-3,4,4a,10b-tétrahydro-lH-thiopyrano[4,3-c]isoquinoléin-6-yl]phényl}carbonyl)pipéridin-4-yl]-1-(3-fluoro-4-méthoxybenzyl)-6-(4-fluoro-2-méthoxyphényl) thiéno[3,2-d]pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]-phényl}carbonyl)pipéridin-4-yl]-1-(3-fluoro-4-méthoxybenzyl)-6-(5-fluoro-2-méthoxyphényl)-thiéno[3,2-d]pyrimidine-2,4(1H,3H)-dione ; 6-(2,5-diméthoxyphényl)-3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)-pipéridin-4-yl]-1-(3-fluoro-4-méthoxybenzyl)thiéno[3,2-d]pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]-phényl}carbonyl)pipéridin-4-yl]-1-(3-fluoro-4-méthoxybenzyl)-6-(2-fluorophényl)thiéno[3,2-d]-pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-1-(3-fluoro-4-méthoxy-benzyl)-6-(4-fluorophényl)thiéno[3,2-d]pyrimidine-2,4(1H,3H)-dione ; 1-(3,5-difluoro-4-méthoxy-benzyl)-3-[1-({4-[(4aR,10bR)-9-éthoxy-8-méthoxy-3,4,4a,10b-tétrahydro-1H-thiopyrano[4,3-c]-isoquinoléin-6-yl]phényl}carbonyl)pipéridin-4-yl]-6-(4-fluoro-2- méthoxyphényl)thiéno[3,2-d]-pyrimidine-2,4(1H,3H)-dione ; 1-(2,3-difluoro-4-méthoxybenzyl)-3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-6-(4-fluoro-2-méthoxyphényl)thiéno[3,2-d]pyrimidine-2,4(1 H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)-pipéridin-4-yl]-6-(4-fluoro-2-méthoxyphényl)-1-(3-fluoro-4-méthylbenzyl) thiéno[3,2-d]pyrimidine-2,4(1H,3H)-dione, 1-benzyl-3-[1-({4-[(4aR, 10bR)-9-éthoxy-8-méthoxy-3,4,4a,10b-ietrahydro-1H-thiopyrano[4,3-c]isoquinoléin-6-yl]phényl}carbonyl)pipéridin-4-yl]-6-(4-fluoro-2-méthoxyphényl)-thiéno[3,2-d]pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-1-(3-fluoro-4-méthoxybenzyl)-6-(4-fluoro-2-méthylphényl) thiéno[3,2-d]pyrimidine-2,4(1H,3H)-dione ; 3-[1-({4-[(4aR,10bR)-9-éthoxy-8-méthoxy-3,4,4a,10b-tétrahydro-1H-thiopyrano[4,3-c]isoquinoléin-6-yl]-phényl}carbonyl)pipéridin-4-yl]-1-(3-fluoro-4-méthoxybenzyl)-6-(2-méthoxyphényl)thiéno[3,2-d]-pyrimidine-2,4(1H,3H)-dione, 6-(1,3-benzodioxol-5-yl)-3-[1-({3-[(4aR,10bR)-9-éthoxy-8-méthoxy-3,4,4a,10b-tétrahydro-1H-thiopyrano[4,3-c]isoquinoléin-6-yl]phényl}carbonyl)pipéridin-4-yl]-1-(3-fluoro-4-méthoxybenzyl)thiéno[3,2-d]pyrimidine-2,4(1H,3H)-dione et 1-(3,5-difluoro-4-méthoxy-benzyl)-3-[1-({3-[(4aR,10bR)-9-éthoxy-8-méthoxy-3,4,4a,10b-tétrahydro-1H-thiopyrano[4,3-c]-isoquinoléin-6-yl]phényl}carbonyl)pipéridin-4-yl]-6-phénylthiéno[3,2-d]pyrimidine-2,4(1H,3H)-dione.

**7.** Composé de formule (1) selon l'une quelconque des revendications 1 à 6 pour utilisation dans le traitement prophylactique ou thérapeutique de maladies.

**8.** Composition pharmaceutique comprenant au moins l'un des composés de formule (1) ou l'un des stéréoisomères de celui-ci selon l'une quelconque des revendications 1 à 6 avec au moins un excipient pharmaceutiquement acceptable.

**9.** Combinaison fixe, combinaison non fixe ou trousse de pièces comprenant au moins un composé de formule (1) ou l'un des stéréoisomères de celui-ci selon l'une quelconque des revendications 1 à 6, au moins un agent thérapeutique choisi dans le groupe constitué par les suivants : corticoïdes, anticholinergiques, agonistes de $\beta_2$-adrénorecepteurs, antagonistes de récepteurs H1, antagonistes de récepteurs de leucotriène, inhibiteurs de 5-lipoxygénase, antagonistes de récepteurs d'endothéline, prostacyclines, agents bloquant les canaux calciques, bêta-bloquants, inhibiteurs de phosphodiestérase de type 4, inhibiteurs de phosphodiestérase de type 5 , immunosuppresseurs, analogues de vitamine D, inhibiteurs de HMG-CoA réductase, agonistes de PPAR$\gamma$, inhibiteurs d'ACE, antagonistes de récepteurs d'angiotensine II, tensioactifs pulmonaires, antibiotiques, agents activant/stimulant la guanylyl-cyclase, tétrahydrobioptérine et dérivés de tétrahydrobioptérine, anticoagulants, diurétiques, pirfénidone et glycosides de digitale, et au moins un excipient pharmaceutiquement acceptable.

**10.** Utilisation d'un composé de formule (1) ou de l'un des stéréoisomères de celui-ci selon l'une quelconque des revendications 1 à 6 dans la fabrication d'une composition pharmaceutique destinée au traitement prophylactique ou thérapeutique d'une maladie aiguë ou chronique des voies respiratoires.

**11.** Utilisation selon la revendication 10, où la maladie aiguë ou chronique des voies respiratoires est choisie dans le groupe constitué par les suivantes : pneumopathie interstitielle, fibrose pulmonaire, mucoviscidose, asthme bronchique, bronchite chronique, emphysème, bronchopneumopathie chronique obstructive (BPCO) et BPCO associée à l'hypertension pulmonaire.

**12.** Composé de formule (1) ou l'un des stéréoisomères de celui-ci selon l'une quelconque des revendications 1 à 6, pour utilisation dans le traitement prophylactique ou thérapeutique d'une maladie aiguë ou chronique des voies respiratoires.

**13.** Composé de formule (1) ou l'un des stéréoisomères de celui-ci selon l'une quelconque des revendications 1 à 6 pour utilisation dans le traitement prophylactique ou thérapeutique d'une maladie aiguë ou chronique des voies respiratoires choisie dans le groupe constitué par les suivantes : pneumopathie interstitielle, fibrose pulmonaire, mucoviscidose, asthme bronchique, bronchite chronique, emphysème, bronchopneumopathie chronique obstructive (BPCO) et BPCO associée à l'hypertension pulmonaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006027345 A
- WO 2011073231 A
- US 3899494 A
- EP 0372777 A
- WO 9104011 A
- WO 9111173 A
- WO 9111495 A
- WO 9114422 A
- WO 9311743 A
- EP 0553298 A
- WO 0230394 A
- WO 0007567 A
- EP 0505321 A
- EP 407028 A
- EP 650410 A
- EP 691865 A
- EP 725725 A
- US 6397838 B
- US 6454193 B
- US 6302331 B


### Non-patent literature cited in the description


- **C. A. G. N. MONTALBETTI ; V. FALQUE.** *Tetrahedron,* 2005, vol. 61, 10827-10852
- **GREENE ; AND WUTS.** Protective Groups in Organic Synthesis. John Wiley & Sons, 1999
- *Bioorganic & Medicinal Chemistry Letters,* 2007, vol. 17, 2535-2539
- *Tetrahedron Letters,* 1996, vol. 37, 3977-3980
- *J. Chem. Soc C,* 1971, 1805-1808
- *J. Chem. Soc.,* 1956, 4280-4282
- *Heterocycles,* 2003, vol. 60 (12), 2707-2715
- **T. W. GREENE.** Protective Groups in Organic Synthesis. John Wiley & Sons, 1999
- **P. KOCIENSKI.** Protecting Groups. Thieme Medical Publishers, 2000
- **DUNKERN et al.** *Eur. J. Pharmacol.,* 2007, vol. 572 (1), 12-22
- **MARTIN-CHOULY CA et al.** *Life Sci.,* 2004, vol. 75 (7), 823-40
- **KOHYAMA T et al.** *Am. J. Respir. Cell Mol. Biol.,* 2002, vol. 26 (6), 694-701
- **CORTIJO J et al.** *Br. J. Pharmacol.,* 2009, vol. 156 (3), 534-44
- **HEMNES AR ; ZAIMAN A ; CHAMPION HC.** *Am. J. Physiol. Lung Cell. Mol. Physiol.,* 26 October 2007, vol. 294 (1), L24-33
- **GHOFRANI et al.** *Lancet,* 2002, vol. 360, 895-900
- **COLLARD et al.** *Chest,* 2007, vol. 131, 897-899